# EUROPEAN PATENT SPECIFICATION

(11) **EP 3 797 103 B1**
(45) Date of publication and mention of the grant of the patent: **08.04.2026**
(21) Application number: 19736494.6
(22) Date of filing: 20.05.2019
(51) Int. Cl.: C07D 401/14, C07D 401/04, C07C 49/255

(54) **A NOVEL PROCESS FOR THE PREPARATION OF ANTHRANILIC DIAMIDES**
NEUARTIGES VERFAHREN ZUR HERSTELLUNG VON ANTHRANILDIAMIDEN
NOUVEAU PROCÉDÉ DE PRÉPARATION DE DIAMIDES ANTHRANILIQUES

(30) Priority: 21.05.2018 IN 201811018973; 27.06.2018 IN 201811023910
(43) Date of publication of application: 31.03.2021
(73) Proprietor: PI Industries Ltd., Udaipur, Rajasthan 313001 (IN)
(72) Inventor: KARRI, Phaneendrasai, Guntur Andhra Pradesh 522006 (IN); PABBA, Jagadish, Udaipur-Rajasthan 313001 (IN); KALWAGHE, Amol Dnyaneshwar, Nagar- Maharashtra 413708 (IN); SHINDE, Bharat Uttamrao, Nagar- Maharashtra 423601 (IN); KLAUSENER, Alexander G. M., 50259 Pulheim (DE)
(74) Representative: Keltie LLP
(86) International application number: PCT/IB2019/054120
(87) International publication number: WO 2019/224678

(56) References cited:
- WO-A1-2011/157664
- DE-A1- 102006 032 168

## Description

### FIELD OF THE INVENTION:

The present invention relates to novel processes for the preparation of compounds of formula II and anthranilic diamides of formula I, and intermediates useful in such novel processes, 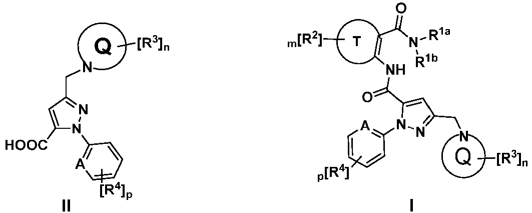 wherein, R^{1a}, R^{1b}, R², R³, R⁴, A, m, n, p, Q, and T are as defined in the description, which can be used as insecticides.

### BACKGROUND OF THE INVENTION AND PROBLEM TO BE SOLVED

1-Heteroarylpyrazole-5-carboxylic acids are known to be important intermediates in the agrochemical industry, e. g. for the synthesis of anthranilic diamides which are useful to protect crops against harmful pests. Several methods have been disclosed in literature, by which these intermediates were obtained.

The formation of pyrazoles by the reaction of 1,3-dicarbonyls or of corresponding 1,3-bis-electrophilic compounds with hydrazines has been described in Synthesis 2004, pages 43-52. However, according to other references, namely WO2003016282, and Tetrahedron (2003), vol. 59, pages 2197-2205, the product formed in such reactions consists of a mixture of regioisomeric pyrazoles, meaning that the selectivity in the synthesis of the desired product is a challenge.

WO2007144100 describes a process for preparing tetrazolyl substituted N-(aryl or heteroaryl) pyrazole-5-carboxylic acid in which diisobutylaluminium hydride (DIBAL) or lithium aluminium hydride (LiAIH₄) is used. Typical shortcomings of this process are that it requires very low temperature conditions and that the use of DIBAL as a reagent is uneconomical.

WO2010069502 describes that tetrazolyl substituted anthranilic diamides can be prepared by reacting tetrazolyl substituted N-heteroaryl pyrazole acid with anthranilamides. It also describes that tetrazolyl substituted anthranilic diamides were prepared by reacting tetrazolyl substituted benzoxazinones with appropriate amines. However, both the processes rarely offer good yield.

WO2010112178 describes a method for producing 1-pyridinylpyrazole-5-carboxylic acids or esters by the reaction of trihalo substituted acetylene ketones with pyridinyl hydrazines to first obtain an intermediate having a trihalomethyl substituent on the dihydro pyrazole ring, followed by dehydration and an oxidation step. However, trihalo substituted acetylene ketones are expensive starting materials which make this process economically unviable.

WO2011157664 and WO2013030100 disclose the following process for preparing tetrazolyl substituted 1-heteroarylpyrazole-5-carboxylic acid esters or 1-arylpyrazole-5-carboxylic acid esters:

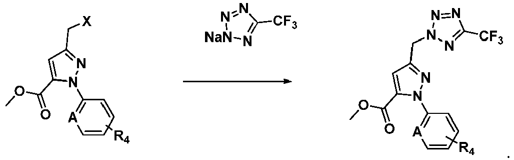

WO2013030100 additionally discloses a method for the preparation of tetrazolyl substituted anthranilic diamides from tetrazolyl substituted pyrazole carboxylic acids.

WO2011073101 describes a process for the preparation of 1-alkyl- or 1-aryl-substituted 5-pyrazolecarboxylic acids which involves the steps of converting substituted 1,3-dioxolanes or 1,4-dioxanes into 1-alkyl- or 1-aryl-substituted dihydro-1H-pyrazoles by reaction with alkyl or aryl hydrazines, and further converting said pyrazoles into 1-alkyl- or 1-aryl-substituted 5-pyrazole carboxylic acids.

Another prior art, DE102006032168 discloses the following two processes for the preparation of 1-heteroarylpyrazole-5-carboxylic acids as key intermediates: 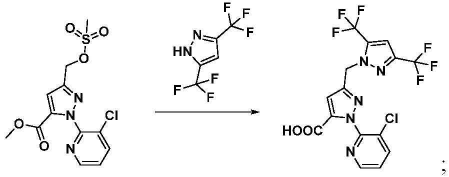 and

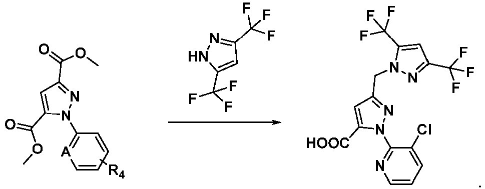

WO2011009551 describes a process for the preparation of 1-heteroaryl substituted pyrazole-5-carboxylic acids by reacting trihalo substituted alkoxy enones or enaminoketones with hydrazines to obtain 1-heteroaryl substituted dihydro-1H-pyrazoles as intermediates, followed by eliminating water and subsequent oxidation.

WO2013007604 describes a method for the preparation of tetrazolyl substituted anthranilic diamides by reacting pyrazole carboxylic acids with anthranilic esters, followed by hydrolysis to obtain acid intermediates which are then reacted with an appropriate amine.

An article published in SYNLETT 2005, No. 20, pp 3079-3082 discloses a method for the synthesis of useful intermediates as 1-phenyl-N-pyrazolylmethylpyrimidinones from 5-bromo-1,1,1-trichloro(fluoro)-4-methoxypent-3-en-2-ones with a moderate yield of 60-70%.

These processes described in the prior art have shortcomings such as poor yields of the desired intermediates or products, or synthetic procedures being not amenable to commercial scale, or of involving extreme reaction conditions or of lengthy reaction sequences making them uneconomical.

Further, 1,1,1,5-Tetrahalo-4-alkoxypent-3-en-2-ones are also key precursors in the synthesis of **1-**heteroarylpyrazole-5-carboxylic acids. Several methods for preparing the key precursors (D) have been disclosed in prior art documents such as Journal of Heterocyclic Chemistry, 50(1), pp. 71-77; Tetrahedron Letters, 51(35), pp. 4623-4626; Journal of F Chemistry, 128(10), pp. 1264-1270; Synthesis, (16), pp. 2353-2358; Synthesis, (13), pp. 1959-1964; and Synthesis (3), pp. 431-436; by acylating enol ether (A) or acetal with trihaloacetic anhydride or trihaloacetic acid or trihaloacetyl halide (B) and then halogenating the intermediate (C).

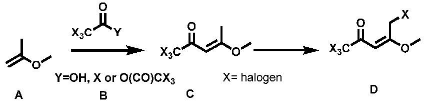

However, in these processes the isolation of the intermediate (C) is inevitable thereby making them uneconomical.

Thus, there is still a need for a process that obviates at least one shortcomings associated with the known processes.

### OBJECT AND SUMMARY OF THE INVENTION

The present invention is directed to a process for preparing a compound of formula II, 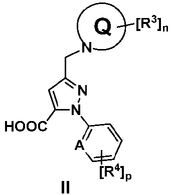 wherein,
A is N or C;
R³ and R⁴ are independently selected from the group consisting of hydrogen, halogen, cyano, nitro, hydroxy, C₁-C₆-alkyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, C₁-C₆-alkylsulphinyl, C₁-C₆-alkylsulphonyl, C₁-C₆-haloalkylthio, C₁-C₆-haloalkylsulphinyl, C₁-C₆-haloalkylsulphonyl and NR^{a}R^{b}; R^{a} and R^{b} are independently selected from the group consisting of hydrogen, C₁-C₆-alkyl, C₃-C₆-cycloalkyl; or R^{a} and R^{b} together with the N atom to which they are attached form a substituted or unsubstituted 3- to 6-membered heterocyclic ring,
   wherein, the substituents on the 3- to 6- membered heterocyclic ring are selected from the group consisting of halogen, cyano, nitro, hydroxy, C₁-C₆-alkyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, C₁-C₆-alkylsulphinyl, C₁-C₆-alkylsulphonyl, C₁-C₆-haloalkylthio, C₁-C₆-haloalkylsulphinyl and C₁-C₆-haloalkylsulphonyl;
Q is a 3-, 4- or 5 membered heterocyclic ring;
n is an integer 0 to 4; and
p is an integer 0 to 5;
wherein the process comprising the steps of:
a. reacting a compound of formula IV with a compound of formula VII in the presence of one or more suitable solvent and optionally, one or more suitable catalyst and or reagent to obtain a compound of formula III, 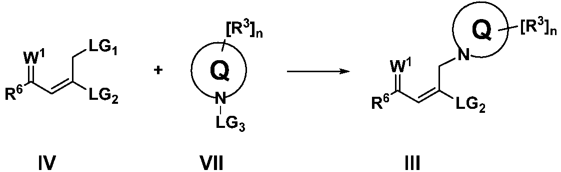 wherein,
   R⁶ is selected from the group consisting of CX₃, COW²(R⁷), C(W⁴R⁷)₃, CH(W⁴R⁷)₂, allylic group, substituted or unsubstituted furanyl, 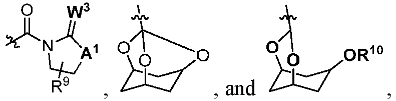 wherein, the substitution on furanyl group is selected from the group consisting of halogen, cyano, nitro, hydroxy, C₁-C₆-alkyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, C₁-C₆-alkylsulphinyl, C₁-C₆-alkylsulphonyl, C₁-C₆-haloalkylthio, C₁-C₆-haloalkylsulphinyl, or C₁-C₆-haloalkylsulphonyl;
   W¹, W², W³, W⁴ and A¹ are independently O, S or NR^{1c}; wherein R^{1c} is hydrogen, C₁-C₆-alkyl, or C₃-C₆-cycloalkyl;
   R⁷ is selected from the group consisting of hydrogen, substituted or unsubstituted C₁-C₆-alkyl, substituted or unsubstituted C₃-C₆-cycloalkyl, substituted or unsubstituted aryl and substituted or unsubstituted arylalkyl, or
   two R⁷ together with the atom to which they are attached form a substituted or unsubstituted 3- to 6- membered carbocyclic or heterocyclic ring,
      wherein, the substitution on C₁-C₆-alkyl, C₃-C₆-cycloalkyl, aryl, and arylalkyl of R⁷ and carbocyclic or heterocyclic ring formed by two R⁷ are independently selected from the group consisting of halogen, cyano, nitro, hydroxy, C₁-C₆-alkyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, C₁-C₆-alkylsulphinyl, C₁-C₆-alkylsulphonyl, C₁-C₆-haloalkylthio, C₁-C₆-haloalkylsulphinyl, and C₁-C₆-haloalkylsulphonyl;
   R⁹ and R¹⁰ are independently selected from the group consisting of hydrogen, halogen, cyano, substituted or unsubstituted C₁-C₆-alkyl, substituted or unsubstituted C₁-C₆-alkoxy, substituted or unsubstituted C₃-C₆-cycloalkyl, substituted or unsubstituted C₁-C₆-alkylthio, substituted or unsubstituted C₁-C₆-alkylsulphinyl, substituted or unsubstituted C₁-C₆-alkylsulphonyl, substituted or unsubstituted aryl and substituted or unsubstituted arylalkyl,
      wherein, the substitution on C₁-C₆-alkyl, C₁-C₆-alkoxy, C₃-C₆-cycloalkyl, C₁-C₆-alkylthio, C₁-C₆-alkylsulphinyl, C₁-C₆-alkylsulphonyl, aryl, and arylalkyl of R⁹ and R¹⁰ is selected from the group consisting of halogen, cyano, nitro, hydroxy, C₁-C₆-alkyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, C₁-C₆-alkylsulphinyl, C₁-C₆-alkylsulphonyl, C₁-C₆-haloalkylthio, C₁-C₆-haloalkylsulphinyl, and C₁-C₆-haloalkylsulphonyl;
   LG₁ is selected from the group consisting of X, OR⁵, and OSi(R¹¹)₃,
      wherein, R⁵ is selected from the group consisting of hydrogen, substituted or unsubstituted C₁-C₆-alkyl, substituted or unsubstituted aryl-C₁-C₆-alkyl, substituted or unsubstituted aryl, -(C=O)-C₁-C₆-alkyl, -(C=O)-C₁-C₆-haloalkyl, -(C=O)O-C₁-C₆-alkyl, -(C=O)O-haloC₁-C₆-alkyl, SO₂-C₁-C₆-alkyl, SO₂-C₁-C₆-haloalkyl and substituted or unsubstituted SO₂-aryl, R¹¹ is selected from the group consisting of hydrogen, halogen, substituted or unsubstituted C₁-C₆-alkyl, substituted or unsubstituted aryl-C₁-C₆-alkyl, substituted or unsubstituted aryl, -(C=O)-C₁-C₆-alkyl, -(C=O)-C₁-C₆-haloalkyl, -(C=O)O-C₁-C₆-alkyl, -(C=O)O-haloC₁-C₆-alkyl, SO₂-C₁-C₆-alkyl, SO₂-C₁-C₆-haloalkyl and substituted or unsubstituted SO₂-aryl;
   LG₂ is X, OR¹²,
      wherein, R¹² is selected from the group consisting of hydrogen, substituted or unsubstituted C₁-C₆-alkyl, substituted or unsubstituted aryl-C₁-C₆-alkyl, substituted or unsubstituted aryl, -(C=O)-C₁-C₆-alkyl, -(C=O)-C₁-C₆-haloalkyl, -(C=O)O-C₁-C₆-alkyl, -(C=O)O-haloC₁-C₆-alkyl, SO₂-C₁-C₆-alkyl, SO₂-C₁-C₆-haloalkyl, substituted or unsubstituted SO₂-aryl, alkylthio, and NR^{a}R^{b}; R^{a} and R^{b} are independently selected from the group consisting of hydrogen, C₁-C₆-alkyl, C₃-C₆-cycloalkyl; or R^{a} and R^{b} together with the N atom to which they are attached form a substituted or unsubstituted 3- to 6- membered heterocyclic ring,
      wherein, the substitution on C₁-C₆-alkyl, aryl-C₁-C₆-alkyl, aryl, and SO₂-aryl of LG₁ and LG₂ group is selected from the group consisting of halogen, cyano, nitro, hydroxy, C₁-C₆-alkyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, C₁-C₆-alkylsulphinyl, C₁-C₆-alkylsulphonyl, C₁-C₆-haloalkylthio, C₁-C₆-haloalkylsulphinyl, and C₁-C₆-haloalkylsulphonyl;
   LG₃ is selected from the group consisting of hydrogen, alkali metal, halogen and Si(R¹¹)₃,
   each X is independently hydrogen, F, Cl, Br or I;
   R³, n, and Q are each as defined above;
b. cyclizing the compound of formula III with a hydrazine of formula VIII in one or more suitable solvent and optionally, one or more suitable reagent to obtain a compound of formula IIA, 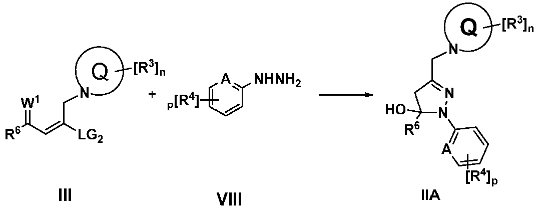 wherein, R³, R⁴, R⁶, A, n, p, Q, W¹, and LG₂ are each as defined herein above;
c. eliminating water from the compound of formula IIA by using one or more suitable dehydrating reagent in one or more suitable solvent to obtain a compound of formula IIB, 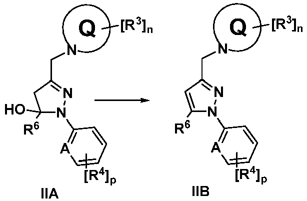 wherein, R³, R⁴, R⁶, A, n, p, and Q are each as defined herein above; and
d. converting the compound of formula IIB into the compound of formula II using one or more suitable reagent in one or more suitable solvent and optionally, one or more suitable catalyst, 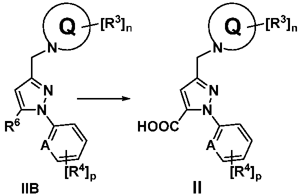
   wherein, R³, R⁴, R⁶, A, n, p, and Q are each as defined herein above; and
   wherein, the compound of formula III obtained in step (a), the compound of formula IIA obtained in step (b) and the compound of formula IIB obtained in step (c) may or may not be isolated.

The present invention provides an alternative process for obtaining the compound of formula IIB, wherein the process comprising the steps of:
i. cyclizing a compound of formula XVII with the hydrazine of formula VIII in one or more suitable solvent and optionally, one or more suitable reagent to obtain a compound of formula XVI, 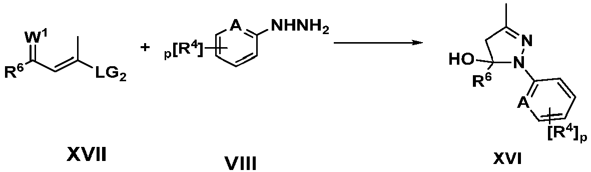
   wherein, R⁶ is CX₃, X is F, Cl, Br and I; R⁴, A, p, W¹, and LG₂ are each as defined above;
      or
   cyclizing the compound of formula IV and the hydrazine of formula VIII in one or more suitable solvent and optionally, one or more suitable reagent to obtain a compound of formula XVIII, 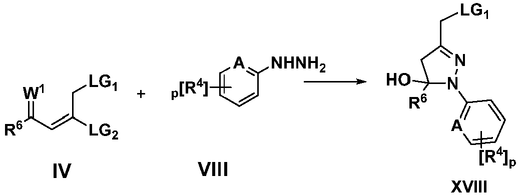
   wherein, R⁶ is CX₃, LG₁ is Cl; X is F, Cl, Br and I, particularly Cl; R⁴, A, p, W¹ and LG₂ are each as defined above;
ii. eliminating water from the compound of formula XVI by using one or more suitable dehydrating reagent in one or more suitable solvent to obtain a compound of formula XV; 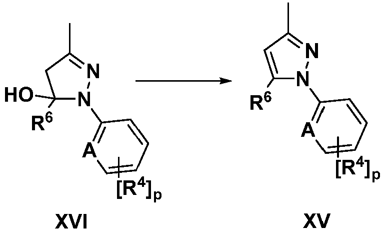
   wherein, R⁶ is CX₃, X is F, Cl, Br and I; R⁴, A, and p are each as defined above;
      or
   eliminating water from the compound of formula XVIII by using one or more suitable dehydrating reagent in one or more suitable solvent to obtain a compound of formula XIV, 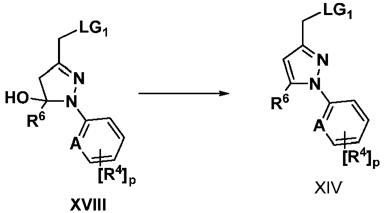
   wherein, R⁶ is CX₃, LG₁ is Cl, X is F, Cl, Br and I; R⁴, A, and p, are each as defined above;
iii. halogenating the compound of formula XV using a suitable halogenating agent in one or more suitable solvent and optionally, one or more radical initiator to obtain a compound of formula XIV, 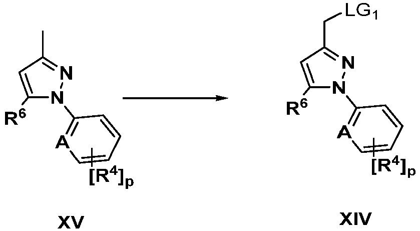 wherein, R⁶ is CX₃, LG₁ is X, X is F, Cl, Br and I; R⁴, A, and p, are each as defined above; and
iv. obtaining the compound of formula IIB by reacting the compound of formula XIV with the compound of formula VII, 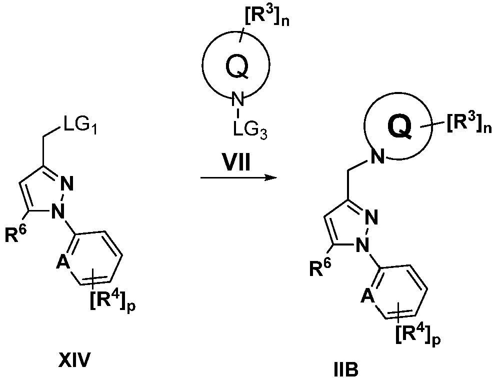
   wherein, R⁶ is CX₃, LG₁ is X, X is F, Cl, Br and I; R³, R⁴, A, Q, n, p, and LG₃ are each as defined above; and
   the compounds of formula XVI and XVIII obtained in step (i), the compounds of formula XV and XIV obtained in step (ii) and the compound XIV obtained in step (iii) may or may not be isolated.

The present invention is also directed to the preparation of a compound of formula I from the compound of formula II which is obtained through sequence of steps a-d as described above, by reacting the compound of formula II optionally after converting it into a compound of formula X with a compound of formula IX to obtain the compound of formula I, or by reacting the compound of formula II optionally after converting into a compound of formula X with a compound of formula XI to obtain a compound of formula XII and then reacting the compound of formula XII with suitable amine XIII to obtain the compound of formula I, 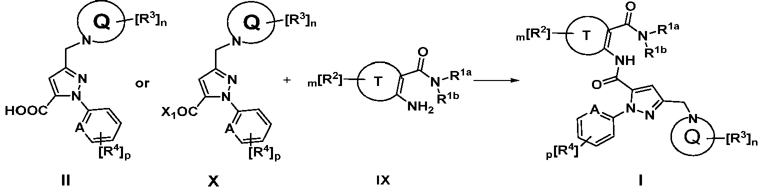 or 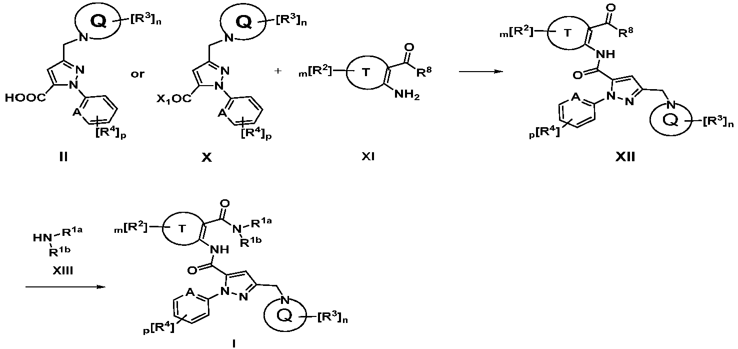 wherein,
X₁ is Cl or Br;
R^{1a} and R^{1b} are independently selected from the group consisting of hydrogen, C₁-C₆-alkyl, C₁-C₆-haloalkyl, C₃-C₆-cycloalkyl, (C₁-C₆-alkyl)-C₃-C₆-cycloalkyl, and (C₃-C₆-cycloalkyl)-C₁-C₆-alkyl; or
R^{1a} and R^{1b} together with the N atom to which they are attached form N=S(=O)₀₋₂(C₁-C₆-alkyl)₂;
T is an aryl or a heteroaryl ring or a fused or a bicyclic aryl or heteroaryl ring or ring system;
R² is selected from the group consisting of hydrogen, halogen, cyano, nitro, C₁-C₆-alkyl, C₁-C₆-haloalkyl, and C₃-C₆-cycloalkyl;
R⁸ is selected from the group consisting of the group consisting of hydroxy, Cl and OR⁷;
m is an integer 0 to 6; and
R³, R⁴, A, n, p, and Q are each as defined above;

The present invention is further directed to a process for obtaining the compound of formula IV, wherein the process steps of:
i. converting a compound of formula IV-A into a compound of formula IV-B using a suitable reagent in one or more suitable solvent, 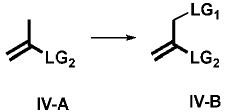 wherein, LG₁ and LG₂ are each as defined above;
ii. reacting the compound of formula IV-B with a compound of formula IV-C in a suitable solvent and optionally using suitable reagent to obtain the compound of formula IV, 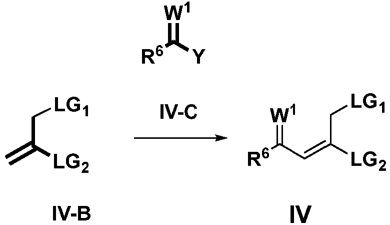
   wherein, Y is OR⁵, X, or -O(C=O)CX₃; X is F, Cl, Br or I; R⁵, R⁶, W¹, LG₁ and LG₂ are each as defined above,
      and
   wherein, the compound of formula IV-B may or may not be isolated.

In another embodiment, the present invention provides a process for preparing a compound of formula II-1 from a compound of formula IV-1 or IV-2, 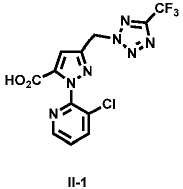 wherein the process comprising the steps of:
a. reacting a compound of formula IV-1 or IV-2 with a compound of formula VII-1 in the presence of one or more suitable solvent and optionally, one or more suitable catalyst and or reagent to obtain a mixture of a compound of formula III-1 and a compound of formula III-2, 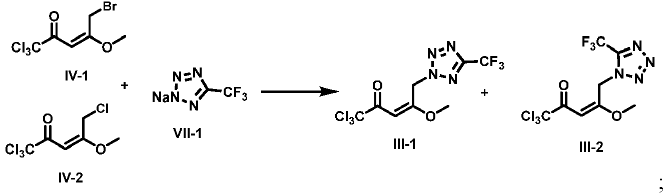
b. cyclizing the compound of formula III-1, or the compound of formula III-1 and the compound of formula III-2 in the mixture, with a hydrazine of formula VIII-1 in one or more suitable solvent and optionally, one or more suitable reagent to obtain a compound of formula IIA-1 or the mixture of the compound of formula IIA-1 and a compound of formula IIA-2 respectively, 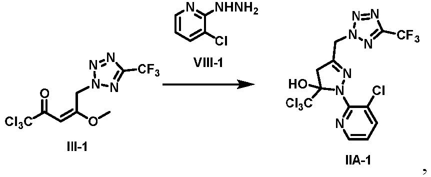 or 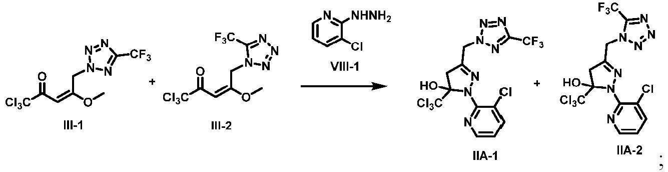
c. eliminating water from the compound of formula IIA-1, or the compound of formula IIA-1 and the compound of formula IIA-2 in the mixture, by using one or more suitable dehydrating reagent in one or more suitable solvent to obtain a compound of formula IIB-1 or the mixture of the compound of formula IIB-1 and a compound of formula IIB-2 respectively, 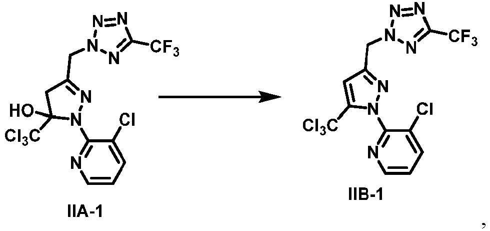 or 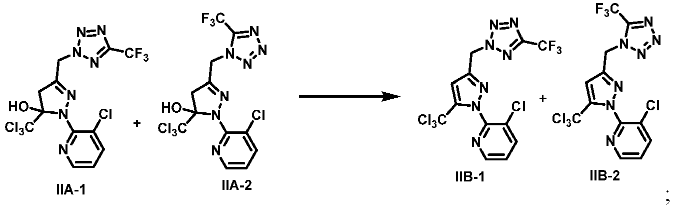 and
d. converting the compound of formula IIB-1, or the compound of formula IIB-1 and the compound of formula IIB-2 in the mixture, into the compound of formula II-1 or the mixture of the compound of formula II-1 and a compound of formula II-2 respectively, using one or more suitable reagent in one or more suitable solvent and optionally, one or more suitable catalyst, 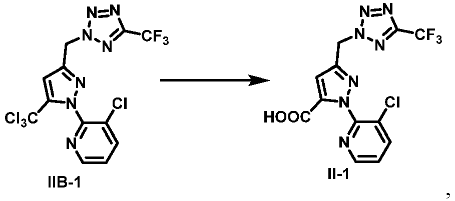 or 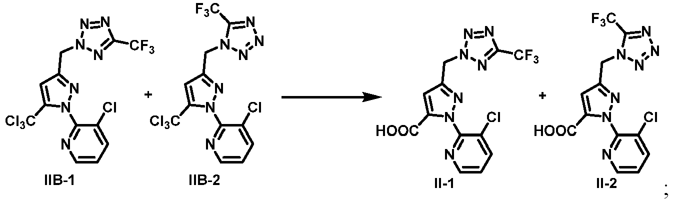 and
   wherein, the compound of formula III-1 or the mixture of the compounds of formula III-1 and III-2 obtained in step (a), the compound of formula IIA-1 or the mixture of the compounds of formula IIA-1 and IIA-2 obtained in step (b) and the compound of formula IIB-1 or the mixture of the compounds of formula IIB-1 and IIB-2 obtained in step (c) may or may not be isolated.

The compound of formula IIB-1 is also alternatively obtained by the process comprising the steps of:
i. cyclizing a compound of formula XVII-1 with the hydrazine of formula VIII-1 in one or more suitable solvent and optionally, one or more suitable reagent to obtain a compound of formula XVI-1, 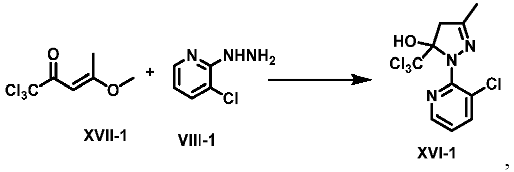 or
   cyclizing the compound of formula IV-2 and the hydrazine of formula VIII-1 in one or more suitable solvent and optionally, one or more suitable reagent to obtain a compound of formula XVIII-1, 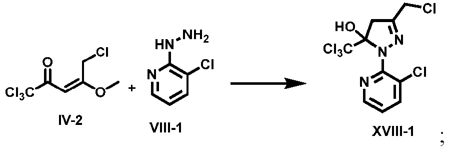
ii. eliminating water from the compound of formula XVI-1 by using one or more suitable dehydrating reagent in one or more suitable solvent to obtain a compound of formula XV-1; 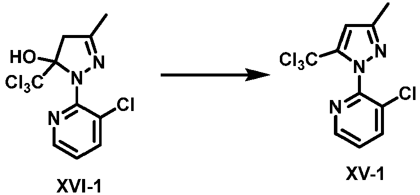 or
   eliminating water from the compound of formula XVIII by using one or more suitable dehydrating reagent in one or more suitable solvent to obtain a compound of formula XIV, 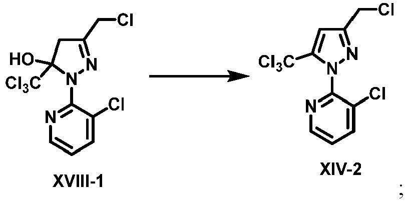
iii. halogenating the compound of formula XV-1 using a suitable halogenating agent in one or more suitable solvent and optionally, one or more radical initiator to obtain a compound of formula XIV-1, 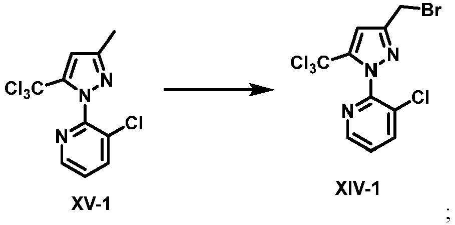 and
iv. obtaining the compound of formula IIB-1 or a mixture of the compound of formula IIB-1 and a compound of formula IIB-2 by reacting the compound of formula XIV-1 or XIV-2 with the compound of formula VII-1, 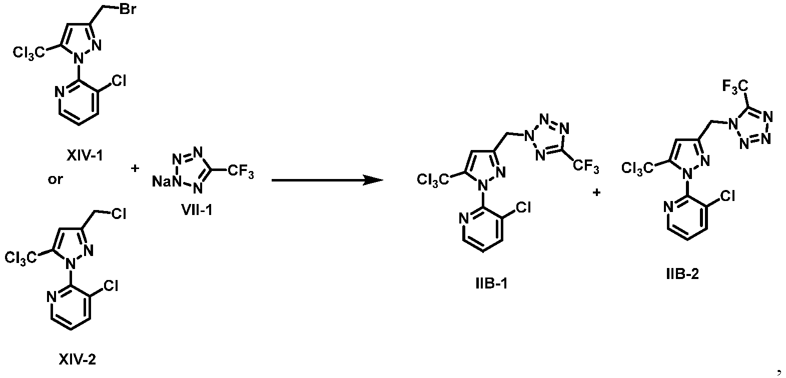 wherein, the compounds of formula XVI-1 and XVIII-1 obtained in step (i), the compounds of formula XV-1 and XIV-2 obtained in step (ii) and the compound XIV-1 obtained in step (iii) may or may not be isolated.

In another embodiment, the present invention also provides a process for preparing a compound of formula II-1 starting from a compound of formula IV-3 or IV-4, wherein the process comprising the steps of:
a. reacting a compound of formula IV-3 or IV-4 with a compound of formula VII-1 in the presence of one or more suitable solvent and optionally, one or more suitable catalyst and or reagent to obtain a mixture of a compound of formula III-3 and a compound of formula III-4 , 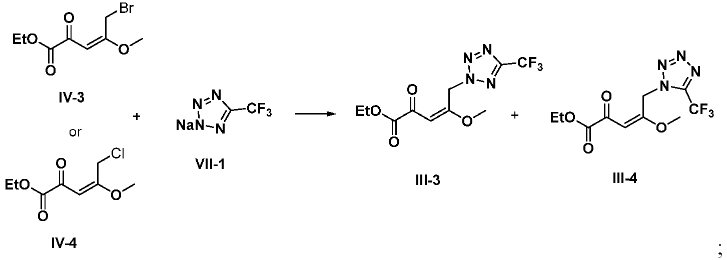
b. cyclizing the compound of formula III-3, or the compound of formula III-3 and the compound of formula III-4 in the mixture, with a hydrazine of formula VIII-1 in one or more suitable solvent and optionally, one or more suitable reagent to obtain a compound of formula IIA-3 or the mixture of the compound of formula IIA-3 and a compound of formula IIA-4 respectively, 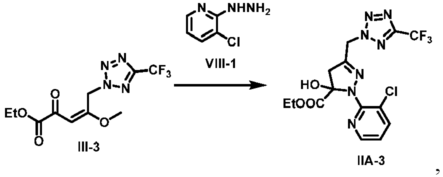 or 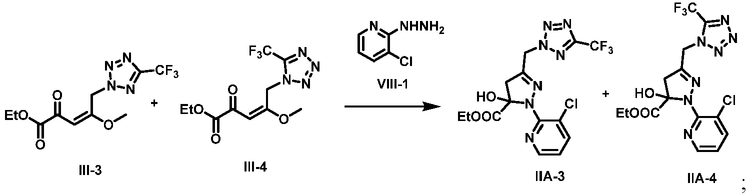
c. eliminating water from the compound of formula IIA-3, or the compound of formula IIA-3 and the compound of formula IIA-4 in the mixture, by using one or more suitable dehydrating reagent in one or more suitable solvent to obtain a compound of formula IIB-3 or the mixture of the compound of formula IIB-3 and a compound of formula IIB-4 respectively, 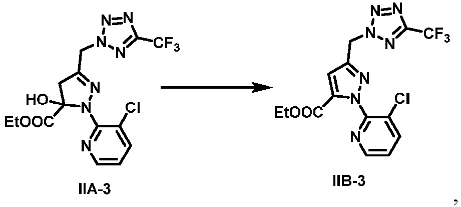 or 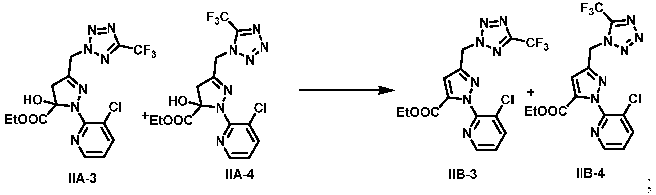 and
d. converting the compound of formula IIB-3, or the compound of formula IIB-3 and the compound of formula IIB-4 in the mixture, into the compound of formula II-1 or the mixture of the compound of formula II-1 and a compound of formula II-2 respectively, using one or more suitable reagent in one or more suitable solvent and optionally, one or more suitable catalyst, 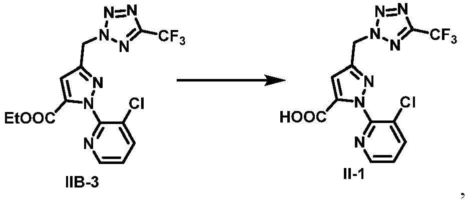 or 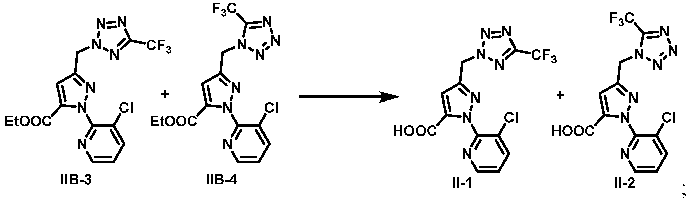 and
   wherein, the compound of formula III-3 or the mixture of the compounds of formula III-3 and III-4 obtained in step (a), the compound of formula IIA-3 or the mixture of the compounds of formula IIA-3 and IIA-4 obtained in step (b) and the compound of formula IIB-3 or the mixture of the compounds of formula IIB-3 and IIB-4 obtained in step (c) may or may not be isolated.

In yet another embodiment, the present invention provides a process for preparing a compound of formula I-1 from the compound of formula II-1 which is obtained through sequence of steps a-d as described above, by reacting the compound of formula II-1 optionally after converting into a compound of formula X-1 with a compound of formula IX-1 to obtain the compound of formula I-1, or reacting the compound of formula II-1 optionally after converting into a compound of formula X-1 with a compound of formula XI to obtain a compound of formula XII and reacting the compound of formula XII with amine XIII-1 to obtain the compound of formula I-1, 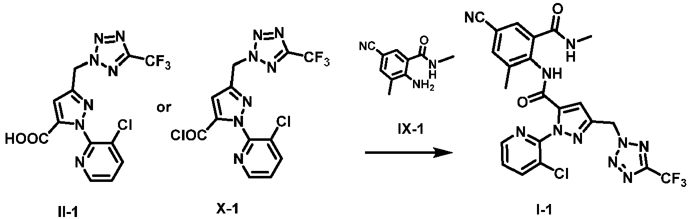 or 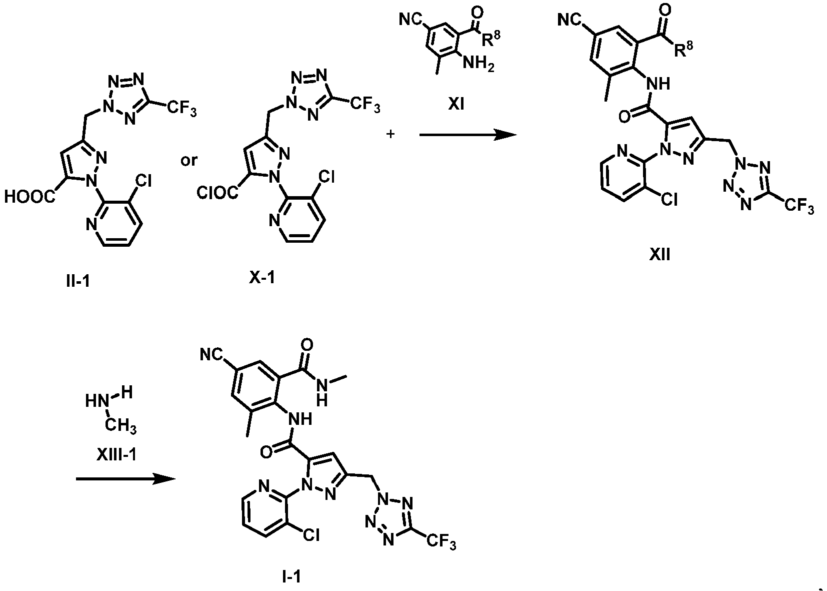 wherein, R⁸ is hydroxy or Cl.

Alternatively, the compound of formula I-1 in the present invention is prepared from the compound of formula II-1 which is obtained through sequence of steps a-d as described above, by reacting the compound of formula II-1 optionally after converting into a compound of formula X-1 with a compound of formula IX-1 to obtain the compound of formula I-1, or by reacting the compound of formula II-1 optionally after converting into a compound of formula X-1 with a compound of formula XI to obtain a compound of formula XII and reacting the compound of formula XII with amine XIII-1 to obtain the compound of formula I-1, 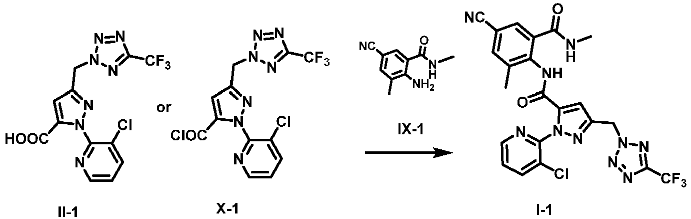 or 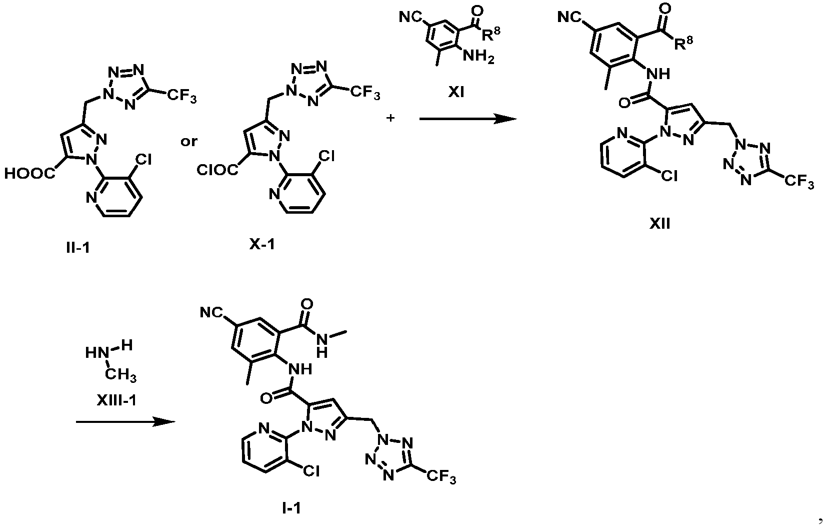 wherein, R⁸ is hydroxy or Cl.

The present invention is also directed to novel intermediates XIX, XX, III, XV-2 and XVI for preparing the compound of formula II and the compound of formula I, 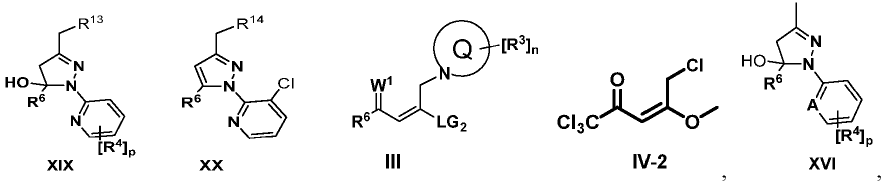 wherein R³, R⁴, R⁶, R¹³, R¹⁴, p and LG₂ are as defined above.

### DETAILED DESCRIPTION OF THE INVENTION

### GENERAL DEFINITIONS

The definitions provided herein for the terminologies used in the present disclosure are for illustrative purpose only and in no manner limit the scope of the present invention disclosed in the present disclosure. As used herein, the terms "comprises", "comprising", "includes", "including", "has", "having", "contains", "containing", "characterized by" or any other variation thereof, are intended to cover a non-exclusive inclusion, subject to any limitation explicitly indicated. For example, a composition, mixture, process or method that comprises a list of elements is not necessarily limited to only those elements but may include other elements not expressly listed or inherent to such composition, mixture, process or method.

The transitional phrase "consisting of" excludes any element, step or ingredient not specified. If in the claim, such would close the claim to the inclusion of materials other than those recited except for impurities ordinarily associated therewith. When the phrase "consisting of" appears in a clause of the body of a claim, rather than immediately following the preamble, it limits only the element set forth in that clause; other elements are not excluded from the claim as a whole.

The transitional phrase "consisting essentially of' is used to define a composition or method that includes materials, steps, features, components or elements, in addition to those literally disclosed, provided that these additional materials, steps, features, components or elements do not materially affect the basic and novel characteristic(s) of the claimed invention. The term "consisting essentially of' occupies a middle ground between "comprising" and "consisting of".

Further, unless expressly stated to the contrary, "or" refers to an inclusive "or" and not to an exclusive "or". For example, a condition A "or" B is satisfied by any one of the following: A is true (or present) and B is false (or not present), A is false (or not present) and B is true (or present), and both A and B are true (or present).

Also, the indefinite articles "a" and "an" preceding an element or component of the present invention are intended to be nonrestrictive regarding the number of instances (i.e. occurrences) of the element or component. Therefore "a" or "an" should be read to include one or at least one, and the singular word form of the element or component also includes the plural unless the number is obviously meant to be singular. Compounds of the present disclosure may be present either in pure form or as mixtures of different possible isomeric forms such as stereoisomers or constitutional isomers. The various stereoisomers include enantiomers, diastereomers, chiral isomers, atropisomers, conformers, rotamers, tautomers, optical isomers, polymorphs, and geometric isomers. Any desired mixtures of these isomers fall within the scope of the claims of the present disclosure. One skilled in the art will appreciate that one stereoisomer may be more active and/or may exhibit beneficial effects when enriched relative to the other isomer(s) or when separated from the other isomer(s). Additionally, the person skilled in the art knows processes or methods or technology to separate, enrich, and/or to selectively prepare said isomers.

The meaning of various terms used in the description shall now be illustrated.

The term "alkyl", used either alone or in compound words such as "alkylthio" or "haloalkyl" or -N(alkyl) or alkylcarbonylalkyl or alkylsuphonylamino includes straight-chain or branched C₁ to C₂₄ alkyl, preferably C₁ to C₁₅ alkyl, more preferably C₁ to C₁₀ alkyl, most preferably C₁ to C₆ alkyl. Non-limiting examples of alkyl include methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl or the different isomers. If the alkyl is at the end of a composite substituent, as, for example, in alkylcycloalkyl, the part of the composite substituent at the start, for example the cycloalkyl, may be mono- or polysubstituted identically or differently and independently by alkyl. The same also applies to composite substituents in which other radicals, for example alkenyl, alkynyl, hydroxyl, halogen, carbonyl, carbonyloxy and the like, are at the end.

The term "alkenyl", used either alone or in compound words includes straight-chain or branched C₂ to C₂₄ alkenes, preferably C₂ to C₁₅ alkenes, more preferably C₂ to C₁₀ alkenes, most preferably C₂ to C₆ alkenes. Non-limiting examples of alkenes include ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2 -propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl- 1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl and 1-ethyl-2-methyl-2-propenyl and the different isomers. "Alkenyl" also includes polyenes such as 1,2-propadienyl and 2,4-hexadienyl. This definition also applies to alkenyl as a part of a composite substituent, for example haloalkenyl and the like, unless defined specifically elsewhere.

Non-limiting examples of alkynes include ethynyl, 1-propynyl, 2-propynyl, 1-butynyl, 2-butynyl, 3-butynyl, 1-methyl-2-propynyl, 1-pentynyl, 2-pentynyl, 3-pentynyl, 4-pentynyl, 1-methyl-2-butynyl, 1-methyl-3-butynyl, 2-methyl-3-butynyl, 3-methyl-1-butynyl, 1,1-dimethyl-2-propynyl, 1-ethyl -2-propynyl, 1-hexynyl, 2-hexynyl, 3-hexynyl, 4-hexynyl, 5-hexynyl, 1-methyl-2-pentynyl, 1-methyl-3-pentynyl, 1-methyl-4-pentynyl, 2-methyl-3-pentynyl, 2-methyl-4-pentynyl, 3-methyl-1-pentynyl, 3-methyl-4-pentynyl, 4-methyl-1-pentynyl, 4-methyl-2-pentynyl, 1,1-dimethyl-2-butynyl, 1,1-dimethyl-3-butynyl, 1,2-dimethyl-3-butynyl, 2,2-dimethyl-3-butynyl, 3,3-dimethyl-1-butynyl, 1-ethyl-2-butynyl, 1-ethyl-3-butynyl, 2-ethyl-3-butynyl and 1-ethyl-1-methyl-2-propynyl and the different isomers. This definition also applies to alkynyl as a part of a composite substituent, for example haloalkynyl etc., unless specifically defined elsewhere. "Alkynyl" can also include moieties comprised of multiple triple bonds such as 2,5-hexadiynyl.

The term "cycloalkyl" means alkyl closed to form a ring. Non-limiting examples include cyclopropyl, cyclobutyl, cyclopentyl and cyclohexyl. This definition also applies to cycloalkyl as a part of a composite substituent, for example cycloalkylalkyl etc., unless specifically defined elsewhere.

Cycloalkenyl means alkenyl closed to form a ring including monocyclic, partially unsaturated hydrocarbyl groups. Non-limiting examples include cyclopentenyl and cyclohexenyl. This definition also applies to cycloalkenyl as a part of a composite substituent, for example cycloalkenylalkyl etc., unless specifically defined elsewhere.

Cycloalkynyl means alkynyl closed to form a ring including monocyclic, partially unsaturated groups. This definition also applies to cycloalkynyl as a part of a composite substituent, for example cycloalkynylalkyl etc., unless specifically defined elsewhere.

The term "cycloalkoxy", "cycloalkenyloxy" and the like are defined analogously. Non limiting examples of cycloalkoxy include cyclopropyloxy, cyclopentyloxy and cyclohexyloxy. This definition also applies to cycloalkoxy as a part of a composite substituent, for example cycloalkoxy alkyl etc., unless specifically defined elsewhere.

The term "halogen", either alone or in compound words such as "haloalkyl", includes F, Cl, Br or I. Further, when used in compound words such as "haloalkyl", said alkyl may be partially or fully substituted with halogen atoms which may be the same or different.

Non-limiting examples of "haloalkyl" include chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pentafluoroethyl, 1,1-dichloro-2,2,2-trifluoroethyl, and 1,1,1-trifluoroprop-2-yl. This definition also applies to haloalkyl as a part of a composite substituent, for example haloalkylaminoalkyl etc., unless specifically defined elsewhere.

The terms "haloalkenyl" and "haloalkynyl" are defined analogously except that, instead of alkyl groups, alkenyl and alkynyl groups are present as a part of the substituent.

The term "haloalkoxy" means straight-chain or branched alkoxy groups where some or all of the hydrogen atoms in these groups may be replaced by halogen atoms as specified above. Non-limiting examples of haloalkoxy include chloromethoxy, bromomethoxy, dichloromethoxy, trichloromethoxy, fluoromethoxy, difluoromethoxy, trifluoromethoxy, chlorofluoromethoxy, dichlorofluoromethoxy, chlorodifluoromethoxy, 1-chloroethoxy, 1-bromoethoxy, 1-fluoroethoxy, 2-fluoroethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy, pentafluoroethoxy and 1,1,1-trifluoroprop-2-oxy. This definition also applies to haloalkoxy as a part of a composite substituent, for example haloalkoxyalkyl etc., unless specifically defined elsewhere.

The term "haloalkylthio" means straight-chain or branched alkylthio groups where some or all of the hydrogen atoms in these groups may be replaced by halogen atoms as specified above. Non-limiting examples of haloalkylthio include chloromethylthio, bromomethylthio, dichloromethylthio, trichloromethylthio, fluoromethylthio, difluoromethylthio, trifluoromethylthio, chlorofluoromethylthio, dichlorofluoromethylthio, chlorodifluoromethylthio, 1-chloroethylthio, 1-bromoethylthio, 1-fluoroethylthio, 2-fluoroethylthio, 2,2-difluoroethylthio, 2,2,2-trifluoroethylthio, 2-chloro-2-fluoroethylthio, 2-chloro-2,2-difluoroethylthio, 2,2-dichloro-2-fluoroethylthio, 2,2,2-trichloroethylthio, pentafluoroethylthio and 1,1,1-trifluoroprop-2-ylthio. This definition also applies to haloalkylthio as a part of a composite substituent, for example haloalkylthioalkyl etc., unless specifically defined elsewhere.

Examples of "haloalkylsulfinyl" include CF₃S(O), CCl₃S(O), CF₃CH₂S(O) and CF₃CF₂S(O). Examples of "haloalkylsulfonyl" include CF₃S(O)₂, CCl₃S(O)₂, CF₃CH₂S(O)₂ and CF₃CF₂S(O)₂.

Hydroxy means -OH, Amino means -NRR, wherein R can be H or any possible substituent such as alkyl. Carbonyl means -C(O)- , carbonyloxy means -OC(O)-, sulfinyl means SO, sulfonyl means S(O)₂.

The term "alkoxy" used either alone or in compound words included C₁ to C₂₄ alkoxy, preferably C₁ to C₁₅ alkoxy, more preferably C₁ to C₁₀ alkoxy, most preferably C₁ to C₆ alkoxy. Examples of alkoxy include methoxy, ethoxy, propoxy, 1-methylethoxy, butoxy, 1-methylpropoxy, 2-methylpropoxy, 1,1-dimethylethoxy, pentoxy, 1-methylbutoxy, 2-methylbutoxy, 3-methylbutoxy, 2,2-dimethylpropoxy, 1-ethylpropoxy, hexoxy, 1,1-dimethylpropoxy, 1,2-dimethylpropoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy and 1-ethyl-2-methylpropoxy and the different isomers. This definition also applies to alkoxy as a part of a composite substituent, for example haloalkoxy, alkynylalkoxy, etc., unless specifically defined elsewhere.

"Alkoxyalkyl" denotes alkoxy substitution on alkyl. Non-limiting examples of "alkoxyalkyl" include CH₃OCH₂, CH₃OCH₂CH₂, CH₃CH₂OCH₂, CH₃CH₂CH₂CH₂OCH₂ and CH₃CH₂OCH₂CH₂.

The term "alkoxyalkoxy" denotes alkoxy substitution on alkoxy.

The term "alkylthio" includes branched or straight-chain alkylthio moieties such as methylthio, ethylthio, propylthio, 1-methylethylthio, butylthio, 1-methylpropylthio, 2-methylpropylthio, 1,1-dimethylethylthio, pentylthio, 1-methylbutylthio, 2-methylbutylthio, 3-methylbutylthio, 2,2-dimethylpropylthio, 1-ethylpropylthio, hexylthio, 1,1-dimethylpropylthio, 1,2-dimethylpropylthio, 1-methylpentylthio, 2-methylpentylthio, 3-methylpentylthio, 4-methylpentylthio, 1,1-dimethylbutylthio, 1,2-dimethylbutylthio, 1,3-dimethylbutylthio, 2,2-dimethylbutylthio, 2,3-dimethylbutylthio, 3,3-dimethylbutylthio, 1-ethylbutylthio, 2-ethylbutylthio, 1,1,2-trimethylpropylthio, 1,2,2-trimethylpropylthio, 1-ethyl-1-methylpropylthio and 1-ethyl-2-methylpropylthio and the different isomers.

Halocycloalkyl, halocycloalkenyl, alkylcycloalkyl, cycloalkylalkyl, cycloalkoxyalkyl, alkylsulfinylalkyl, alkylsulfonylalkyl, haloalkylcarbonyl, cycloalkylcarbonyl, haloalkoxylalkyl, and the like, are defined analogously to the above examples.

The term "alkylthioalkyl" denotes alkylthio substitution on alkyl. Non-limiting examples of "alkylthioalkyl" include CH₂SCH₂, CH₂SCH₂CH₂, CH₃CH₂SCH₂, CH₃CH₂CH₂CH₂SCH₂ and CH₃CH₂SCH₂CH₂. "Alkylthioalkoxy" denotes alkylthio substitution on alkoxy. The term "cycloalkylalkylamino" denotes cycloalkyl substitution on alkyl amino.

The terms alkoxyalkoxyalkyl, alkylaminoalkyl, dialkylaminoalkyl, cycloalkylaminoalkyl, cycloalkylaminocarbonyl and the like, are defined analogously to "alkylthioalkyl" or cycloalkylalkylamino. The term "alkoxycarbonyl" is an alkoxy group bonded to a skeleton via a carbonyl group (-CO-). This definition also applies to alkoxycarbonyl as a part of a composite substituent, for example cycloalkylalkoxycarbonyl and the like, unless specifically defined elsewhere.

The term "alkoxycarbonylalkylamino" denotes alkoxy carbonyl substitution on alkyl amino.

"Alkylcarbonylalkylamino" denotes alkyl carbonyl substitution on alkyl amino. The terms alkylthioalkoxycarbonyl, cycloalkylalkylaminoalkyl and the like are defined analogously.

Non-limiting examples of "alkylsulfinyl" include methylsulphinyl, ethylsulphinyl, propylsulphinyl, 1-methylethylsulphinyl, butylsulphinyl, 1-methylpropylsulphinyl, 2-methylpropylsulphinyl, 1,1-dimethylethylsulphinyl, pentylsulphinyl, 1-methylbutylsulphinyl, 2-methylbutylsulphinyl, 3-methylbutylsulphinyl, 2,2-dimethylpropylsulphinyl, 1-ethylpropylsulphinyl, hexylsulphinyl, 1,1-dimethylpropylsulphinyl, 1,2-dimethylpropylsulphinyl, 1-methylpentylsulphinyl, 2-methylpentylsulphinyl, 3-methylpentylsulphinyl, 4-methylpentylsulphinyl, 1,1-dimethylbutylsulphinyl, 1,2-dimethylbutylsulphinyl, 1,3-dimethylbutylsulphinyl, 2,2-dimethylbutylsulphinyl, 2,3-dimethylbutylsulphinyl, 3,3-dimethylbutylsulphinyl, 1-ethylbutylsulphinyl, 2-ethylbutylsulphinyl, 1,1,2-trimethylpropylsulphinyl, 1,2,2-trimethylpropylsulphinyl, 1-ethyl-1-methylpropylsulphinyl and 1-ethyl-2-methylpropylsulphinyl and the different isomers. The term "arylsulfinyl" includes Ar-S(O), wherein Ar can be any carbocyle or heterocylcle. This definition also applies to alkylsulphinyl as a part of a composite substituent, for example haloalkylsulphinyl etc., unless specifically defined elsewhere.

Non-limiting examples of "alkylsulfonyl" include methylsulphonyl, ethylsulphonyl, propylsulphonyl, 1-methylethylsulphonyl, butylsulphonyl, 1-methylpropylsulphonyl, 2-methylpropylsulphonyl, 1,1-dimethylethylsulphonyl, pentylsulphonyl, 1-methylbutylsulphonyl, 2-methylbutylsulphonyl, 3-methylbutylsulphonyl, 2,2-dimethylpropylsulphonyl, 1-ethylpropylsulphonyl, hexylsulphonyl, 1,1-dimethylpropylsulphonyl, 1,2-dimethylpropylsulphonyl, 1-methylpentylsulphonyl, 2-methylpentylsulphonyl, 3-methylpentylsulphonyl, 4-methylpentylsulphonyl, 1,1-dimethylbutylsulphonyl, 1,2-dimethylbutylsulphonyl, 1,3-dimethylbutylsulphonyl, 2,2-dimethylbutylsulphonyl, 2,3-dimethylbutylsulphonyl, 3,3-dimethylbutylsulphonyl, 1-ethylbutylsulphonyl, 2-ethylbutylsulphonyl, 1,1,2-trimethylpropylsulphonyl, 1,2,2-trimethylpropylsulphonyl, 1-ethyl-1-methylpropylsulphonyl and 1-ethyl-2-methylpropylsulphonyl and the different isomers. The term "arylsulfonyl" includes Ar-S(O)₂, wherein Ar can be any carbocyle or heterocylcle. This definition also applies to alkylsulphonyl as a part of a composite substituent, for example alkylsulphonylalkyl etc., unless defined elsewhere.

"Alkylamino", "dialkylamino", and the like, are defined analogously to the above examples.

The term "carbocycle or carbocyclic" includes "aromatic carbocyclic ring system" and "nonaromatic carbocylic ring system" or polycyclic or bicyclic (spiro, fused, bridged, nonfused) ring compounds in which ring may be aromatic or non-aromatic (where aromatic indicates that the Hückel's rule is satisfied and nonaromatic indicates that the Hückel's rule is not statisfied).

The term "heterocycle" or "heterocyclic" includes "aromatic heterocycle" or "heteroaryl ring system" and "nonaromatic heterocycle ring system" or polycyclic or bicyclic (spiro, fused, bridged, non-fused) ring compounds in which ring may be aromatic or non-aromatic, wherein the heterocycle ring contains at least one heteroatom selected from N, O, S(O)₀₋₂, and or C ring member of the heterocycle may be replaced by C(=O), C(=S), C(=CR*R*) and C=NR*, * indicates integers.

The term "non-aromatic heterocycle" or "non-aromatic heterocyclic" means three- to fifteen-membered, preferably three- to twelve-membered, saturated or partially unsaturated heterocycle containing one to four heteroatoms from the group of oxygen, nitrogen and sulphur: mono, bi- or tricyclic heterocycles which contain, in addition to carbon ring members, one to three nitrogen atoms and/or one oxygen or sulphur atom or one or two oxygen and/or sulphur atoms; if the ring contains more than one oxygen atom, they are not directly adjacent; for example (but not limited to) oxetanyl, oxiranyl, aziridinyl, 2-tetrahydrofuranyl, 3-tetrahydrofuranyl, 2-tetrahydrothienyl, 3-tetrahydrothienyl, 1-pyrrolidinyl, 2-pyrrolidinyl, 3-pyrrolidinyl, 3-isoxazolidinyl, 4-isoxazolidinyl, 5-isoxazolidinyl, 3-isothiazolidinyl, 4-isothiazolidinyl, 5-isothiazolidinyl, 1-pyrazolidinyl, 3-pyrazolidinyl, 4-pyrazolidinyl, 5-pyrazolidinyl, 2-oxazolidinyl, 4-oxazolidinyl, 5-oxazolidinyl, 2-thiazolidinyl, 4-thiazolidinyl, 5-thiazolidinyl, 1-imidazolidinyl, 2-imidazolidinyl, 4-imidazolidinyl, 1,2,4-oxadiazolidin-3-yl, 1,2,4-oxadiazolidin-5-yl, 1,2,4-thiadiazolidin-3-yl, 1,2,4-thiadiazolidin-5-yl, 1,2,4-triazolidin-1-yl, 1,2,4-triazolidin-3-yl, 1,3,4-oxadiazolidin-2-yl, 1,3,4-thiadiazolidin-2-yl, 1,3,4-triazolidin-1-yl, 1,3,4-triazolidin-2-yl, 2,3-dihydrofur-2-yl, 2,3-dihydrofur-3-yl, 2,4-dihydrofur-2-yl, 2,4-dihydrofur-3-yl, 2,3-dihydrothien-2-yl, 2,3-dihydrothien-3-yl, 2,4-dihydrothien-2-yl, 2,4-dihydrothien-3-yl, pyrrolinyl, 2-pyrrolin-2-yl, 2-pyrrolin-3-yl, 3-pyrrolin-2-yl, 3-pyrrolin-3-yl, 2-isoxazolin-3-yl, 3-isoxazolin-3-yl, 4-isoxazolin-3-yl, 2-isoxazolin-4-yl, 3-isoxazolin-4-yl, 4-isoxazolin-4-yl, 2-isoxazolin-5-yl, 3-isoxazolin-5-yl, 4-isoxazolin-5-yl, 2-isothiazolin-3-yl, 3-isothiazolin-3-yl, 4-isothiazolin-3-yl, 2-isothiazolin-4-yl, 3-isothiazolin-4-yl, 4-isothiazolin-4-yl, 2-isothiazolin-5-yl, 3-isothiazolin-5-yl, 4-isothiazolin-5-yl, 2,3-dihydropyrazol-1-yl, 2,3-dihydropyrazol-2-yl, 2,3-dihydropyrazol-3-yl, 2,3-dihydropyrazol-4-yl, 2,3-dihydropyrazol-5-yl, 3,4-dihydropyrazol-1-yl, 3,4-dihydropyrazol-3-yl, 3,4-dihydropyrazol-4-yl, 3,4-dihydropyrazol-5-yl, 4,5-dihydropyrazol-1-yl, 4,5-dihydropyrazol-3-yl, 4,5-dihydropyrazol-4-yl, 4,5-dihydropyrazol-5-yl, 2,3-dihydrooxazol-2-yl, 2,3-dihydrooxazol-3-yl, 2,3-dihydrooxazol-4-yl, 2,3-dihydrooxazol-5-yl, 3,4-dihydrooxazol-2-yl, 3,4-dihydrooxazol-3-yl, 3,4-dihydrooxazol-4-yl, 3,4-dihydrooxazol-5-yl, 3,4-dihydrooxazol-2-yl, 3,4-dihydrooxazol-3-yl, 3,4-dihydrooxazol-4-yl, piperidinyl, 2-piperidinyl, 3-piperidinyl, 4-piperidinyl, pyrazynyl, morpholinyl, thiomorphlinyl, 1,3-dioxan-5-yl, 2-tetrahydropyranyl, 4-tetrahydropyranyl, 2-tetrahydrothienyl, 3-hexahydropyridazinyl, 4-hexahydropyridazinyl, 2-hexahydropyrimidinyl, 4-hexahydropyrimidinyl, 5-hexahydropyrimidinyl, 2-piperazinyl, 1,3,5-hexahydrotriazin-2-yl, 1,2,4-hexahydrotriazin-3-yl, cycloserines, 2,3,4,5-tetrahydro[1H]azepin-1- or -2- or -3- or -4- or -5- or -6- or -7-yl, 3,4,5,6-tetra-hydro[2H]azepin-2- or -3- or -4- or -5- or -6- or-7-yl, 2,3,4,7-tetrahydro[1H]azepin-1- or - 2- or -3- or -4- or -5- or -6- or-7- yl, 2,3,6,7-tetrahydro[1H]azepin-1- or -2- or -3- or -4- or -5- or -6- or -7-yl, hexahydroazepin-1- or -2- or -3- or -4- yl, tetra- and hexahydrooxepinyl such as 2,3,4,5-tetrahydro[1 H]oxepin-2- or -3- or -4- or -5- or -6- or -7- yl, 2,3,4,7-tetrahydro[1H]oxepin-2- or -3- or -4- or -5- or -6-or -7- yl, 2,3,6,7-tetrahydro[1H]oxepin-2- or -3- or -4- or -5- or -6- or -7- yl, hexahydroazepin-1- or -2- or -3- or -4- yl, tetra- and hexahydro-1,3-diazepinyl, tetra- and hexahydro-1,4-diazepinyl, tetra- and hexahydro-1,3-oxazepinyl, tetra- and hexahydro-1,4-oxazepinyl, tetra- and hexahydro-1,3-dioxepinyl, tetra- and hexahydro-1,4-dioxepinyl. This definition also applies to heterocyclyl as a part of a composite substituent, for example heterocyclylalkyl etc., unless specifically defined elsewhere.

The term "heteroaryl" means 5 or 6-membered, fully unsaturated monocyclic ring system containing one to four heteroatoms from the group of oxygen, nitrogen and sulphur; if the ring contains more than one oxygen atom, they are not directly adjacent; 5-membered heteroaryl containing one to four nitrogen atoms or one to three nitrogen atoms and one sulphur or oxygen atom: 5-membered heteroaryl groups which, in addition to carbon atoms, may contain one to four nitrogen atoms or one to three nitrogen atoms and one sulphur or oxygen atom as ring members, for example (but not limited thereto) furyl, thienyl, pyrrolyl, isoxazolyl, isothiazolyl, pyrazolyl, oxazolyl, thiazolyl, imidazolyl, 1,2,4-oxadiazolyl, 1,2,4-thiadiazolyl, 1,2,4-triazolyl, 1,3,4-oxadiazolyl, 1,3,4-thiadiazolyl, 1,3,4-triazolyl, tetrazolyl; nitrogen-bonded 5-membered heteroaryl containing one to four nitrogen atoms, or benzofused nitrogen-bonded 5-membered heteroaryl containing one to three nitrogen atoms: 5-membered heteroaryl groups which, in addition to carbon atoms, may contain one to four nitrogen atoms or one to three nitrogen atoms as ring members and in which two adjacent carbon ring members or one nitrogen and one adjacent carbon ring member may be bridged by a buta-1,3-diene-1,4-diyl group in which one or two carbon atoms may be replaced by nitrogen atoms, where these rings are attached to the skeleton via one of the nitrogen ring members, for example (but not limited to) 1-pyrrolyl, 1-pyrazolyl, 1,2,4-triazol-1- yl, 1-imidazolyl, 1,2,3-triazol-1-yl and 1,3,4-triazol-1-yl.

6-membered heteroaryl which contains one to four nitrogen atoms: 6-membered heteroaryl groups which, in addition to carbon atoms, may contain, respectively, one to three and one to four nitrogen atoms as ring members, for example (but not limited thereto) 2-pyridinyl, 3-pyridinyl, 4-pyridinyl, 3-pyridazinyl, 4-pyridazinyl, 2-pyrimidinyl, 4-pyrimidinyl, 5-pyrimidinyl, 2-pyrazinyl, 1,3,5-triazin-2-yl, 1,2,4-triazin-3-yl and 1,2,4,5-tetrazin-3-yl; benzofused 5-membered heteroaryl containing one to three nitrogen atoms or one nitrogen atom and one oxygen or sulphur atom: for example (but not limited to) indol-1-yl, indol-2-yl, indol-3-yl, indol-4-yl, indol-5-yl, indol-6-yl, indol-7-yl, benzimidazol-1-yl, benzimidazol-2-yl, benzimidazol-4-yl, benzimidazol-5-yl, indazol-1-yl, indazol-3-yl, indazol-4-yl, indazol-5-yl, indazol-6-yl, indazol-7-yl, indazol-2-yl, 1-benzofuran-2-yl, 1-benzofuran-3-yl, 1-benzofuran-4-yl, 1-benzofuran-5-yl, 1-benzofuran- 6-yl, 1-benzofuran-7-yl, 1-benzothiophen-2-yl, 1-benzothiophen-3-yl, 1-benzothiophen-4-yl, 1-benzothiophen-5-yl, 1-benzothiophen-6-yl, 1-benzothiophen-7-yl, 1,3-benzothiazol-2-yl, 1,3- benzothiazol-4-yl, 1,3-benzothiazol-5-yl, 1,3-benzothiazol-6-yl, 1,3-benzothiazol-7-yl, 1,3-benzoxazol-2-yl, 1,3-benzoxazol-4-yl, 1,3-benzoxazol-5-yl, 1,3-benzoxazol-6-yl and 1,3-benzoxazol-7-yl; benzofused 6-membered heteroaryl which contains one to three nitrogen atoms: for example (but not limited to) quinolin-2-yl, quinolin-3-yl, quinolin-4-yl, quinolin-5-yl, quinolin-6-yl, quinolin-7-yl, quinolin-8-yl, isoquinolin-1-yl, isoquinolin-3-yl, isoquinolin-4-yl, isoquinolin-5-yl, isoquinolin-6-yl, isoquinolin-7-yl and isoquinolin-8-yl.

This definition also applies to heteroaryl as a part of a composite substituent, for example heteroarylalkyl etc., unless specifically defined elsewhere.

"Trialkylsilyl" includes 3 branched and/or straight-chain alkyl radicals attached to and linked through a silicon atom such as trimethylsilyl, triethylsilyl and t-butyl-dimethylsilyl. "Halotrialkylsilyl" denotes at least one of the three alkyl radicals is partially or fully substituted with halogen atoms which may be the same or different. "Alkoxytrialkylsilyl" denotes at least one of the three alkyl radicals is substituted with one or more alkoxy radicals which may be the same or different. "Trialkylsilyloxy" denotes a trialkylsilyl moiety attached through oxygen.

Non-limiting examples of "alkylcarbonyl" include C(O)CH₃, C(O)CH₂CH₂CH₃ and C(O)CH(CH₃)₂. Non-limiting examples of "alkoxycarbonyl" include CH₃OC(=O), CH₃CH₂OC(=O), CH₃CH₂CH₂OC(=O), (CH₃)₂CHOC(=O) and the different butoxy or pentoxycarbonyl isomers. Non-limiting examples of "alkylaminocarbonyl" include CH₃NHC(=O), CH₃CH₂NHC(=O), CH₃CH₂CH₂NHC(=O), (CH₃)₂CHNHC(=O) and the different butylamino -or pentylaminocarbonyl isomers. Non-limiting examples of "dialkylaminocarbonyl" include (CH₃)₂NC(=O), (CH₃CH₂)₂NC(=O), CH₃CH₂(CH₃)NC(=O), CH₃CH₂CH₂(CH₃)NC(=O) and (CH₃)₂CHN(CH₃)C(=O). Non-limiting examples of "alkoxyalkylcarbonyl" include CH₃OCH₂C(=O), CH₃OCH₂CH₂C(=O), CH₃CH₂OCH₂C(=O), CH₃CH₂CH₂CH₂OCH₂C(=O) and CH₃CH₂OCH₂CH₂C(=O). Non-limiting examples of "alkylthioalkylcarbonyl" include CH₃SCH₂C(=O), CH₃SCH₂CH₂C(=O), CH₃CH₂SCH₂C(=O), CH₃CH₂CH₂CH₂SCH₂C(=O) and CH₃CH₂SCH₂CH₂C(=O). The term haloalkylsufonylaminocarbonyl, alkylsulfonylaminocarbonyl, alkylthioalkoxycarbonyl, alkoxycarbonylalkyl amino and the like are defined analogously.

Non-limiting examples of "alkylaminoalkylcarbonyl" include CH₃NHCH₂C(=O), CH₃NHCH₂CH₂C(=O), CH₃CH₂NHCH₂C(=O), CH₃CH₂CH₂CH₂NHCH₂C(=O) and CH₃CH₂NHCH₂CH₂C(=O).

The term "amide" means A-R'C=ONR"-B, wherein R' and R" indicates substituents and A and B indicate any group.

The term "thioamide" means A-R'C=SNR"-B, wherein R' and R" indicates substituents and A and B indicate any group.

The total number of carbon atoms in a substituent group is indicated by the "Cᵢ-Cⱼ" prefix where i and j are numbers from 1 to 21. For example, C₁-C₃ alkylsulfonyl designates methylsulfonyl through propylsulfonyl; C₂ alkoxyalkyl designates CH₃OCH₂; C₃ alkoxyalkyl designates, for example, CH₃CH(OCH₃), CH₃OCH₂CH₂ or CH₃CH₂OCH₂; and C₄ alkoxyalkyl designates the various isomers of an alkyl group substituted with an alkoxy group containing a total of four carbon atoms, examples including CH₃CH₂CH₂OCH₂ and CH₃CH₂OCH₂CH₂. In the above recitations, when a compound of Formula I is comprised of one or more heterocyclic rings, all substituents are attached to these rings through any available carbon or nitrogen by replacement of a hydrogen on said carbon or nitrogen.

When a compound is substituted with a substituent bearing a subscript that indicates the number of said substituents can exceed 1, said substituents (when they exceed 1) are independently selected from the group of defined substituents. Further, when the subscript m in (R)ₘ indicates an integer ranging from for example 0 to 4 then the number of substituents may be selected from the integers between 0 and 4 inclusive.

When a group contains a substituent which can be hydrogen, then, when this substituent is taken as hydrogen, it is recognized that said group is being un-substituted.

The embodiments herein and the various features and advantageous details thereof are explained with reference to the non-limiting embodiments in the description. Descriptions of well-known components and processing techniques are omitted so as to not unnecessarily obscure the embodiments herein. The examples used herein are intended merely to facilitate an understanding of ways in which the embodiments herein may be practiced and to further enable those of skilled in the art to practice the embodiments herein. Accordingly, the examples should not be construed as limiting the scope of the embodiments herein.

The description of the specific embodiments will so fully reveal the general nature of the embodiments herein that others can, by applying current knowledge, readily modify and/or adapt for various applications such specific embodiments without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation. Therefore, while the embodiments herein have been described in terms of preferred embodiments, those skilled in the art will recognize that the embodiments herein can be practiced with modification within the scope of the embodiments as described herein.

Any discussion of documents, acts, materials, devices, articles and the like that has been included in this specification is solely for the purpose of providing a context for the disclosure. It is not to be taken as an admission that any or all of these matters form a part of the prior art base or were common general knowledge in the field relevant to the disclosure as it existed anywhere before the priority date of this application.

The numerical values mentioned in the description and the description/claims though might form a critical part of the present invention of the present disclosure, any deviation from such numerical values shall still fall within the scope of the present disclosure if that deviation follows the same scientific principle as that of the present invention disclosed in the present disclosure.

The compounds synthesized by the novel and inventive process of the present invention may, if appropriate, be present as mixtures of different possible isomeric forms, especially of stereoisomers, for example E and Z, threo and erythro, and also optical isomers, but if appropriate also of tautomers. Both the E and the Z isomers, and also the threo and erythro isomers, and the optical isomers, any desired mixtures of these isomers and the possible tautomeric forms are disclosed and claimed.

The present invention relates to a novel process for preparing a compound of formula I; 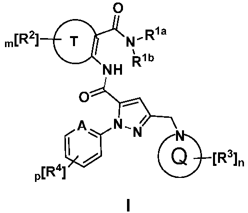 wherein,
R^{1a} and R^{1b} are independently selected from the group consisting of hydrogen, C₁-C₆-alkyl, C₁-C₆-haloalkyl, C₃-C₆-cycloalkyl, (C₁-C₆-alkyl)-C₃-C₆-cycloalkyl, or (C₃-C₆-cycloalkyl)-C₁-C₆-alkyl; or R^{1a} and R^{1b} together with the N atom to which they are attached form N=S(=O)₀₋₂(C₁-C₆-alkyl)₂;
T is an aryl or a heteroaryl ring or a fused or a bicyclic aryl or heteroaryl ring or ring system;
R² is selected from the group consisting of hydrogen, halogen, cyano, nitro, C₁-C₆-alkyl, C₁-C₆-haloalkyl, or C₃-C₆-cycloalkyl;
A is N or C;
R³ and R⁴ are independently selected from the group consisting of hydrogen, halogen, cyano, nitro, hydroxy, C₁-C₆-alkyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, C₁-C₆-alkylsulphinyl, C₁-C₆-alkylsulphonyl, C₁-C₆-haloalkylthio, C₁-C₆-haloalkylsulphinyl, C₁-C₆-haloalkylsulphonyl or NR^{a}R^{b}, R^{a} and R^{b} are independently selected from the group consisting of hydrogen, C₁-C₆-alkyl, C₃-C₆-cycloalkyl or R^{a} and R^{b} together with the N atom to which they are attached form a substituted or unsubstituted 3- to 6- membered heterocyclic ring;
Q is a 3-, 4- or 5 membered heterocyclic ring;
n is an integer 0 to 4;
m is an integer 0 to 6; and
p is an integer 0 to 5.

The substituents on the 3- to 6- membered heterocyclic ring are selected from the group consisting of halogen, cyano, nitro, hydroxy, C₁-C₆-alkyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, C₁-C₆-alkylsulphinyl, C₁-C₆-alkylsulphonyl, C₁-C₆-haloalkylthio, C₁-C₆-haloalkylsulphinyl or C₁-C₆-haloalkylsulphonyl.

In one of the particular embodiments the present invention relates to a novel process for preparing a compound of formula I;
wherein,
R^{1a} is hydrogen; R^{1b} is selected from the group consisting of hydrogen, C₁-C₆-alkyl and (C₃-C₆-cycloalkyl)-C₁-C₆-alkyl; T is a phenyl ring; R² is selected from the group consisting of halogen, cyano, and C₁-C₆-alkyl; A is N; R³ is C₁-C₆-haloalkyl; R⁴ is halogen; Q is a 5 membered heterocyclic ring; n is an integer 1; m is an integer 2; and p is an integer 1 or 2.

In another particular embodiment the present invention relates to a novel process for preparing a compound of formula I;
wherein,
R^{1a} is hydrogen; R^{1b} is C₁-C₆-alkyl; T is a phenyl ring; R² is selected from the group consisting of Cl, cyano and methyl; A is N; R³ is trifluoroalkyl or difluoroalkyl; R⁴ is Cl; Q is a tetrazole ring; n is an integer 1; m is an integer 2; and p is an integer 1 or 2.

The process for preparing the compound of formula I is described herein after.

**PROCESS STEP (a):** In the step (a) of the process, a compound of formula IV is reacted with a compound of formula VII to obtain a compound of formula III; 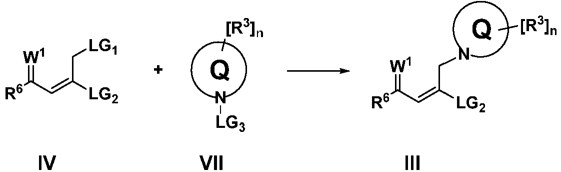 wherein,
R⁶ is selected from the group consisting of CX₃, COW²(R⁷), C(W⁴R⁷)₃, CH(W⁴R⁷)₂, allylic group, substituted or unsubstituted furanyl, 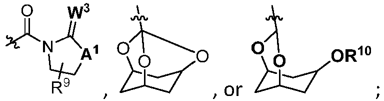 wherein,
   W¹, W², W³, W⁴ and A¹ are independently O, S or NR^{1c}; wherein R^{1c} is hydrogen, C₁-C₆-alkyl, or C₃-C₆-cycloalkyl;
   R⁷ is selected from the group consisting of hydrogen, substituted or unsubstituted C₁-C₆-alkyl, substituted or unsubstituted C₃-C₆-cycloalkyl, substituted or unsubstituted aryl and substituted or unsubstituted arylalkyl groups; or
   two R⁷ together with the atom to which they are attached form a substituted or unsubstituted 3- to 6- membered carbocyclic or heterocyclic ring; and
   R⁹ and R¹⁰ are independently selected from the group consisting of hydrogen, halogen, cyano, substituted or unsubstituted C₁-C₆-alkyl, substituted or unsubstituted C₁-C₆-alkoxy, substituted or unsubstituted C₃-C₆-cycloalkyl, substituted or unsubstituted C₁-C₆-alkylthio, substituted or unsubstituted C₁-C₆-alkylsulphinyl, substituted or unsubstituted C₁-C₆-alkylsulphonyl, substituted or unsubstituted aryl and substituted or unsubstituted arylalkyl groups;
LG₁ is selected from the group consisting of X, OR⁵, and OSi(R¹¹)₃;
   R⁵ is selected from the group consisting of hydrogen, substituted or unsubstituted C₁-C₆-alkyl, substituted or unsubstituted aryl-C₁-C₆-alkyl, substituted or unsubstituted aryl, - (C=O)-C₁-C₆-alkyl, -(C=O)-C₁-C₆-haloalkyl, -(C=O)O-C₁-C₆-alkyl, -(C=O)O-haloC₁-C₆-alkyl, SO₂-C₁-C₆-alkyl, SO₂-C₁-C₆-haloalkyl or substituted or unsubstituted SO₂-aryl;
   R¹¹ is selected from the group consisting of hydrogen, halogen, substituted or unsubstituted C₁-C₆-alkyl, substituted or unsubstituted aryl-C₁-C₆-alkyl, substituted or unsubstituted aryl, -(C=O)-C₁-C₆-alkyl, -(C=O)-C₁-C₆-haloalkyl, -(C=O)O-C₁-C₆-alkyl, -(C=O)O-haloC₁-C₆-alkyl, SO₂-C₁-C₆-alkyl, SO₂-C₁-C₆-haloalkyl and substituted or unsubstituted SO₂-aryl;
LG₂ is X, OR¹²; R¹² is selected from the group consisting of hydrogen, substituted or unsubstituted C₁-C₆-alkyl, substituted or unsubstituted aryl-C₁-C₆-alkyl, substituted or unsubstituted aryl, - (C=O)-C₁-C₆-alkyl, -(C=O)-C₁-C₆-haloalkyl, -(C=O)O-C₁-C₆-alkyl, -(C=O)O-haloC₁-C₆-alkyl, SO₂-C₁-C₆-alkyl, SO₂-C₁-C₆-haloalkyl, substituted or unsubstituted SO₂-aryl, alkylthio and NR^{a}R^{b}; R^{a} and R^{b} are independently selected from the group consisting of hydrogen, C₁-C₆-alkyl, C₃-C₆-cycloalkyl; or R^{a} and R^{b} together with the N atom to which they are attached form a substituted or unsubstituted 3- to 6- membered heterocyclic ring;
LG₃ is selected from the group consisting of hydrogen, alkali metal, halogen and Si(R¹¹)₃;
each X is independently hydrogen, F, Cl, Br or I;
R³, n, and Q are each as defined above.

The substitution on furanyl group is selected from the group consisting of halogen, cyano, nitro, hydroxy, C₁-C₆-alkyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, C₁-C₆-alkylsulphinyl, C₁-C₆-alkylsulphonyl, C₁-C₆-haloalkylthio, C₁-C₆-haloalkylsulphinyl, and C₁-C₆-haloalkylsulphonyl.

The substitution on C₁-C₆-alkyl, C₃-C₆-cycloalkyl, aryl, arylalkyl groups of R⁷ and carbocyclic or heterocyclic ring formed by two R⁷ are independently selected from the group consisting of halogen, cyano, nitro, hydroxy, C₁-C₆-alkyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, C₁-C₆-alkylsulphinyl, C₁-C₆-alkylsulphonyl, C₁-C₆-haloalkylthio, C₁-C₆-haloalkylsulphinyl, and C₁-C₆-haloalkylsulphonyl.

The substitution on C₁-C₆-alkyl, C₁-C₆-alkoxy, C₃-C₆-cycloalkyl, C₁-C₆-alkylthio, C₁-C₆-alkylsulphinyl, C₁-C₆-alkylsulphonyl, aryl, and arylalkyl groups of R⁹ and R¹⁰ is selected from the group consisting of halogen, cyano, nitro, hydroxy, C₁-C₆-alkyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, C₁-C₆-alkylsulphinyl, C₁-C₆-alkylsulphonyl, C₁-C₆-haloalkylthio, C₁-C₆-haloalkylsulphinyl, and C₁-C₆-haloalkylsulphonyl.

The substitution on C₁-C₆-alkyl, aryl-C₁-C₆-alkyl, aryl, and SO₂-aryl of LG₁ and LG₂ group is selected from the group consisting of halogen, cyano, nitro, hydroxy, C₁-C₆-alkyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, C₁-C₆-alkylsulphinyl, C₁-C₆-alkylsulphonyl, C₁-C₆-haloalkylthio, C₁-C₆-haloalkylsulphinyl, and C₁-C₆-haloalkylsulphonyl.

Particularly, definitions of the substituents of the compounds of formula IV and VII used in the process step (a) and the compound of formula III obtained in the process step (a) are as follows:
R⁶ is CX₃ or C(=O)W²R⁷,
   wherein, R⁷ is selected from the group consisting of hydrogen, C₁-C₆-alkyl, C₃-C₆-cycloalkyl, aryl and arylalkyl groups;
W¹ and W² are O; LG₁ is X; LG₂ is X or OR¹²; R¹² is C₁-C₆-alkyl; each X is independently F, Cl, Br or I; LG₃ is hydrogen or alkali metal; R³ is C₁-C₆-haloalkyl; n is an integer 1; and Q is a 3-, 4-or 5 membered heterocyclic ring.

More particularly, definitions of the substituents of the compounds of formula IV and VII used in the process step (a) and the compound of formula III obtained in the process step (a) are as follows:
R⁶ is CCl₃, CBr₃, C(=O)W²CH₃, C(=O)W²C₂H₅; W¹ and W² are O; LG₁ is Cl, Br or I, preferably Br; LG₂ is Cl, Br, I, OCH₃, or OC₂H₅; LG₃ is sodium metal ion; R³ is trifluoromethyl; n is an integer 1; and Q is a tetrazole ring.

The process step (a) is carried out in the presence of one or more suitable solvent and optionally, in the presence of one or more suitable catalyst and or reagent under particular reaction conditions.

The suitable solvent useful for the purpose of the process step (a) is preferably selected from the group consisting of acetone, acetonitrile, methyl tert-butyl ether, chlorobenzene, dichloroethane, dichloromethane, dioxane, ethyl acetate, dimethylformamide, dimethylacetamide, dimethyl sulfoxide, 2-methyltetrahydrofuran, tetrahydrofuran, 1,2-dimethoxyether, toluene, p-xylene and N-methyl-2-pyrrolidone.

The suitable catalyst for the purpose of the process step (a) is selected from the group consisting of potassium iodide, sodium iodide, copper iodide, cupric iodide.

The process step (a) of the present invention can be performed particularly within a temperature range from 20 °C to 150 °C.

The reaction time is not critical and depends on the batch size, temperature, reagent and solvent employed. Typically, the reaction time may vary from few minutes to several hours.

The process step (a) of the present invention is usually performed under standard pressure conditions. It is, however, also possible to perform the process step (a) under reduced pressure conditions to effect complete conversion of the reactants into the product.

It has been found that the replacement of LG₁ by the compound of formula VII in the process step (a) is critical to the present invention as none of the prior art discloses or suggests to couple the compound of formula IV and the compound of formula VII.

It is also found that the reaction is highly regioselective. For example, when R⁶ is CCl₃, W¹ is O, Q is tetrazole, R³ is trifluoromethyl, LG₂ is OMe and n is an integer 1, the product III-1 is formed in higher percentage than that of the product III-2:

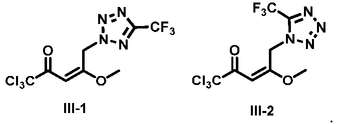

The process to obtain the compound of formula IV is known in the prior art. One of the methods involves reacting a compound 1 and 2 and then the obtained intermediate XVII-1 is converted into the compound of formula IV-1. The known reaction is depicted below:

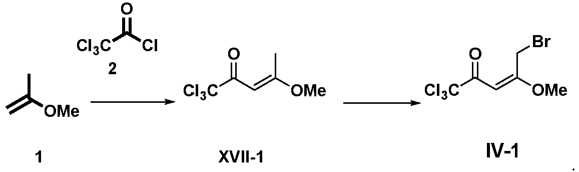

Alternatively, the compound of formula IV can be prepared by the novel process of the present invention. It has also been found, surprisingly, that if the conversion of IV-A to IV-B is carried out first followed by coupling with IV-C, then the intermediate IV-B may not be isolated and the process can be carried out in one pot.

Consequently, it is considered to be surprising that the compound of formula IV can be obtained by one pot process as depicted herein below, as pre-step of step (a), in accordance with the present invention: 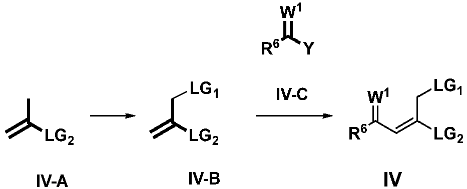 wherein, Y is OR⁵, X, or -O(C=O)CX₃; R⁵, R⁶, W¹, X, LG₁, and LG₂, are as defined herein above.

In another embodiment the compound of formula IV-B may optionally be isolated if required.

Alternatively, the compound of formula IV may also be procured commercially or can be obtained by various methods known in the prior art documents, for example, Synthesis (16), pp. 2353-2358, 2002.

Particularly, definitions of the substituents of the compounds of formula IV-A, IV-B and IV-C used and the compound of formula IV obtained are as follows:
R⁶ is CX₃ or C(=O)W²R⁷,
   wherein, R⁷ is selected from the group consisting of hydrogen, C₁-C₆-alkyl, C₃-C₆-cycloalkyl, aryl and arylalkyl groups;
W¹ and W² are O; LG₁ is X; LG₂ is X or OR¹²; R¹² is C₁-C₆-alkyl; and each X is independently F, Cl, Br or I.

More particularly, definitions of the substituents of the compounds of formula IV-A, IV-B and IV-C used and the compound of formula IV are as follows:
R⁶ is CCl₃, CBr₃, C(=O)W²CH₃, C(=O)W²C₂H₅; W¹ and W² are O; LG₁ is Cl, Br or I; LG₂ is Cl, Br, I, OCH₃, or OC₂H₅.

In accordance with the present invention, the conversion of the compound of formula IV-A into the compound of formula IV-B is carried out using one or more suitable reagent and one or more suitable solvent under particular reaction conditions. For example, if LG₁ is halogen then the suitable reagent is a halogenating reagent selected from the group consisting of HX, NaX, KX, CuX₂, MgX₂, CsX, ZnX₂, SOCl₂, SO₂Cl₂, COCl₂, X₂, C(=O)(OCl₃)₂, t-BuOCl, NaOCl, chloramine-T, N-halosuccinimides, N-halosaccharine, halohydantoines, POX₃, PX₃ and PX₅; wherein X or halo is Cl, Br, I or F. The preference is given to SOCl₂, COCl₂, X₂, and N-halosuccinimides.

The suitable solvent, used in the conversion of the compound of formula IV-A into the compound of formula IV-B, is selected preferably from the group consisting of acetone, acetonitrile, methyl tert-butyl ether, chlorobenzene, dichloroethane, dichloromethane, dioxane, ethyl acetate, dimethylformamide, dimethylacetamide, dimethyl sulfoxide, 2-methyltetrahydrofuran, tetrahydrofuran, 1,2-dimethoxyether, toluene, p-xylene and N-methyl-2-pyrrolidone.

The conversion of the compound of formula IV-A into the compound of formula IV, without isolating the compound of formula IV-B and in the same pot, is carried out using one or more suitable reagent and one or more suitable solvent under particular reaction conditions.

The suitable reagent used for assisting the conversion of formula IV-B into the compound of formula IV is selected preferably from the group consisting of pyridine, 1- or 2- or 3-picoline, triethyl amine, *N,N-*Diisopropylethylamine, 2,6-Di-tert-butylpyridine, 1,5-Diazabicyclo(4.3.0)non-5-ene, 1,8-Diazabicycloundec-7-ene, lithium diisopropylamide, sodium bis(trimethylsilyl)amide, and potassium bis(trimethylsilyl)amide.

The suitable solvent in which the conversion of formula IV-B into the compound of formula IV is preferably selected from the group consisting of acetone, acetonitrile, methyl tert-butyl ether, chlorobenzene, dichloroethane, dichloromethane, dioxane, ethyl acetate, dimethylformamide, dimethylacetamide, dimethyl sulfoxide, 2-methyltetrahydrofuran, tetrahydrofuran, 1,2-dimethoxyether, toluene, p-xylene and N-methyl-2-pyrrolidone.

The temperature conditions employed for obtaining the compound of formula IV-B and the compound of formula IV range from 20 °C to 100 °C.

The reaction time is not critical and depends on the batch size, temperature, reagent and solvent employed. Typically, the reaction time may vary from few minutes to several hours.

This process step is usually performed under standard pressure conditions. It is, however, also possible to perform this process step under reduced pressure conditions to achieve complete conversion of the reactants into the product.

**PROCESS STEP (b):** In the step (b) of the process, the compound of formula III is cyclized with hydrazine of formula VIII to obtain a compound of formula IIA; 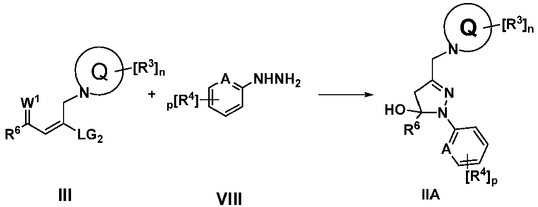 wherein, R³, R⁴, R⁶, A, n, p, Q, W¹, and LG₂ are each as defined herein above.

Consequently, the process of the present invention is non-obvious and inventive in light of the technical as well as economical advantage of first attaching the compound of formula VII with the compound of formula IV as shown in the process step (a) and then executing cyclization reaction as shown in the process step (b) not only reduces the number of steps but also facilitates the synthesis of the compound of formula I in a single pot. By virtue of reduction in number of steps for the synthesis of the compound of formula I in a single pot or multi pot the overall yield obtained is higher with reduced time, labor and operational cost.

Thus, though the compound of formula III formed in the process step (a) or the compound of formula IIA formed in the process step (b) can be isolated by suitable workup and optionally further purification, it is also possible to proceed without these compounds being isolated for the reason of yield and operational efficacy.

Particularly, definitions of the substituents of the compounds of formula III and VIII used in the process step (b) and the compound of formula IIA obtained in the process step (b) are as follows:
R⁶ is CX₃ or C(=O)W²R⁷,
   wherein, R⁷ is selected from the group consisting of hydrogen, C₁-C₆-alkyl, C₃-C₆-cycloalkyl, aryl and arylalkyl groups;
W¹ and W² are O; LG₂ is X or OR¹²; R¹² is C₁-C₆-alkyl; R³ is C₁-C₆-haloalkyl; n is an integer 1; Q is a 3-, 4- or 5 membered heterocyclic ring; A is N; R⁴ is X; p is an integer 1 or 2; and each X is independently F, Cl, Br or I.

More particularly, definitions of the substituents of the compounds of formula III and VIII used in the process step (b) and the compound of formula IIA obtained in the process step (b) are as follows:
R⁶ is CCl₃, CBr₃, C(=O)W²CH₃, C(=O)W²C₂H₅; W¹ and W² are O; LG₂ is Cl, Br, I, OCH₃, or OC₂H₅; R³ is trifluoroalkyl; n is an integer 1; Q is a tetrazole ring; A is N; R⁴ is Cl; and p is an integer 1.

The process step (b) is carried out in the presence of one or more suitable solvent and optionally, in the presence of one or more suitable reagent and or one or more suitable catalyst under particular reaction conditions.

The wordings "n is an integer 1" and "p is an integer 1" are sounding strange.

The suitable solvent useful for the purpose of the process step (b) is preferably selected from the group consisting of acetone, acetonitrile, ethyl alcohol, acetic acid, methyl tert-butyl ether, chlorobenzene, dichloroethane, dichloromethane, dioxane, dimethylformamide, dimethylacetamide, dimethyl sulfoxide, ethyl acetate, 2-metyltetrahedrafuran, tetrahydrofuran, 1,2-dimethoxyether, toluene, xylene and N-methyl-2-pyrrolidone.

The suitable reagent useful for the purpose of the process step (b) is preferably selected from the group consisting of acetic acid, trifluoroacetic acid, hydrochloric acid, hydrobromic acid, sulphuric acid, methanesulfonic acid, triflic acid, phosphoric acid and p-toluenesulfonic acid.

The process step (b) of the present invention can be performed particularly within a temperature range from 20 °C to 150 °C.

The reaction time is not critical and depends on the batch size, temperature, reagent and solvent employed. Typically, the reaction time may vary from few minutes to several hours.

The process step (b) of the present invention is usually performed under standard pressure conditions. It is, however, also possible to perform the process step (b) under reduced pressure conditions for regioselective cyclization proceeding into the compound of formula IIA.

**PROCESS STEP (c):** In the process step (c) of the present invention, the compound of formula IIA is converted into the compound of formula IIB by eliminating water with one or more suitable dehydrating reagent; 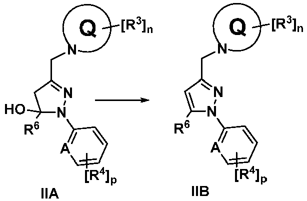 wherein, R³, R⁴, R⁶, A, n, p, and Q are each as defined above.

For elimination of water leading to the formation of the compound of formula IIB from the compound of formula IIA the following dehydrating reagents are useful: sulphuric acid, trifluoroacetic acid, phosphorous trichloride, phosphorous oxychloride, thionyl chloride, acetic anhydride, trifluoroacetic anhydride, oxalyl chloride, phosgene, diphosgene, methanolic hydrochloric acid, hydrogen chloride gas, acetic acid, hydrogen bromide, triflic acid, methanesulfonic acid, p-toluenesulfonic acid, hydrogen chloride-1,4-dioxane and silica gel.

Particularly, sulphuric acid, trifluoroacetic acid, thionyl chloride, trifluoroacetic anhydride, oxalyl chloride, methanolic hydrochloric acid, hydrogen chloride gas, acetic acid, hydrogen bromide, triflic acid, methanesulfonic acid, p-toluenesulfonic acid, hydrogen chloride-1,4-dioxane and silica gel are used for elimination of water from the compound of formula IIA.

The suitable solvent useful for the purpose of step (c) is preferably selected from the group consisting of acetonitrile, methyl tert-butyl ether, dichloromethane, dioxane, thionyl chloride, acetic acid, methyl alcohol, ethyl alcohol, tetrahydrofuran, isopropyl alcohol and tert-butyl alcohol.

The elimination of water is performed within a temperature range of 20 °C to 150 °C, more preferably to 25 °C to 100 °C.

The reaction time is not critical and depends on the batch size, temperature, reagent and solvent employed. Typically, the reaction time may vary from few minutes to several hours.

The process of elimination of water is usually performed under standard pressure conditions. It is, however, also possible to perform elimination of water under reduced pressure or elevated pressure conditions. The water can also be eliminated thermally.

**PROCESS STEP (d):** In the process step (d), the compound of formula IIB, obtained after elimination of water from the compound of formula IIA, is converted into the compound of formula II; 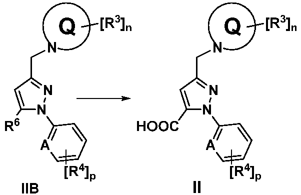 wherein, R³, R⁴, R⁶, A, n, p, and Q are each as defined above.

The process step (d) can be performed in water only, without addition of acid or base. The process step (d) can also be performed under acidic or basic conditions.

Acidic condition is maintained by using mineral acids such as sulphuric acid, chlorosulphuric acid, hydrochloric acid, hydrofluoric acid, hydroboric acid, and phosphoric acid; or organic acids such as acetic acid, trifluoroacetic acid, p-toluenesulphonic acid, methanesulphonic acid, and trifluoromethanesulphonic acid.

Basic condition is maintained by using organic bases such as trialkylamines, pyridine, alkylpyridines, phosphazines and 1,8-diazabicyclo[5.4.0]undecene (DBU) or by using inorganic bases such as alkali metal hydroxides, for example lithium, sodium or potassium hydroxide; alkali metal carbonates such as sodium carbonate, and potassium carbonate; acetates such as sodium acetate, potassium acetate, and lithium acetate; and alkoxides such as sodium methoxide, sodium ethoxide, sodium tert-butoxide, and potassium tert-butoxide.

This reaction can be accelerated by the addition of catalysts such as ferric chloride (FeCl₃), aluminum chloride (AlCl₃), boron trifluoride (BF₃), antimony trichloride (SbCl₃) and monosodium phosphate (NaH₂PO₄).

The suitable solvent useful for the purpose of the process step (d) is selected from the group consisting of water, acetonitrile, dioxane, acetic acid, methyl alcohol, ethyl alcohol, tetrahydrofuran, isopropyl alcohol and tert-butyl alcohol.

This reaction is performed within a temperature range of 20 °C to 150 °C, more preferably to 25 °C to 100 °C.

The reaction time is not critical and depends on the batch size, temperature, reagent and solvent employed. Typically, the reaction time may vary from few minutes to several hours.

This reaction is usually performed under standard pressure conditions. It is, however, also possible to perform this reaction under reduced pressure or elevated pressure conditions.

Though the compounds of formula IIA obtained in step (b), IIB in step (c), and II obtained in step (d) can be isolated by suitable workup and optionally further purification, it is also possible to proceed without these compounds being isolated for the reason of yield and operational efficacy.

Particularly, definitions of the substituents of the compounds of formula IIA used in the process step (c) and the compounds of formula IIB and II obtained in the process step (c) are as follows:
R⁶ is CX₃ or C(=O)W²R⁷,
   wherein, R⁷ is selected from the group consisting of hydrogen, C₁-C₆-alkyl, C₃-C₆-cycloalkyl, aryl and arylalkyl groups;
W² is O; R³ is C₁-C₆-haloalkyl; n is an integer 1; Q is a 3-, 4- or 5 membered heterocyclic ring; A is N; R⁴ is X; p is an integer 1 or 2; and each X is independently F, Cl, Br or I.

More particularly, definitions of the substituents of the compounds of formula III and VIII used in the process step (b) and the compound of formula IIA obtained in the process step (b) are as follows:
R⁶ is CCl₃, CBr₃, C(=O)W²CH₃, C(=O)W²C₂H₅; W² is O; R³ is trifluoroalkyl; n is an integer 1; Q is a tetrazole ring; A is N; R⁴ is Cl; and p is an integer 1.

Alternatively, the compound of formula IIB can be prepared according to the reaction scheme depicted below: 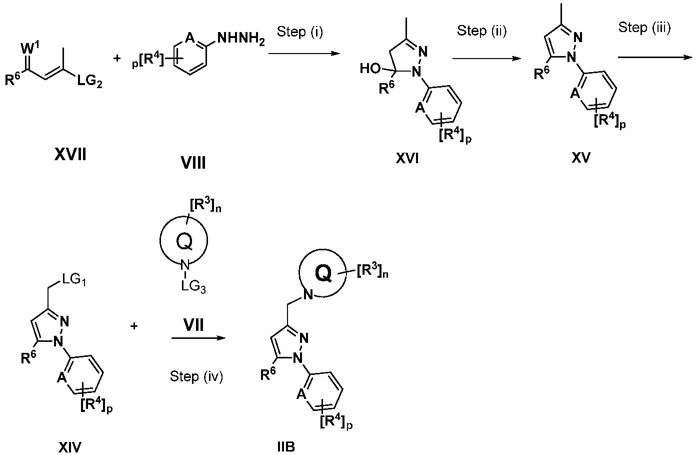 wherein, the definition of R³, R⁴, R⁶, R⁷, R⁹, R¹⁰, A, A¹, n, p, Q, W¹, W², W³, W⁴, X, LG₁, LG₂ and LG₃ are each as defined above.

Particularly, definitions of the substituents of the compounds of formula in the above reaction scheme are as follows:
R⁶ is CX₃ or C(=O)W²R⁷,
   wherein, R⁷ is selected from the group consisting of hydrogen, C₁-C₆-alkyl, C₃-C₆-cycloalkyl, aryl and arylalkyl groups;
W¹ and W² are O; LG₂ is X or OR¹²; R¹² is C₁-C₆-alkyl; LG₃ is hydrogen or alkali metal; R³ is C₁-C₆-haloalkyl; n is an integer 1; Q is a 3-, 4- or 5 membered heterocyclic ring; A is N; R⁴ is X; p is an integer 1 or 2; and each X is independently F, Cl, Br or I.

More particularly, definitions of the substituents of the compounds of formula in the above reaction scheme are as follows:
R⁶ is CCl₃, CBr₃, C(=O)W²CH₃, C(=O)W²C₂H₅; W¹ and W² are O; LG₂ is Cl, Br, I, OCH₃, or OC₂H₅; LG₃ is hydrogen or sodium metal ion; R³ is trifluoroalkyl; n is an integer 1; Q is a tetrazole ring; A is N; R⁴ is Cl; and p is an integer 1.

**PROCESS STEP (i):** The process step (i) is carried out in the presence of one or more suitable solvent and optionally, in the presence of one or more suitable reagent and or one or more suitable catalyst under particular reaction conditions.

The suitable solvent useful for the purpose of the process step (i) is selected preferably from the group consisting of acetone, acetonitrile, ethyl alcohol, methyl tert-butyl ether, chlorobenzene, dichloroethane, dichloromethane, dioxane, dimethylformamide, dimethylacetamide, dimethyl sulfoxide, ethyl acetate, 2-metyltetrahedrafuran, tetrahydrofuran, 1,2-dimethoxyether, toluene, xylene and N-methyl-2-pyrrolidone.

The suitable reagent useful for the purpose of the process step (i) is selected preferably from the group consisting of acetic acid, trifluoroacetic acid, hydrochloric acid, hydrobromic acid, sulphuric acid, methanesulfonic acid, triflic acid, phosphoric acid and p-toluenesulfonic acid.

The process step (i) of the present invention can be performed particularly within a temperature range from 20 °C to 150 °C.

The reaction time is not critical and depends on the batch size, temperature, reagent and solvent employed. Typically, the reaction time may vary from few minutes to several hours.

The process step (i) of the present invention is usually performed under standard pressure conditions. It is, however, also possible to perform the process step (i) under reduced pressure conditions for regioselective cyclization proceeding into the compound of formula XVI.

**PROCESS STEP (ii):** The compound of formula XVI is converted into the compound of formula XV by elimination of water in the process step (ii).

For elimination of water the reagent/s selected preferably from the group consisting of sulphuric acid, trifluoroacetic acid, phosphorous trichloride, phosphorous oxychloride, thionyl chloride, acetic anhydride, trifluoroacetic anhydride, oxalyl chloride, phosgene and diphosgene is/are useful.

Particularly, trifluoroacetic anhydride, thionyl chloride, oxalyl chloride and phosgene are used for elimination of water from the compound of formula XVI.

The suitable solvent useful for the purpose of step (ii) is selected preferably from the group consisting of acetonitrile, methyl tert-butyl ether, dichloromethane, dioxane, thionyl chloride, acetic acid, methyl alcohol, ethyl alcohol, tetrahydrofuran, isopropyl alcohol and tert-butyl alcohol.

The elimination of water is performed within a temperature range of 20 °C to 150 °C, more preferably to 25 °C to 100 °C.

The reaction time is not critical and depends on the batch size, temperature, reagent and solvent employed. Typically, the reaction time may vary from few minutes to several hours.

The process of elimination of water is usually performed under standard pressure conditions. It is, however, also possible to perform elimination of water under reduced pressure or elevated pressure conditions. The water can also be eliminated thermally.

**PROCESS STEP (iii):** In the process step (iii), the compound of formula XIV is obtained from the compound of formula XV.

For instance, XIV wherein LG₁ is halogen, is obtained by halogenating the compound of formula XV using one or more halogenating agent selected from the group consisting of N-halosuccinimide, X₂, X₂/hv, N-halosaccharine, and halohydantoine, optionally in the presence of a radical initiator.

X₂ is Cl₂, Br₂ or I₂ and hv indicates that the halogenating reaction is carried out in the presence of light.

N-halosuccinimide is selected from the group consisting of N-chlorosuccinimide, N-bromosuccinimide and N-iodosuccinimide. Preferably, N-halosuccinimide is N-chlorosuccinimide and N-bromosuccinimide.

N-halosaccharine is selected from the group consisting of N-chlorosaccharine, N-bromosaccharine and N-iodosaccharine. Preferably, N-halosaccharine is N-chlorosaccharine and N-bromosaccharine.

N-halohydantoine is selected from the group consisting of N-chlorohydantoine, N-bromohydantoine and N-iodohydantoine. Preferably, N-halohydantoine is N-chlorohydantoine and N-bromohydantoine.

Non-limiting examples of radical initiators useful for halogenating the compound of formula XV include dibenzoyl peroxide, hydrogen peroxide, di(n-propyl)peroxydicarbonate, t-butyl peroxybenzoate, methyl ethyl ketone peroxide, 2,5-dimethyl-2,5-di(t-butylperoxy)-3-hexyne, di(t-butyl)peroxide, acetone peroxide, dicumyl peroxide, azobisisobutyronitrile, bis(2-ethylhexyl)peroxydicarbonate, (peroxybis(propane-2,2-diyl))dibenzene, peracetic acid, metachloroperbenzoic acid, Payne's reagent, magnesium monoperphthalate, trifluoroperacetic acid, trichloroperacetic acid, 2, 4-dinitorperbenzoic acid, Caro's Acid and potassium caroate.

The process step (iii) is carried out in the presence of one or more suitable solvent(s) and optionally, in the presence of one or more suitable reagent(s) and or one or more suitable catalyst(s) under particular reaction conditions.

The suitable solvent useful for the purpose of step (iii) is selected from the group consisting of acetone, acetonitrile, ethyl alcohol, methyl tert-butyl ether, chlorobenzene, dichloroethane, dichloromethane, dioxane, ethyl acetate, dimethylformamide, dimethylacetamide, dimethyl sulfoxide, 2-methyltetrahydrofuran, tetrahydrofuran, 1,2-dimethoxyether, toluene, p-xylene and N-methyl-2-pyrrolidone.

The process step (iii) of the present invention can be performed particularly within a temperature range from 20 °C to 150 °C.

The reaction time is not critical and depends on the batch size, temperature, reagent and solvent employed. Typically, the reaction time may vary from few minutes to several hours.

The process step (iii) is usually performed under standard pressure conditions. It is, however, also possible to perform the process step (iii) under reduced pressure or elevated pressure conditions.

**PROCESS STEP (iv):** The process step (iv) is carried out in the presence of one or more suitable solvent(s) and optionally, in the presence of one or more suitable reagent(s) and or one or more suitable catalyst(s) under particular reaction conditions. The process step (iv) is highly regioselective. For example, the product IIB-1 is formed regioselectively over the product IIB-2.

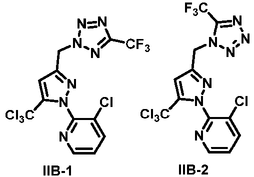

The suitable solvent useful for the purpose of step (iv) is selected preferably from the group consisting of acetone, acetonitrile, methyl tert-butyl ether, chlorobenzene, dichloroethane, dichloromethane, dioxane, ethyl acetate, dimethylformamide, dimethylacetamide, dimethyl sulfoxide, 2-methyltetrahydrofuran, tetrahydrofuran, 1,2-dimethoxyether, toluene, p-xylene and N-methyl-2-pyrrolidone.

The suitable catalyst for the purpose of step (iv) is preferably selected from the group consisting of potassium iodide, sodium iodide, copper iodide and cupric iodide.

The process step (iv) of the present invention can be performed particularly within a temperature range from 20 °C to 150 °C.

The reaction time is not critical and depends on the batch size, temperature, reagent and solvent employed. Typically, the reaction time may vary from few minutes to several hours.

The process step (iv) of the present invention is usually performed under standard pressure conditions. It is, however, also possible to perform the process step (iv) under reduced pressure conditions to effect complete conversion of the reactants into the product.

The compound of formula XIV, wherein LG₁ is Cl can alternatively be prepared by regioselectively cyclizing a compound of formula IV and the compound of formula VIII to provide XVIII using the process, reagent/catalyst, solvent and reaction conditions similar to that for preparing the compound of formula IIA. It is surprisingly and unexpectedly observed that the compound of formula IV and the compound of formula VIII do not cyclize to produce XVIII when LG₁ is Br or I.

Subsequently, the compound of formula XVIII is converted into the compound of formula XIV by elimination of water. The water is eliminated according to the process described for the preparation of IIB from IIA. 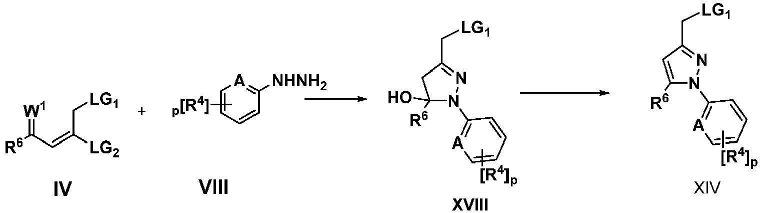 wherein, the definition of R⁴, R⁶, A, p, W¹, LG₁, and LG₂ are each as defined above.

Particularly, definitions of the substituents of the compounds of formula IV, VIII, XVIII and XIV are as follows:
R⁶ is CX₃ or C(=O)W²R⁷,
   wherein, R⁷ is selected from the group consisting of hydrogen, C₁-C₆-alkyl, C₃-C₆-cycloalkyl, aryl and arylalkyl groups;
W¹ and W² are O; LG₁ is Cl; LG₂ is X or OR¹²; R¹² is C₁-C₆-alkyl; A is N; R⁴ is X; p is an integer 1 or 2; and each X is independently F, Cl, Br or I.

More particularly, definitions of the substituents of the compounds of formula IV and VII used in the process step (a) and the compound of formula III obtained in the process step (a) are as follows:
R⁶ is CCl₃, CBr₃, C(=O)W²CH₃, C(=O)W²C₂H₅; W¹ and W² are O; LG₂ is Cl, Br, I, OCH₃, or OC₂H₅; A is N; R⁴ is Cl; and p is an integer 1.

Though the compounds of formula XVI, XV, XIV and XVIII prepared according to shown reaction scheme can be isolated by suitable workup steps and optionally further purification, it is also possible to prepare the compound of formula IIB without these compounds being isolated for the reason of yield and operational efficacy.

**PROCESS STEP (e):** In the process step (e) of the present invention, the compound of formula II, optionally after converting into a compound of formula X using a halogenating agent, is reacted with a compound of formula IX to obtain the compound of formula I; 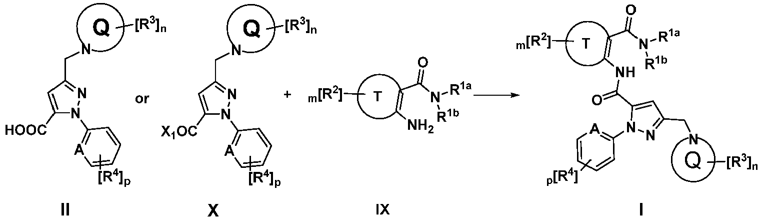 wherein, X₁ is Cl or Br; R^{1a}, R^{1b}, R², R³, R⁴, A, m, n, p, Q, and T are each as defined above.

Alternatively, the compound of formula II, optionally after converting into a compound of formula X using halogenating agent, is reacted with a compound of formula XI to obtain a compound of formula XII, and then the compound of formula XII, optionally after hydrolyzing, is reacted with an amine of formula XIII to obtain a compound of formula I, 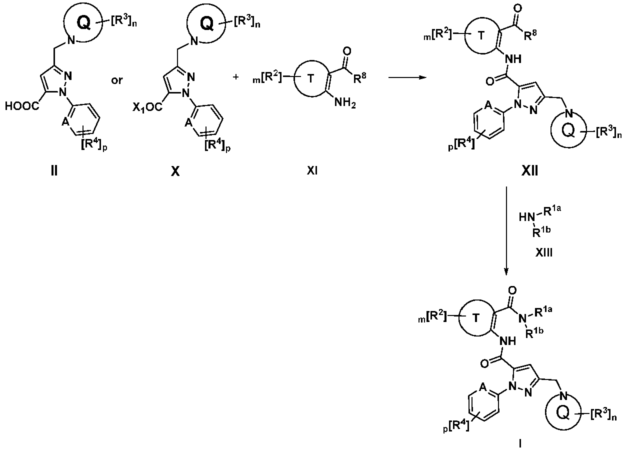 wherein, R⁸ is selected from the group consisting of hydroxy, Cl and OR⁷; R^{1a}, R^{1b} R², R³, R⁴, R⁷, A, m, n, p, Q, T and X₁ are each as defined hereinabove.

The present invention also relates to novel and inventive intermediates formed in the process of the instant invention.

The present invention relates to a compound of formula XIX, 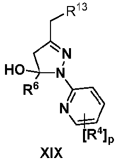 wherein, R¹³ is 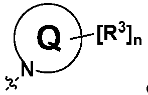 or LG₁; R³, R⁴, R⁶, n, p, and Q are each as defined herein above,
with the proviso that when R¹³ is LG₁ then R⁶ is CX₃, wherein LG₁ is Cl, Br or I; and X is F, Cl, Br, or I.

In one embodiment, the compound of formula XIX is the compound of formula IIA, 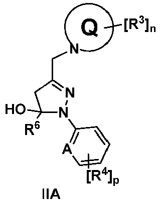 wherein, R³, R⁴, R⁶, A, n, p, and Q are each as defined hereinabove.

Particularly, definitions of the substituents of the compounds of formula IIA are as follows:
R⁶ is CX₃ or C(=O)W²R⁷,
   wherein, R⁷ is selected from the group consisting of hydrogen, C₁-C₆-alkyl, C₃-C₆-cycloalkyl, aryl and arylalkyl groups;
W² is O; R³ is C₁-C₆-haloalkyl; n is an integer 1; Q is a 5 membered heterocyclic ring; A is N; R⁴ is X; p is an integer 1 or 2; and each X is independently F, Cl, Br or I.

More particularly, definitions of the substituents of the compounds of formula IIA are as follows:
R⁶ is CCl₃, CBr₃, C(=O)W²CH₃, C(=O)W² C₂H₅; W² is O; R³ is trifluoroalkyl; n is an integer 1; Q is a tetrazole ring; A is N; R⁴ is Cl; and p is an integer 1.

In second embodiment, the compound of formula XIX is the compound of formula XVIII, 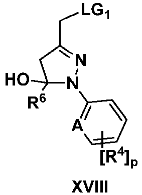 wherein, R⁴, A, p, and LG₁ are each as defined above, and R⁶ is CCl₃ or CBr₃;; LG₁ is Cl, Br or I, preferably Br; A is N; R⁴ is Cl; and p is an integer 1.

The present invention also relates to a compound of formula XX, 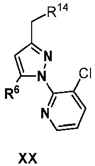
wherein, R¹⁴ is 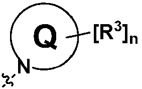 or LG₁ or hydrogen;
R⁶ is CX₃, C(W⁴R⁷)₃, CH(W⁴R⁷)₂, allylic group, substituted or unsubstituted furanyl, 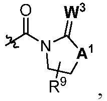 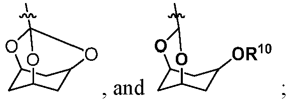
R³, R⁴, R⁷, R⁹, R¹⁰, LG₁, A, A¹, n, p, Q, W³, W⁴, X and the substituents on furanyl are each as defined herein above,
with the proviso that when R¹⁴ is LG₁ or hydrogen then R⁶ is CX₃, wherein LG₁ and X are Cl, Br, or I.

In one embodiment, the compound of formula XX is the compound of formula IIB, 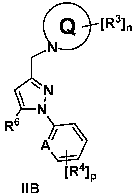 wherein, R⁶ is CX₃, C(W⁴R⁷)₃, CH(W⁴R⁷)₂, allylic group, substituted or unsubstituted furanyl, 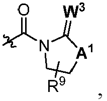 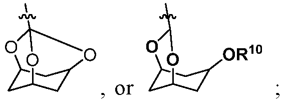 R³, R⁴, R⁷, R⁹, R¹⁰, A, A¹, n, p, Q, W³, W⁴, and X are each as defined hereinabove.

Particularly, definitions of the substituents of the compounds of formula IIA are as follows:
R⁶ is CX₃, R³ is C₁-C₆-haloalkyl; n is an integer 1; Q is a 5 membered heterocyclic ring; A is N; R⁴ is X; p is an integer 1 or 2; and each X is independently F, Cl, Br or I.

More particularly, definitions of the substituents of the compounds of formula IIA are as follows:
R⁶ is CCl₃ or CBr₃; R³ is trifluoroalkyl; n is an integer 1; Q is a tetrazole ring; A is N; R⁴ is Cl; and
p is an integer 1.

In second embodiment, the compound of formula XX is the compound of formula XV, 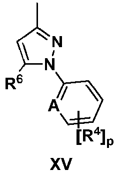 wherein, R⁶ is CX₃, C(W⁴R⁷)₃, CH(W⁴R⁷)₂, allylic group, substituted or unsubstituted furanyl, 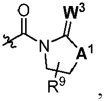 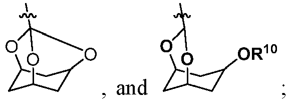 A is N; and R⁴, R⁷, R⁹, R¹⁰, A¹, p, W³, W⁴, and X are each as defined hereinabove.

Particularly, definitions of the substituents of the compounds of formula XV are as follows:
R⁶ is CX₃, A is N; R⁴ is X; p is an integer 1 or 2; and each X is independently F, Cl, Br or I.

More particularly, definitions of the substituents of the compounds of formula XV are as follows:
R⁶ is CCl₃ or CBr₃; A is N; R⁴ is Cl; and p is an integer 1.

In third embodiment, the compound of formula XX is the compound of formula XIV, 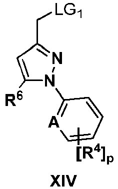 wherein, R⁶ is CX₃, X is F, Cl, Br and I; R⁴, A, and p and LG₁ are each as defined hereinabove.

Particularly, definitions of the substituents of the compounds of formula XIV are as follows:
R⁶ is CX₃, LG₁ is X; A is N; R⁴ is X; p is an integer 1 or 2; and each X is independently F, Cl, Br or I.

More particularly, definitions of the substituents of the compounds of formula XIV are as follows:
R⁶ is CCl₃ or CBr₃; LG₁ is Cl, Br or I, preferably Br; A is N; R⁴ is Cl; and p is an integer 1.

The present invention further relates to the compound of formula III, 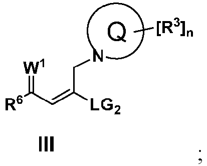 wherein, Q is a 3-, 4- or 5 membered heterocyclic ring excluding triazole ring; R³, R⁶, n, W¹, and LG₂ are each as defined in herein above.

Particularly, definitions of the substituents of the compounds of formula III are as follows:
R⁶ is CX₃ or C(=O)W²R⁷,
   wherein, R⁷ is selected from the group consisting of hydrogen, C₁-C₆-alkyl, C₃-C₆-cycloalkyl, aryl and arylalkyl groups;
W¹ & W² are O; LG₂ is X or OR¹²; R¹² is C₁-C₆-alkyl; R³ is C₁-C₆-haloalkyl; n is an integer 1; Q is a 5 membered heterocyclic ring; and each X is independently F, Cl, Br or I.

More particularly, definitions of the substituents of the compounds of formula III are as follows:
R⁶ is CCl₃, CBr₃, C(=O)W²CH₃, C(=O)W²C₂H₃; W¹ and W² are O; LG₂ is Cl, Br, I, OCH₃, or OC₂H₅; R³ is trifluoroalkyl; n is an integer 1; and Q is a tetrazole ring.

The present disclosure still further relates to the compound of formula IV-2,

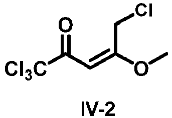

The present invention still further relates to the compound of formula XVI, 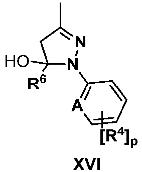 wherein, R⁶ is CX₃, C(W⁴R⁷)₃, CH(W⁴R⁷)₂, allylic group, substituted or unsubstituted furanyl, 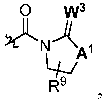 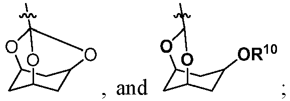 A is N; and R⁴, R⁷, R⁹, R¹⁰, A¹, p, W³, W⁴, and X are each as defined hereinabove.

Particularly, definitions of the substituents of the compounds of formula XVI are as follows:
R⁶ is CX₃; A is N; R⁴ is X; p is an integer 1 or 2; and each X is independently F, Cl, Br or I.

More particularly, definitions of the substituents of the compounds of formula XVI are as follows:
R⁶ is CCl₃ or CBr₃; A is N; R⁴ is Cl; and p is an integer 1.

In absence of specific mention of a solvent in a process step, the following solvents are useful in the process of the present invention aliphatic, alicyclic or aromatic hydrocarbons, for example petroleum ether, n-hexane, n-heptane, cyclohexane, methylcyclohexane, benzene, toluene, xylene or decalin; halogenated hydrocarbons, for example chlorobenzene, dichlorobenzene, dichloromethane, chloroform, tetrachloromethane, dichloroethane or trichloroethane; ethers such as diethyl ether, diisopropyl ether, methyl tert-butyl ether, methyl tert-amyl ether, dioxane, tetrahydrofuran, 2-methyl tetrahydrofuran, 1,2-dimethoxyethane, 1,2-diethoxyethane or anisole; nitriles such as acetonitrile, propionitrile, n- or isobutyronitrile or benzonitrile; amides such as N,N-dimethylformamide, N,N-dimethylacetamide, N-methylformanilide, N-methylpyrrolidone or hexanlethylphosphoranlide; sulphoxides such as dimethyl sulphoxide; sulphones such as sulpholane; alcohols such as methyl alcohol, ethyl alcohol, or isopropyl alcohol.

The present invention is further described in detail as illustrated in the non-limiting examples.

### PREPARATION EXAMPLES

### Example 1:

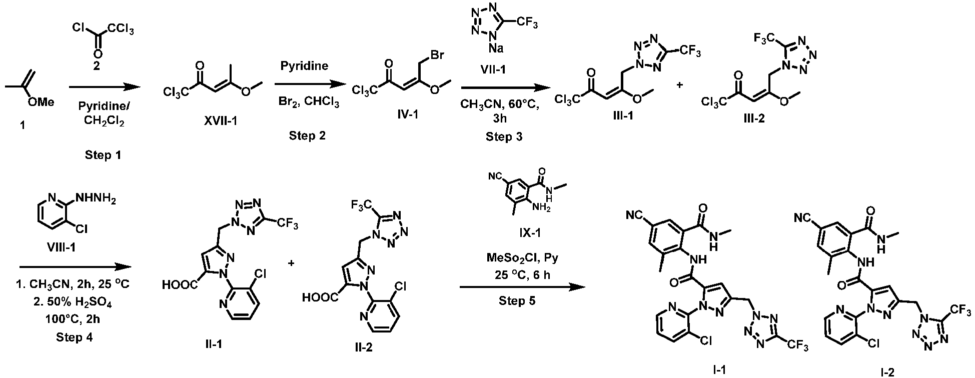

### Step-1: Synthesis of 1,1,1-trichloro-4-methoxypent-3-en-2-one (XVII-1) (ref: Synthesis 12, 1013-14, 1986)

A solution of 2,2,2-trichloroacetyl chloride (9.75 mL, 86 mmol) in dichloromethane (30 mL) was added to a stirred solution of 2-methoxyprop-1-ene (9.4 mL, 100 mmol) and pyridine (9.0 mL, 112 mmol) in dichloromethane (60 mL) at 0 °C over 30 min. The resultant mixture was stirred at 25 °C for 16 h and then diluted with dichloromethane (200 mL), washed with 10% hydrogen chloride (50 mL) and finally twice with water (200 mL). The dichloromethane layer was dried over anhydrous sodium sulphate and concentrated under reduced pressure to give crude product 1,1,1-trichloro-4-methoxypent-3-en-2-one (18 g, 83 mmol, 96 % yield).

¹H-NMR (400 MHz, CDCl₃) δ 6.00 (s, 1H), 3.79 (s, 3H), 2.40 (s, 3H)

### Step-2: Synthesis of (E/Z)-5-bromo-1,1,1-trichloro-4-methoxypent-3-en-2-one (IV-1) (ref: Synthesis 16, 2353-2358, 2002; WO2011009551)

A solution of Br (1.9 mL, 37 mmol) in dichloromethane (10 mL) was added drop wise to a stirred solution of 1,1,1-trichloro-4-methoxypent-3-en-2-one (8 g, 37 mmol) in dichloromethane (50 mL) at 0 °C. The reaction mixture was stirred at 0 °C for 15 min followed by addition of a solution of pyridine (3 mL, 37 mmol) in dichloromethane (10 mL) at 0 °C. After completion of the reaction, the reaction mixture was partitioned between water (100 mL) and dichloromethane (100 mL). The dichloromethane layer was washed twice with 2N aqueous hydrogen chloride (50 mL) and then with water (100 mL). The dichloromethane layer was dried over anhydrous sodium sulphate and concentrated under reduced pressure to get crude product, which was purified by flash chromatography using 10% ethyl acetate in hexane as eluent to afford desired product 5-bromo-1,1,1-trichloro-4-methoxypent-3-en-2-one (6.6 g, 22 mmol, 60 % yield).

¹H-NMR (400 MHz, CDCl₃) δ 6.09 (s, 1H), 4.50 (s, 2H), 3.90 (s, 3H)

### Step-3: Synthesis of 1,1,1-trichloro-4-methoxy-5-(5-(trifluoromethyl)-2H-tetrazol-2-yl)pent-3-en-2-one (III-1) and 1,1,1-trichloro-4-methoxy-5-(5-(trifluoromethyl)-1H-tetrazol-1-yl)pent-3-en-2-one (III-2)

Sodium 5-(trifluoromethyl)tetrazol-1-ide (1.3 g, 8.0 mmol) was added at once to a stirred solution of 5-bromo-1,1,1-trichloro-4-methoxypent-3-en-2-one (2.0 g, 6.7 mmol) in acetonitrile (30 mL) at 25 °C. The reaction mixture was heated to 60 °C for 3 h under stirring. After completion of the reaction, the reaction mixture was cooled to 25 °C and was partitioned between water (20 mL) and ethyl acetate (50 mL). The ethyl acetate layer was washed subsequently with water (25 mL) and brine solution (25 mL), dried over anhydrous sodium sulphate and concentrated under reduced pressure to get the crude product which was purified by flash chromatography using 20% ethyl acetate in hexane as eluent to get pure desired product 1,1,1-trichloro-4-methoxy-5-(5-(trifluoromethyl)-2H-tetrazol-2-yl)pent-3-en-2-one and 1,1,1-trichloro-4-methoxy-5-(5-(trifluoromethyl)-1H-tetrazol-1-yl)pent-3-en-2-one (1.7 g, 5.0 mmol, 81% yield).

¹H-NMR (400 MHz, CDCl₃) δ 6.28 (s, 1H), 6.15 (d, J = 0.7 Hz, 2H), 3.84 (s, 3H); LCMS: [352.80]^{M+H}

### Step-4: Synthesis of 1-(3-chloropyridin-2-yl)-3-((5-(trifluoromethyl)-2H-tetrazol-2-yl)methyl)-1H-pyrazole-5-carboxylic acid (II-1) and 1-(3-chloropyridin-2-yl)-3-((5-(trifluoromethyl)-1H-tetrazol-1-yl)methyl)-1H-pyrazole-5-carboxylic acid (II-2)

3-Chloro-2-hydrazinylpyridine (0.2 g, 1.4 mmol) was added to a stirred solution of 1,1,1-trichloro-4-methoxy-5-(5-(trifluoromethyl)-2H-tetrazol-2-yl)pent-3-en-2-one (0.5 g, 1.4 mmol) in acetonitrile (5 mL) at 25 °C. The reaction mixture was stirred for 2 h at the same temperature. Then the reaction mixture was cooled to 0 °C and 50% aqueous sulphuric acid solution (6 mL) was added. The reaction mixture was heated with stirring to 100 °C for 2 h, cooled to 25 °C and then poured into ice-cold water. The mixture was extracted twice with ethyl acetate (20 mL), dried over anhydrous sodium sulphate and concentrated under reduced pressure to obtain the crude product, which was triturated with hexane and dried under reduced pressure to obtain 1-(3-chloropyridin-2-yl)-3-((5-(trifluoromethyl)-2H-tetrazol-2-yl)methyl)-1H-pyrazole-5-carboxylic acid and 1-(3-chloropyridin-2-yl)-3-((5-(trifluoromethyl)-1H-tetrazol-1-yl)methyl)-1H-pyrazole-5-carboxylic acid (0.44 g, 1.2 mmol, 89% yield).

¹H-NMR (400 MHz, DMSO-D₆) δ 8.55 (dd, J = 4.6, 1.5 Hz, 1H), 8.23-8.28 (m, 1H), 7.68 (dd, J = 8.1, 4.6 Hz, 1H), 7.20 (s, 1H), 6.25 (s, 2H); LCMS: [373.90]^{M+H}

### Step-5: Preparation of mixture of 1-(3-chloropyridin-2-yl)-N-(4-cyano-2-methyl-6-(methylcarbamoyl)phenyl)-3-((5-(trifluoromethyl)-2H-tetrazol-2-yl)methyl)-1H-pyrazole-5-carboxamide (I-1) and 1-(3-chloropyridin-2-yl)-N-(4-cyano-2-methyl-6-(methylcarbamoyl)phenyl)-3-((5-(trifluoromethyl)-1H-tetrazol-1-yl)methyl)-1H-pyrazole-5-carboxamide (I-2):

Methanesulfonyl chloride (CH₃SO₂Cl; 520 mg, 4 mmol) was added drop wise to a solution containing the compound II-1, the compound II-2 (1.0 g, 2.7 mmol) and pyridine (570 mg, 7.2 mmol) in dimethyl acetamide (10 mL) at 0 °C. Then, a compound IX-1 was added portion wise over 2 min to the reaction mixture. The resulting reaction mixture was heated to 50 °C with stirring for 4 h. The reaction mixture was then cooled to 25 °C and was poured on to crushed ice (50 mL). The precipitate was filtered and washed with cold water (50 mL) to get a mixture of products I-1 and I-2 (1.38 g, 2.56 mmol, 95%).

### Example 2:

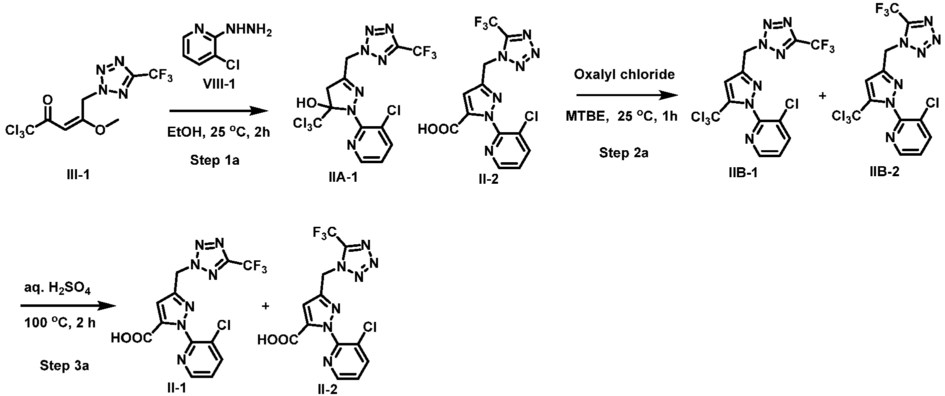

### Step-1a: 1-(3-chloropyridin-2-yl)-5-(trichloromethyl)-3-((5-(trifluoromethyl)-2H-tetrazol-2-yl)methyl)-4,5-dihydro-1H-pyrazol-5-ol (IIA-1)

A solution of 3-chloro-2-hydrazinylpyridine (0.2 g, 1.4 mmol) and 1,1,1-trichloro-4-methoxy-5-(5-(trifluoromethyl)-2H-tetrazol-2-yl)pent-3-en-2-one (0.5 g, 1.4 mmol) in ethyl alcohol (10 mL) was stirred at 25 °C for 2 h. After completion of the reaction, ethyl alcohol was removed under reduced pressure to get a crude product, which was purified by flash chromatography using 30% ethyl acetate in hexane as eluent, to get the desired product 1-(3-chloropyridin-2-yl)-5-(trichloromethyl)-3-((5-(trifluoromethyl)-2H-tetrazol-2-yl)methyl)-4,5-dihydro-1H-pyrazol-5-ol (0.57 g, 1.2 mmol, 87% yield).

¹H-NMR (400 MHz, CDCl₃) δ 9.80-9.08 (1H), 8.17 (dd, J = 4.9, 1.7 Hz, 1H), 7.86 (dd, J = 8.1, 1.7 Hz, 1H), 7.13-7.17 (m, 1H), 5.71 (s, 2H), 3.84 (d, J = 19.1 Hz, 1H), 3.34 (d, J = 19.1 Hz, 1H); LCMS: [463.8]^{M-H}

### Step-2a: 3-chloro-2-(5-(trichloromethyl)-3-((5-(trifluoromethyl)-2H-tetrazol-2-yl)methyl)-1H-pyrazol-1-yl)pyridine (IIB-1)

Oxalyl chloride (0.17 mL, 1.9 mmol) was added drop wise to a solution of 1-(3-chloropyridin-2-yl)-5-(trichloromethyl)-3-((5-(trifluoromethyl)-2H-tetrazol-2-yl)methyl)-4,5-dihydro-1H-pyrazol-5-ol (0.3 g, 0.6 mmol) in methyl tert-butyl ether (3 mL) at 25 °C. The reaction mixture was stirred for 1 h at 25 °C. After completion of the reaction, volatiles were removed from the reaction mixture under reduced pressure to get a crude product, which was purified by flash chromatography using 20% ethyl acetate in hexane as eluent to get the desired product 3-chloro-2-(5-(trichloromethyl)-3-((5-(trifluoromethyl)-2H-tetrazol-2-yl)methyl)-1H-pyrazol-1-yl)pyridine (0.27 g, 0.6 mmol, 94% yield).

¹H-NMR (400 MHz, CDCl₃) δ 8.57 (dd, J = 4.6, 1.7 Hz, 1H), 7.97 (dd, J = 8.1, 1.7 Hz, 1H), 7.50-7.54 (m, 1H), 7.03 (s, 1H), 5.98 (s, 2H); LCMS: [448]^{M+H}

### Step-3a: 1-(3-chloropyridin-2-yl)-3-((5-(trifluoromethyl)-2H-tetrazol-2-yl)methyl)-1H-pyrazole-5-carboxylic acid (II-1)

A suspension of 3-chloro-2-(5-(trichloromethyl)-3-((5-(trifluoromethyl)-2H-tetrazol-2-yl)methyl)-1H-pyrazol-1-yl)pyridine (0.25 g, 0.56 mmol) in 50% aqueous sulphuric acid solution (5 mL) was heated at 100 °C with stirring for 2 h. After completion of the reaction, the reaction mixture was cooled to 0 °C, and was diluted with ice-cold water (10 mL) to get a precipitate, which was filtered and dried to get the desired product 1-(3-chloropyridin-2-yl)-3-((5-(trifluoromethyl)-2H-tetrazol-2-yl)methyl)-1H-pyrazole-5-carboxylic acid (140 mg, 0.4 mmol, 67.0% yield).

¹H-NMR (400 MHz, DMSO-D₆) δ 8.55 (dd, J = 4.6, 1.5 Hz, 1H), 8.23-8.28 (m, 1H), 7.68 (dd, J = 8.1, 4.6 Hz, 1H), 7.20 (s, 1H), 6.25 (s, 2H); LCMS: [373.9]^{M-H}

### Example 3 (reference):

### Synthesis of sodium 5-(trifluoromethyl)tetrazol-1-ide (VII-1)

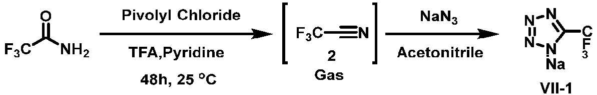

To a solution of trifluoroacetic acid (4.8 mL, 62 mmol) in pyridine (72 mL, 890 mmol) was added 2,2,2-trifluoroacetamide (20 g, 177 mmol). Subsequently, trimethylacetyl chloride (47 g, 391 mmol) was added drop wise at 25 °C to this solution over 3 h. The gaseous trifluoroacetonitrile formed in the course of drop wise addition was purged into another container containing a solution of sodium azide (17 g, 260 mmol) in acetonitrile (100 mL) at 25 °C. The reaction mixture was allowed to stir for 24 h. The resultant solids were filtered and washed with acetonitrile (50 mL), the filtrate was concentrated under reduced pressure to obtain sodium 5-(trifluoromethyl)tetrazol-1-ide (15 g, 94 mmol, 53.0% yield).

⁹F-NMR (400 MHz, DMSO-D₆) δ -59.60 (s, 3F)

A similar process is also disclosed in an article by Crawford, Margaret-J. et al., Journal of F Chemistry, 129(12), 1199-1205; 2008.

### Example 4:

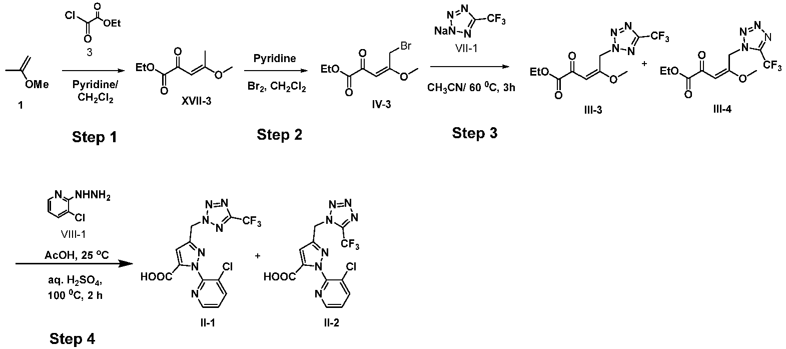

### Step-1: Synthesis of ethyl 4-methoxy-2-oxopent-3-enoate (XVII-2).

To a stirred solution of 2-methoxyprop-1-ene (13.0 mL, 139 mmol) in dichloromethane (170 mL), was added pyridine (11 mL, 139 mmol) at 0 °C, followed by addition of a solution of ethyl 2-chloro-2-oxoacetate (15.5 mL, 139 mmol) in dichloromethane (30 mL) at 0 °C. The reaction mixture was stirred at 25 °C for 12 h. After the completion of the reaction, the reaction mixture was diluted with dichloromethane (200 mL) and washed successively with water (100 mL), 10% hydrogen chloride (50 mL) and brine solution (50 mL). The dichloromethane layer was dried over anhydrous sodium sulphate and concentrated under reduced pressure to obtain ethyl 4-methoxy-2-oxopent-3-enoate (22.0 g, 128 mmol, 93% yield).

¹H-NMR (400 MHz, CDCl₃) δ 6.22 (s, 1H), 4.25-4.38 (m, 2H), 3.76 (s, 3H), 2.36 (s, 3H), 1.25-1.40 (m, 3H); LCMS: [173.15]^{M+H}

### Step-2: Synthesis of ethyl 5-bromo-4-methoxy-2-oxopent-3-enoate (IV-3).

A solution of Br (1.5 mL, 29.0 mmol) in dichloromethane (20 mL) was added to a stirred solution of ethyl 4-methoxy-2-oxopent-3-enoate (5.0 g, 29.0 mmol) in dichloromethane (50 mL) at 0 °C and was stirred at 0 °C for 15 min. Then, a solution of pyridine (2.3 mL, 29.0 mmol) in dichloromethane (20 mL) was added drop wise to the reaction mixture. After completion of the reaction, the reaction mixture was diluted with dichloromethane (100 mL) and washed with 2N hydrogen chloride (40 mL). The dichloromethane layer was dried over anhydrous sodium sulphate and concentrated under reduced pressure to get a crude product, which was purified by flash chromatography using 10% ethyl acetate in hexane as eluent to afford the desired product ethyl 5-bromo-4-methoxy-2-oxopent-3-enoate (5.0 g, 19.91 mmol, 69% yield).

¹H-NMR (400 MHz, CDCl₃) δ 6.36 (s, 1H), 4.48 (s, 2H), 4.28-4.42 (m, 2H), 3.86 (s, 3H), 1.31-1.48 (m, 3H); LCMS: [252.75]^{M+2}

### Step-3: Synthesis of ethyl 4-methoxy-2-oxo-5-(5-(trifluoromethyl)-2H-tetrazol-2-yl)pent-3-enoate (III-3) and ethyl 4-methoxy-2-oxo-5-(5-(trifluoromethyl)-1H-tetrazol-1-yl)pent-3-enoate (III-4)

Sodium 5-(trifluoromethyl)tetrazol-1-ide (1.5 g, 9.6 mmol) was added to a stirred solution of ethyl 5-bromo-4-methoxy-2-oxopent-3-enoate (2.0 g, 8.0 mmol) in acetonitrile (20 mL) at 25 °C and the resulting reaction mixture was heated at 60 °C for 3 h. After the completion of the reaction, the reaction mixture was diluted with water and extracted thrice with ethyl acetate (30 mL). The ethyl acetate layer was washed with brine solution (30 mL), dried over anhydrous sodium sulphate and concentrated under reduced pressure to get a crude product, which was purified by flash to obtain the desired product ethyl 4-methoxy-2-oxo-5-(5-(trifluoromethyl)-2H-tetrazol-2-yl)pent-3-enoate (2.0 g, 6.5 mmol, 87% yield).

¹H-NMR (400 MHz, CDCl₃) δ 6.55 (s, 1H), 6.10 (s, 2H), 4.34-4.42 (m, 2H), 3.79 (s, 3H), 1.28-1.44 (m, 3H); LCMS: [309.10]^{M+H}

### Step-4: Synthesis of 1-(3-chloropyridin-2-yl)-3-((5-(trifluoromethyl)-2H-tetrazol-2-yl)methyl)-1H-pyrazole-5-carboxylic acid (II-1) and 1-(3-chloropyridin-2-yl)-3-((5-(trifluoromethyl)-1H-tetrazol-1-yl)methyl)-1H-pyrazole-5-carboxylic acid (II-2)

A solution of ethyl 4-methoxy-2-oxo-5-(5-(trifluoromethyl)-2H-tetrazol-2-yl)pent-3-enoate (0.2 g, 0.65 mmol) and 3-chloro-2-hydrazinylpyridine (0.1 g, 0.65 mmol) in glacial acetic acid (2 mL) was stirred at 25 °C for 6 h. After completion of the reaction, the reaction mixture was cooled to 0 °C and 50% sulphuric acid (3 mL) was added to it. The reaction mixture was then heated at 100 °C for 2 h. The reaction mixture was then cooled to 25 °C and poured into ice-cold water (20 mL), and was extracted twice with dichloromethane (25 mL). The combined dichloromethane layer was dried over anhydrous sodium sulphate and concentrated under reduced pressure to obtain a crude product, which was triturated with hexane, filtered and dried to obtain 1-(3-chloropyridin-2-yl)-3-((5-(trifluoromethyl)-2H-tetrazol-2-yl)methyl)-1H-pyrazole-5-carboxylic acid and 1-(3-chloropyridin-2-yl)-3-((5-(trifluoromethyl)-1H-tetrazol-1-yl)methyl)-1H-pyrazole-5-carboxylic acid (0.2 g, 0.51 mmol, 84% yield).

¹H-NMR (400 MHz, DMSO- D₆) δ 8.55 (dd, J = 4.6, 1.5 Hz, 1H), 8.23-8.28 (m, 1H), 7.68 (dd, J = 8.1, 4.6 Hz, 1H), 7.20 (s, 1H), 6.25 (s, 2H); LCMS: [373.90]^{M+H}

### Example 5:

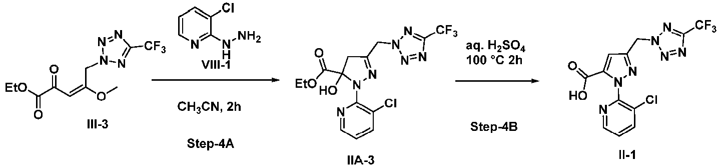

### Step-4A: Synthesis of ethyl 1-(3-chloropyridin-2-yl)-5-hydroxy-3-((5-(trifluoromethyl)-2H-tetrazol-2-yl)methyl)-4,5-dihydro-1H-pyrazole-5-carboxylate (IIA-3).

A solution of ethyl 4-methoxy-2-oxo-5-(5-(trifluoromethyl)-2H-tetrazol-2-yl)pent-3-enoate (0.25 g, 0.8 mmol) and 3-chloro-2-hydrazinylpyridine (0.1 g, 0.8 mmol) in acetonitrile (5 mL) was stirred at 25 °C for 2 h. After the completion of the reaction, the reaction mixture was diluted with water and extracted twice with ethyl acetate (20 mL). The ethyl acetate layers were combined, dried over anhydrous sodium sulphate and concentrated under reduced pressure to obtain ethyl 1-(3-chloropyridin-2-yl)-5-hydroxy-3-((5-(trifluoromethyl)-2H-tetrazol-2-yl)methyl)-4,5-dihydro-1H-pyrazole-5-carboxylate (0.3 g, 0.7 mmol, 88% yield).

¹H-NMR (400 MHz, CDCl₃) δ 7.96-8.04 (m, 1H), 7.73 (dd, J = 7.9, 1.6 Hz, 1H), 6.88-6.93 (m, 1H), 5.72-5.76 (s, 2H), 4.21-4.29 (m, 2H), 3.30-3.35 (m, 1H), 3.07-3.11 (m, 1H), 1.15-1.20 (m, 3H); LCMS: [419]^{M+H}

### Step-4B: Synthesis of 1-(3-chloropyridin-2-yl)-3-((5-(trifluoromethyl)-2H-tetrazol-2-yl)methyl)-1H-pyrazole-5-carboxylic acid (II-1).

A suspension of ethyl 1-(3-chloropyridin-2-yl)-5-hydroxy-3-((5-(trifluoromethyl)-2H-tetrazol-2-yl)methyl)-4,5-dihydro-1H-pyrazole-5-carboxylate (0.3 g, 0.7 mmol) in 50% (v/v) aqueous sulphuric acid (2.5 mL) was heated at 100 °C for 2 h under stirring. After the completion of the reaction, the reaction mixture was cooled to 25 °C and poured in ice-cold water. The resultant solid was filter and washed with water (5 mL) and dried under reduced pressure to obtain 1-(3-chloropyridin-2-yl)-3-((5-(trifluoromethyl)-2H-tetrazol-2-yl)methyl)-1H-pyrazole-5-carboxylic acid (0.22 g, 0.6 mmol, 82% yield).

¹H-NMR (400 MHz, DMSO-D₆) δ 8.55 (dd, J = 4.6, 1.5 Hz, 1H), 8.23-8.28 (m, 1H), 7.68 (dd, J = 8.1, 4.6 Hz, 1H), 7.20 (s, 1H), 6.25 (s, 2H); LCMS: [373.90]^{M+H}

### Example 6 (reference):

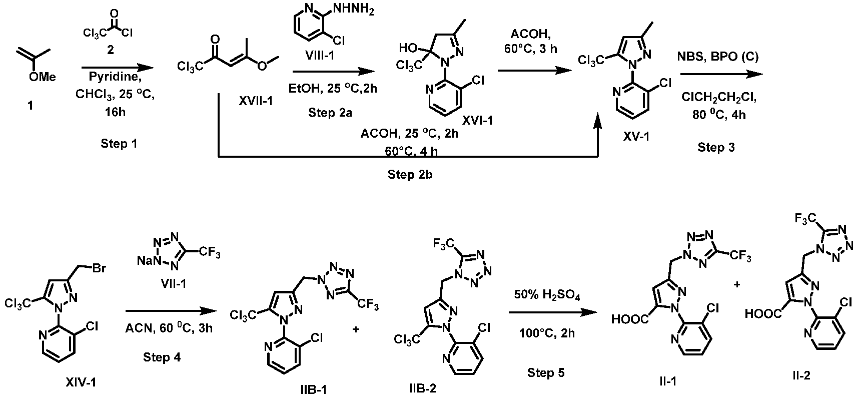

### Step-2a: 1-(3-chloropyridin-2-yl)-3-methyl-5-(trichloromethyl)-4,5-dihydro-1H-pyrazol-5-ol (XVI-1)

A solution of 1,1,1-trichloro-4-methoxypent-3-en-2-one (1.0 g, 4.60 mmol) (prepared in accordance with the process described in Step 1 of scheme 1) and 3-chloro-2-hydrazinylpyridine (0.7 g, 4.6 mmol) in ethyl alcohol (15 mL) was stirred for 1 h at 25 °C. After completion of the reaction, volatiles of reaction mixture were removed under reduced pressure to get a crude product, which was purified by flash chromatography using 10% ethyl acetate in hexane as eluent to get 1-(3-chloropyridin-2-yl)-3-methyl-5-(trichloromethyl)-4,5-dihydro-1H-pyrazol-5-ol (1.24 g, 3.76 8 mmol, 82% yield).

¹H-NMR (400 MHz, CDCl₃) δ 8.12 (dd, 1H), 7.82 (dd, 1H), 7.07 (m, 1H), 3.70 (s, 1H), 3.22 (s, 1H), 2.09 (s, 3H)

### Step-2b: Synthesis of 3-chloro-2-(3-methyl-5-(trichloromethyl)-1H-pyrazol-1-yl)pyridine (XV-1)

3-Chloro-2-hydrazinylpyridine (0.3 g, 2.3 mmol) was added to a solution of 1,1,1-trichloro-4-methoxypent-3-en-2-one (0.5 g, 2.3 mmol) in acetic acid ( 15 mL) at 25 °C and stirred for 2 h. The reaction mixture was heated at 60 °C for 6 h. The reaction mixture was cooled to 25 °C and partitioned between ethyl acetate (25 mL) and water (20 mL), ethyl acetate layer was washed successively with saturated aqueous sodium bicarbonate solution (25 mL), water (25 mL) and brine solution (20 mL). The ethyl acetate layer was dried over anhydrous sodium sulphate, concentrated under reduced pressure to get the desired product 3-chloro-2-(3-methyl-5-(trichloromethyl)-1H-pyrazol-1-yl)pyridine (0.5 g, 1.64 mmol, 71% yield).

¹H-NMR (400 MHz, CDCl₃) δ 8.52 (dd, 1H), 7.90 (dd, 1H), 7.42 (m, 1H), 6.70 (s, 1H), 2.35 (s, 3H); LCMS: [311.9]^{M+H}

### Step-3: Synthesis of 2-(3-(bromomethyl)-5-(trichloromethyl)-1H-pyrazol-1-yl)-3-chloropyridine (XIV-1)

To a solution of 3-chloro-2-(3-methyl-5-(trichloromethyl)-1H-pyrazol-1-yl)pyridine (0.2 g, 0.6 mmol) in dichloroethane (5 mL) was added N-bromosuccinimide (0.17 g, 0.96 mmol) and benzoyl peroxide (0.1 g, 0.3 mmol) and stirred at 80 °C for 4 h. After completion of the reaction, the reaction mixture was diluted with water and extracted thrice with ethyl acetate (20 mL), dried over anhydrous sodium sulphate, concentrated under reduced pressure to get a crude product, which was purified by flash chromatography using 20% ethyl acetate in hexane as eluent to get the desired product 2-(3-(bromomethyl)-5-(trichloromethyl)-1H-pyrazol-1-yl)-3-chloropyridine (0.2 g, 0.4 mmol, 67% yield).

¹H-NMR (400 MHz, CDCl₃) δ 8.53 (dd, 1H), 7.92 (dd, 1H), 7.47 (m, 1H), 6.97 (s, 1H), 4.49 (s, 2H); LCMS: [389.9]^{M-H}

### Step-4: 3-chloro-2-(5-(trichloromethyl)-3-((5-(trifluoromethyl)-2H-tetrazol-2-yl)methyl)-1H-pyrazol-1-yl)pyridine (IIB-1) and 3-chloro-2-(5-(trichloromethyl)-3-((5-(trifluoromethyl)-1H-tetrazol-1-yl)methyl)-1H-pyrazol-1-yl)pyridine (IIB-2)

Sodium 5-(trifluoromethyl)tetrazol-1-ide (0.1 g, 0.7 mmol) was added to a stirred solution of 2-(3-(bromomethyl)-5-(trichloromethyl)-1H-pyrazol-1-yl)-3-chloropyridine (0.2 g, 0.6 mmol) in acetonitrile (2 mL) at 25 °C and the resulting reaction mixture was heated with stirring at 60 °C for 3 h. After completion of the reaction, the reaction mixture was diluted with water and extracted thrice with ethyl acetate (20 mL). The combined ethyl acetate layer was washed with brine (25 mL), dried over anhydrous sodium sulphate and concentrated under reduced pressure to get a crude product, which was purified by flash chromatography to get 3-chloro-2-(5-(trichloromethyl)-3-((5-(trifluoromethyl)-2H-tetrazol-2-yl)methyl)-1H-pyrazol-1-yl)pyridine and 3-chloro-2-(5-(trichloromethyl)-3-((5-(trifluoromethyl)-1H-tetrazol-1-yl)methyl)-1H-pyrazol-1-yl)pyridine (0.2 g, 0.4 mmol, 75% yield).

¹H-NMR (400 MHz, CDCl₃) δ 8.53 (dd, 1H), 7.92 (dd, 1H), 7.48 (m, 1H), 7.0 (s, 1H), 5.96 (s, 2H); LCMS: [448]^{M+H}

### Step-5: 1-(3-chloropyridin-2-yl)-3-((5-(trifluoromethyl)-2H-tetrazol-2-yl)methyl)-1H-pyrazole-5-carboxylic acid (II-1) and 1-(3-chloropyridin-2-yl)-3-((5-(trifluoromethyl)-1H-tetrazol-1-yl)methyl)-1H-pyrazole-5-carboxylic acid (II-2)

A stirred solution of 3-chloro-2-(5-(trichloromethyl)-3-((5-(trifluoromethyl)-2H-tetrazol-2-yl)methyl)-1H-pyrazol-1-yl)pyridine (0.25 g, 0.56 mmol) in 50% (v/v) aqueous sulphuric acid solution (3 mL) was heated with stirring at 100 °C for 2 h. After completion of the reaction, the reaction mixture was cooled to 25 °C and was diluted with ice-cold water to get a precipitate, which was filtered and dried to get the desired product 1-(3-chloropyridin-2-yl)-3-((5-(trifluoromethyl)-2H-tetrazol-2-yl)methyl)-1H-pyrazole-5-carboxylic acid and 1-(3-chloropyridin-2-yl)-3-((5-(trifluoromethyl)-1H-tetrazol-1-yl)methyl)-1H-pyrazole-5-carboxylic acid (0.2 g, 0.5 mmol, 97% yield).

¹H-NMR (400 MHz, DMSO-D₆) δ 8.55 (dd, J = 4.6, 1.5 Hz, 1H), 8.23-8.28 (m, 1H), 7.68 (dd, J = 8.1, 4.6 Hz, 1H), 7.20 (s, 1H), 6.25 (s, 2H); LCMS: [373.9]^{M-H}

### Example 7:

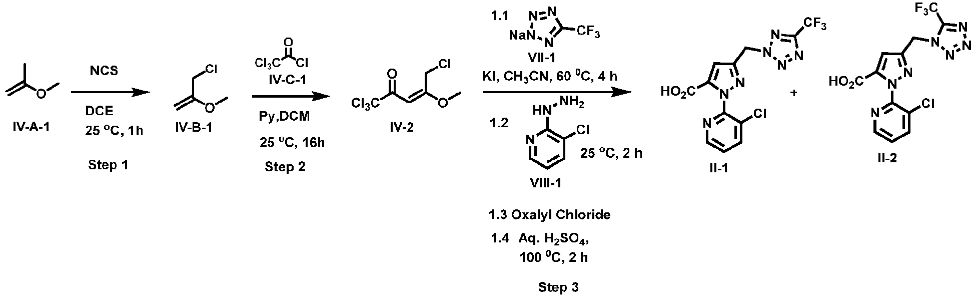

### Step-1

### Synthesis of 3-chloro-2-methoxyprop-1-ene

To a solution of 2-methoxyprop-1-ene (3.9 mL, 41.6 mmol) in dichloroethane (50 mL) was added N-chlorosuccinimide (5.6 g, 41.6 mmol) at 0 °C and stirred for 1 h at 0 °C. The temperature of the reaction mixture was allowed to rise to 25 °C and filtered to remove precipitated succinimide. The filtrate was concentrated under reduced pressure to obtain crude 3-chloro-2-methoxyprop-1-ene (50% by GCMS). GC: 106 M+

### Step-2

### Synthesis of 1,1,1,5-tetrachloro-4-methoxypent-3-en-2-one

To a stirred solution of 3-chloro-2-methoxyprop-1-ene (3.4 g, 31.6 mmol) and pyridine (2.9 mL, 35.7 mmol) in dichloromethane (30 mL), 2,2,2-trichloroacetyl chloride (3.1 mL, 27.5 mmol) in dichloromethane (10 mL) was added drop wise over 30 min. The reaction mixture was stirred at 25 °C for 16 h and diluted with dichloromethane (50 mL) washed subsequently with 0.1 M hydrochloric acid (30 mL) and twice with water (50 mL). The dichloromethane layer was dried over anhydrous sodium sulphate and concentrated under reduced pressure to obtain a crude product, which was purified by flash chromatography using 10% ethyl acetate in hexane as eluent to obtain pure product 1,1,1,5-tetrachloro-4-methoxypent-3-en-2-one (3 g, 4 mmol, 43% yield).

¹H-NMR (400 MHz, CDCl₃) δ 6.08 (s, 1H), 4.62 (s, 2H), 3.88 (s, 3H)

### Step-3

### Synthesis of 1-(3-chloropyridin-2-yl)-3-((5-(trifluoromethyl)-2H-tetrazol-2-yl)methyl)-1H-pyrazole-5-carboxylic acid (II-1) and 1-(3-chloropyridin-2-yl)-3-((5-(trifluoromethyl)-1H-tetrazol-1-yl)methyl)-1H-pyrazole-5-carboxylic acid (II-2)

A mixture of 1,1,1,5-tetrachloro-4-methoxypent-3-en-2-one (1.0 g, 4.0 mmol), sodium 5-(trifluoromethyl)tetrazol-2-ide (0.7 g, 0.44 mmol) and potassium iodide (0.1 g, 0.8 mmol) in acetonitrile (10 mL) was heated at 60 °C with stirring for 4 h. The reaction mixture was cooled to 25 °C and 3-chloro-2-hydrazinylpyridine (0.6 g, 4.0 mmol) was added to it. The reaction mixture was stirred for 2 h at 25 °C. Then, oxalyl chloride (0.4 mL, 4.6 mmol) was added drop wise to the reaction mixture during 15 min and allowed to stir at 25 °C for 2 h. Volatiles were evaporated from the reaction mixture, then 40% aqueous sulphuric acid was added (3 mL) under cooling (5 °C) and the reaction mixture was heated at 100 °C for 2 h. The reaction mixture was cooled to 25 °C and was poured onto crushed ice to obtain the crude product as a precipitate, which was filtered and triturated with a solution of 10% dichloromethane in hexane to obtain a solid product (1.3 g, 3.4 mmol, 85% yield), which consisted of 1-(3-chloropyridin-2-yl)-3-((5-(trifluoromethyl)-2H-tetrazol-2-yl)methyl)-1H-pyrazole-5-carboxylic acid (II-1) and 1-(3-chloropyridin-2-yl)-3-((5-(trifluoromethyl)-1H-tetrazol-1-yl)methyl)-1H-pyrazole-5-carboxylic acid (II-2) in 93:07 ratio.
**II-1:** 1H-NMR (400 MHz, DMSO-D6) δ 13.74 (s, 1H), 8.55 (dd, J = 4.6, 1.5 Hz, 1H), 8.23 (dd, J = 8.2, 1.6 Hz, 1H), 7.67 (dd, J = 8.1, 4.6 Hz, 1H), 7.22 (s, 1H), 6.27 (s, 2H)
**19F NMR:-** δ 62.67
**II-1:** 1H-NMR (400 MHz, DMSO-D6) δ 13.75 (s, 1H), 8.53 (dd, J = 4.8, 1.6 Hz, 1H), 8.21 (dd, J = 8.1, 1.7 Hz, 1H), 7.66 (dd, J = 8.1, 4.9 Hz, 1H), 7.15 (s, 1H), 6.05 (s, 2H)
**19F NMR:-** δ 60.36

### Example 8:

### Synthesis of mixture of 1,1,1-trichloro-4-methoxy-5-(5-(trifluoromethyl)-2H-tetrazol-2-yl)pent-3-en-2-one (III-1) and 1,1,1-trichloro-4-methoxy-5-(5-(trifluoromethyl)-1H-tetrazol-1-yl)pent-3-en-2-one (III-2)

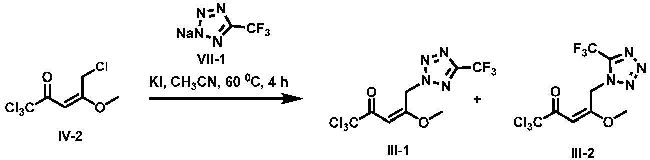

A mixture of 1,1,1,5-tetrachloro-4-methoxypent-3-en-2-one (0.5 g, 2.0 mmol), sodium 5-(trifluoromethyl)tetrazol-2-ide (0.4 g, 0.2 mmol) and potassium iodide (0.1 g, 0.4 mmol) in acetonitrile (10 mL) was heated at 60 °C with stirring for 4 h. After completion of the reaction, the reaction mixture was diluted with water and extracted twice with ethyl acetate (20 mL). Combined ethyl acetate layer was washed with brine solution (20 mL), dried over anhydrous sodium sulphate and concentrated to obtain a crude product, which was purified by flash chromatography using 10% ethyl acetate in hexane as eluent to obtain a mixture of products, consisting of 1,1,1-trichloro-4-methoxy-5-(5-(trifluoromethyl)-2H-tetrazol-2-yl)pent-3-en-2-one (III-1) and 1,1,1-trichloro-4-methoxy-5-(5-(trifluoromethyl)-1H-tetrazol-1-yl)pent-3-en-2-one (III-2) in 93:07 ratio (0.6 g, 1.8 mmol, 90% yield).

¹H-NMR (400 MHz, CDCl₃) δ 6.26 (s, 1H), 6.12 (d, J = 0.8 Hz, 2H), 3.82 (s, 3H); LCMS: [353]^{M-H}

### Example 9:

### Synthesis of mixture of (E)-1,1,1-trichloro-4-methoxy-5-(5-(trifluoromethyl)-2H-tetrazol-2-yl)pent-3-en-2-one (III-1) and (E)-1,1,1-trichloro-4-methoxy-5-(5-(trifluoromethyl)-1H-tetrazol-1-yl)pent-3-en-2-one (III-2)

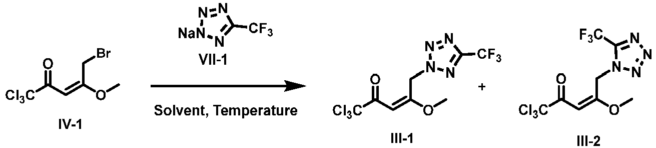

(E)-5-bromo-1,1,1-trichloro-4-methoxypent-3-en-2-one (IV-1; 158 g; 533 mmol) was reacted with 5-(trifluoromethyl)-2H-tetrazole (VII-1; 85 g, 533 mmol) for 5 h in a suitable solvent (460 mL) as shown in Table 1.

**Table No.1: The preparation of III-1 and III-2 using different solvents at 50 °C to 80 °C**

| **Sr. No.** | **Solvent** |
|---|---|
| 1 | Acetone |
| 2 | Acetonitrile |
| 3 | Methyl tert-butyl ether |
| 4 | Chlorobenzene |
| 5 | Dichloroethane |
| 6 | Dichloromethane |
| 7 | Dioxane |
| 8 | Ethyl acetate |
| 9 | Dimethylformamide |
| 10 | Dimethylacetamide |
| 11 | Dimethyl sulfoxide |
| 12 | 2-Methyltetrahydrofuran |
| 13 | Tetrahydrofuran |
| 14 | 1,2-Dimethoxyether |
| 15 | Toluene |
| 16 | *p*-Xylene |
| 17 | N-methyl-2-pyrrolidone |

A mixture of III-1+III-2 was obtained in low to high yield.

### Example 10:

### Synthesis of mixture of 1-(3-chloropyridin-2-yl)-3-((5-(trifluoromethyl)-2H-tetrazol-2-yl)methyl)-1H-pyrazole-5-carboxylic acid (II-1) and 1-(3-chloropyridin-2-yl)-3-((5-(trifluoromethyl)-1H-tetrazol-1-yl)methyl)-1H-pyrazole-5-carboxylic acid (II-2)

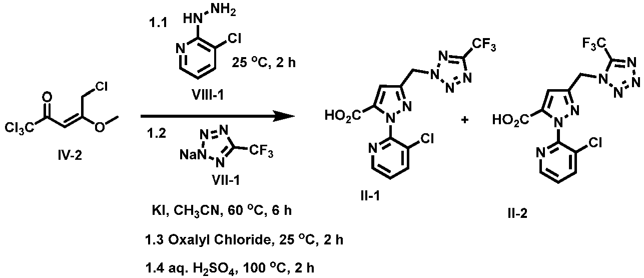

A mixture of 1,1,1,5-tetrachloro-4-methoxypent-3-en-2-one (1.0 g, 4.0 mmol) and 3-chloro-2-hydrazinylpyridine (0.6 g, 4.0 mmol) in acetonitrile (10 mL) was stirred at 25 °C for 2 h. Then, sodium 5-(trifluoromethyl)tetrazol-2-ide (0.7 g, 4.4 mmol) and potassium iodide (0.1 g, 0.8 mmol) were added, and the reaction mixture was heated at 60 °C under stirring for 6 h. Then, oxalyl chloride (0.4 mL, 4.6 mmol) was added drop wise to the reaction mixture during 5 min at 25 °C, and the resulting mixture was allowed to stir at 25 °C for 2 h. Volatiles were evaporated from the reaction mixture. Then, 40% aqueous sulphuric acid was added (5 ml) to the reaction mixture at 5 °C. Subsequently, the reaction mixture was heated at 100 °C under stirring for 2 h. The reaction mixture was cooled to 25 °C and was poured onto crushed ice to obtain a crude precipitate, which was filtered and triturated with a solution of 10% dichloromethane in hexane to obtain a solid product (1.10 g, 3 mmol, 75% yield), which consists of 1-(3-chloropyridin-2-yl)-3-((5-(trifluoromethyl)-2H-tetrazol-2-yl)methyl)-1H-pyrazole-5-carboxylic acid (II-1) and 1-(3-chloropyridin-2-yl)-3-((5-(trifluoromethyl)-1H-tetrazol-1-yl)methyl)-1H-pyrazole-5-carboxylic acid (II-2) in 84:16 ratio.
**II-1:** 1H-NMR (400 MHz, DMSO-D6) δ 13.74 (s, 1H), 8.55 (dd, J = 4.6, 1.5 Hz, 1H), 8.23 (dd, J = 8.2, 1.6 Hz, 1H), 7.67 (dd, J = 8.1, 4.6 Hz, 1H), 7.22 (s, 1H), 6.27 (s, 2H)
**19F NMR:-** δ 62.67
**II-1:** 1H-NMR (400 MHz, DMSO-D6) δ 13.75 (s, 1H), 8.53 (dd, J = 4.8, 1.6 Hz, 1H), 8.21 (dd, J = 8.1, 1.7 Hz, 1H), 7.66 (dd, J = 8.1, 4.9 Hz, 1H), 7.15 (s, 1H), 6.05 (s, 2H)
**19F NMR:-** δ 60.36

### Example 11:

### Preparation of 3-(chloromethyl)-1-(3-chloropyridin-2-yl)-5-(trichloromethyl)-4,5-dihydro-1H-pyrazol-5-ol (XVIII-1)

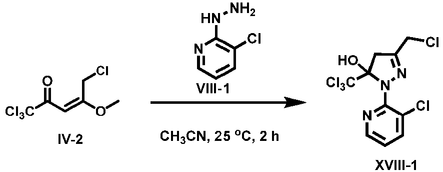

To a stirred solution of 1,1,1,5-tetrachloro-4-methoxypent-3-en-2-one (1 g, 4.0 mmol) in acetonitrile (3 mL), 3-chloro-2-hydrazinylpyridine (0.6 g, 4.0 mmol) was added, and stirring was continued at 25 °C for 2 h. After completion of the reaction, the reaction mixture was diluted with water and extracted twice with ethyl acetate (20 mL). The combined ethyl acetate layers were dried over anhydrous sodium sulphate and concentrated under reduced pressure to obtain a crude product, which was purified by flash chromatography using 10% ethyl acetate in hexane as eluent giving the pure product 3-(chloromethyl)-1-(3-chloropyridin-2-yl)-5-(trichloromethyl)-4,5-dihydro-1H-pyrazol-5-ol (0.8 g, 2.2 mmol, 55% yield).

¹H-NMR (400 MHz, CDCl₃) δ 9.48 (bs, 1H), 8.14 (dd, J = 4.9, 1.7 Hz, 1H), 7.83 (dd, J = 8.1, 1.7 Hz, 1H), 7.08-7.12 (m, 1H), 4.36 (s, 2H), 3.90 (d, J = 18.8 Hz, 1H), 3.45 (d, J = 18.8 Hz, 1H); LCMS: [363.8]^{M-H}

### Example-12:

### Synthesis of 3-(bromomethyl)-1-(3-chloropyridin-2-yl)-5-(trichloromethyl)-4,5-dihydro-1H-pyrazol-5-ol (XVIII-2)

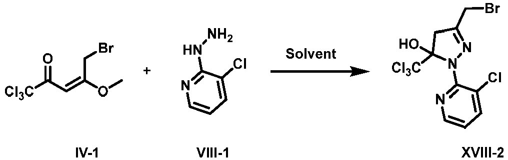

3-chloro-2-hydrazineylpyridine (VIII-1; 143 mg; 1 mmol) was added to a solution of (E)-5-bromo-1,1,1-trichloro-4-methoxypent-3-en-2-one (IV-1; 296 mg; 1.0 mmol) in a suitable solvent (5 mL) as shown in Table No. 2 herein below at 25 °C with stirring for 4 h.

**Table No. 2: The preparation of XVIII-2 using different solvents**

| **Sr. No.** | **Solvent** |
|---|---|
| 1 | Acetone |
| 2 | Acetonitrile |
| 3 | Methyl tert-butyl ether |
| 4 | Chlorobenzene |
| 5 | Dichloroethane |
| 6 | Dichloromethane |
| 7 | Dioxane |
| 8 | Dimethylformamide |
| 9 | Dimethylacetamide |
| 10 | Dimethylsulfoxide |
| 11 | Methanol |
| 12 | Ethanol |
| 13 | Isopropanol |
| 14 | Ethyl acetate |
| 15 | 2-metyl tetrahydrofuran |
| 16 | Tetrahydrofuran |
| 17 | 1,2-Dimethoxyether |
| 18 | Toluene |
| 19 | N-methyl-2-pyrrolidone |

XVIII-2 formation was not observed.

### Example 13:

### Preparation of 3-chloro-2-(3-(chloromethyl)-5-(trichloromethyl)-1H-pyrazol-1-yl)pyridine (XIV-2)

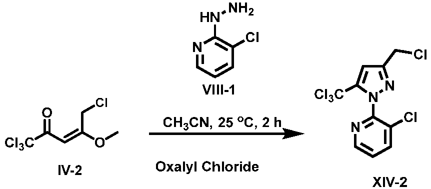

To a stirred solution of 1,1,1,5-tetrachloro-4-methoxypent-3-en-2-one (1 g, 4.0 mmol) in acetonitrile (3 mL), 3-chloro-2-hydrazinylpyridine (0.6 g, 4.0 mmol) was added, and stirring was continued at 25 °C for 1 h. After complete consumption of the starting material, oxalyl chloride (1.0 mL, 11.9 mmol) was added to the reaction mixture. Stirring was continued at 25 °C for another 1 h. After completion of reaction, the reaction mixture was concentrated under reduced pressure to obtain a crude product which was purified by combi flash chromatography to obtain 3-chloro-2-(3-(chloromethyl)-5-(trichloromethyl)-1H-pyrazol-1-yl)pyridine (1.2 g, 3.5 mmol, 88% yield).

¹H-NMR (400 MHz, DMSO-D6) δ 8.63 (dd, J = 4.6, 1.5 Hz, 1H), 8.31 (dd, J = 8.1, 1.5 Hz, 1H), 7.76 (dd, J = 8.1, 4.6 Hz, 1H), 7.14-7.18 (m, 1H), 4.81 (s, 2H); LCMS: [345]^{M+H}

### Example 14:

### Synthesis of 1-(3-chloropyridin-2-yl)-3-((5-(trifluoromethyl)-2H-tetrazol-2-yl)methyl)-1H-pyrazole-5-carboxylic acid (IIB-1) and 1-(3-chloropyridin-2-yl)-3-((5-(trifluoromethyl)-1H-tetrazol-1-yl)methyl)-1H-pyrazole-5-carboxylic acid (IIB-2)

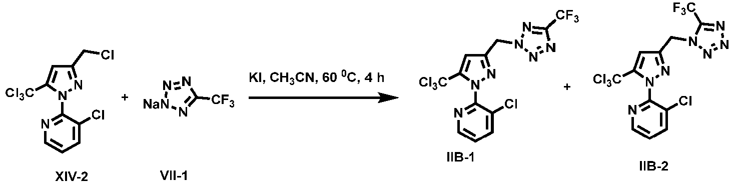

A mixture of (3-chloro-2-(3-(chloromethyl)-5-(trichloromethyl)-1H-pyrazol-1-yl)pyridine (1.0 g, 2.9 mmol), sodium 5-(trifluoromethyl)tetrazol-2-ide (0.5 g, 3.2 mmol) and potassium iodide (0.1 g, 0.3 mmol) in acetonitrile (10 mL) was heated at 60 °C under stirring for 4 h. The reaction mixture was cooled and was poured onto crushed ice to obtain a precipitate, which was filtered and triturated with hexane to get a crude product (1.3 g, 2.6 mmol, 90 % yield), which consists of 1-(3-chloropyridin-2-yl)-3-((5-(trifluoromethyl)-2H-tetrazol-2-yl)methyl)-1H-pyrazole-5-carboxylic acid (IIB-1) and 1-(3-chloropyridin-2-yl)-3-((5-(trifluoromethyl)-1H-tetrazol-1-yl)methyl)-1H-pyrazole-5-carboxylic acid (IIB-2) in 85:15 ratio.

¹H-NMR (400 MHz, DMSO-D6) δ 8.61 (dd, J = 4.6, 1.7 Hz, 1H), 8.29-8.26 (dd, J = 8.1, 1.7 Hz, 1H), 7.52-7.56 (m, 1H), 7.26 (s, 1H), 6.23 (s, 2H); LCMS: [448]^{M+H}

### Example 15:

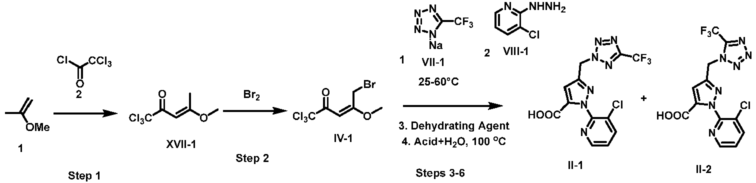

### Steps- 1 & 2: Synthesis of (E)-3-bromo-1,1,1-trichloro-4-methoxypent-3-en-2-one (IV-1) (ref: Synthesis 12, 1013-14, 1986 and Synthesis 16, 2353-2358, 2002; WO2011009551)

A solution of 2,2,2-trichloroacetyl chloride (100 g, 533 mmol) in chlorobenzene (75 mL) was added to a stirred solution of 2-methoxyprop-1-ene (42.5 g, 560 mmol) and 3-picoline (52.7 g, 560 mmol) in chlorobenzene (250 mL) at 0 °C over 1 h. The resultant mixture was stirred at 25 °C for 3 h and which was then filtered and washed with chlorobenzene (150 mL). The resulting filtrate was added drop wise to a solution of Br (87 g, 533 mmol) in chlorobenzene (70 mL) at 0 °C. The reaction mixture was stirred at 0 °C for 20 min followed by addition of a solution of 3-picoline (45.2 g, 480 mmol) in chlorobenzene (30 mL) at 0 °C. The reaction mixture was filtered and the filtrate was washed successively with water (200 mL), aqueous sodium carbonate solution (200 ml) and brine solution (200 mL). Chlorobenzene was distilled out under reduced pressure to obtain desired product IV-1 (158 g; 533 mmol, 99% yield).

¹H-NMR (400 MHz, CDCl₃) δ 6.07 (s, 1H), 4.46 (s, 2H), 3.87 (s, 3H).

### Steps-3 to 6: Synthesis of 1-(3-chloropyridin-2-yl)-3-((5-(trifluoromethyl)-2H-tetrazol-2-yl)methyl)-1H-pyrazole-5-carboxylic acid (II-1 and II-2)

(E)-5-bromo-1,1,1-trichloro-4-methoxypent-3-en-2-one (IV-1; 158 g; 533 mmol) was added at once to a solution of sodium 5-(trifluoromethyl)-2H-tetrazole (VII-1; 85 g, 533 mmol) in acetonitrile (460 mL) at 25 °C, then heated to 60 °C and stirred for 5 h. After completion of the reaction, the reaction mixture was cooled to 25 °C, and 3-chloro-2-hydrazineylpyridine (74.0 g, 517 mmol) was added to it. The reaction mixture was stirred for 4 h at 25 °C. Hydrogen chloride gas (39 g; 1.1 mol) was bubbled into the reaction mixture and allowed to stir at 75 °C for 4 h. Acetonitrile was distilled out from the reaction mixture. The resulting residue was dissolved in glacial acetic acid (320 mL) and subsequently water (480 mL) was added at 100 °C and allowed it to stir for 8 h. The obtained reaction mixture was allowed to cool to 15 °C with stirring. The resulting precipitated product was filtered and washed successively with water (200 mL) and a solution of 30% methyl alcohol in water (200 mL). The resulting product is air-dried to obtain 150 g of desired product II-1 and II-2. The product was further purified by using a mixture ethyl acetate and hexane.

### Example 16:

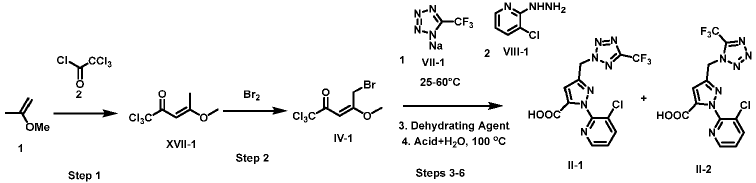

### Steps- 1 & 2: Synthesis of (E)-3-bromo-1,1,1-trichloro-4-methoxypent-3-en-2-one (IV-1) (ref: Synthesis 12, 1013-14, 1986 and Synthesis 16, 2353-2358, 2002; WO2011009551)

A solution of 2,2,2-trichloroacetyl chloride (100 g, 533 mmol) in chlorobenzene (75 mL) was added to a stirred solution of 2-methoxyprop-1-ene (42.5 g, 560 mmol) and 3-picoline (52.7 g, 560 mmol) in chlorobenzene (250 mL) at 0 °C over 1 h. The resultant mixture was stirred at 25 °C for 3 h and was then filtered and washed with chlorobenzene (150 mL). The resulting filtrate was added drop wise to a solution of bromine (87 g, 533 mmol) in chlorobenzene (70 mL) at 0 °C. The reaction mixture was stirred at 0 °C for 20 min followed by addition of a solution of 3-picoline (45.2 g, 480 mmol) in chlorobenzene (30 mL) at 0 °C. The reaction mixture was filtered and the filtrate was washed successively with water (200 mL), aqueous sodium carbonate solution (200 mL) and brine solution (200 mL). Chlorobenzene was distilled out under reduced pressure to obtain desired product IV-1 (158 g; 533 mmol, 99% yield).

¹H-NMR (400 MHz, CDCl₃) δ 6.07 (s, 1H), 4.46 (s, 2H), 3.87 (s, 3H).

### Steps-3 to 6: Synthesis of 1-(3-chloropyridin-2-yl)-3-((5-(trifluoromethyl)-2H-tetrazol-2-yl)methyl)-1H-pyrazole-5-carboxylic acid (II-1 and II-2)

(E)-5-bromo-1,1,1-trichloro-4-methoxypent-3-en-2-one (IV-1; 158 g; 533 mmol) and a solution of sodium 5-(trifluoromethyl)-2H-tetrazole (VII-1; 85 g, 533 mmol) in acetonitrile (450 mL), separately, were added at a rate of 1:3 respectively to acetonitrile (460 mL) at 60 °C and stirred for 5 h. After completion of the reaction, the reaction mixture was cooled to 25 °C, and 3-chloro-2-hydrazineylpyridine (74.0 g, 517 mmol) was added to it. The reaction mixture was stirred for 4 h at 25 °C. Hydrogen chloride gas (39 g; 1.1 mol) was bubbled into the reaction mixture and allowed to stir at 75 °C for 4 h. Acetonitrile was distilled out from the reaction mixture. The resulting residue was dissolved in glacial acetic acid (320 mL) and subsequently water (480 mL) was added at 100 °C and allowed it to stir for 8 h. The obtained reaction mixture was allowed to cool to 15 °C with stirring. The resulting precipitated product was filtered and washed successively with water (200 mL) and a solution of 20% methyl alcohol in water (200 mL). The resulting product is air-dried to obtain 150 g of desired product II-1 (97%) and II-2 (3%). The product was further purified by using a mixture ethyl acetate and hexane.

### Example 17:

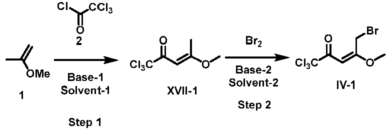

### Steps- 1 & 2: Synthesis of (E)-3-bromo-1,1,1-trichloro-4-methoxypent-3-en-2-one (IV-1) (ref: Synthesis 12, 1013-14, 1986 and Synthesis 16, 2353-2358, 2002; WO2011009551)

A solution of 2,2,2-trichloroacetyl chloride (100 g, 533 mmol) in solvent-1 (75 mL) was added to a stirred solution of 2-methoxyprop-1-ene (42.5 g, 560 mmol) and base-1 (52.7 g, 560 mmol) in solvent-1 (250 mL) at 0 °C over 1 h. The resultant mixture was stirred at 25 °C for 3 h and which was then filtered and washed with solvent-1 (150 mL). The resulting filtrate was added drop wise to a solution of bromine (87 g, 533 mmol) in solvent-2 (70 mL) at 0 °C. The reaction mixture was stirred at 0 °C for 20 min followed by addition of a solution of base-2 (45.2 g, 480 mmol) in solvent-2 (30 mL) at 0 °C. The reaction mixture was filtered and filtrate was washed successively with water (200 mL), aqueous sodium carbonate solution (200 ml) and brine solution (200 mL). Solvent-2 was distilled out under reduced pressure to obtain desired product IV-1. ¹H-NMR (400 MHz, CDCl₃) δ 6.07 (s, 1H), 4.46 (s, 2H), 3.87 (s, 3H).

The yields are provided in table no. 3 below:

The above reaction was carried out additionally using different bases and solvents as shown in Table No. 3.

**Table No. 3: The preparation of IV-1 using different solvents.**

| **Sr. No.** | **Base-1 and Solvent-1** | **Base-2 and Solvent-2** |
|---|---|---|
| 1 | Pyridine, Chlorobenzene | Pyridine, Chlorobenzene |
| 2 | 3-Picoline, Chlorobenzene | 3-Picoline, Chlorobenzene |
| 3 | Pyridine, Methyl tert-butyl ether | Pyridine, Methyl tert-butyl ether |
| 4 | 3-Picoline, Methyl tert-butyl ether | 3-Picoline, Methyl tert-butyl ether |
| 5 | Pyridine, Dichloromethane | Pyridine, Dichloromethane |
| 6 | 3-Picoline, Dichloromethane | 3-Picoline, Dichloromethane |
| 7 | Pyridine, Dichloromethane | Sodium carbonate, Dichloromethane |
| 8 | Pyridine, Dichloromethane | Sodium bicarbonate, Dichloromethane |
| 9 | Pyridine, Dichloromethane | Potassium bicarbonate, Dichloromethane |
| 10 | Pyridine, Dichloromethane | Cesium bicarbonate, Dichloromethane |
| 11 | Pyridine, Dichloromethane | Sodium carbonate, Acetonitrile |
| 12 | Pyridine, Dichloromethane | Sodium bicarbonate, Acetonitrile |
| 13 | Pyridine, Dichloromethane | Potassium bicarbonate, Acetonitrile |
| 14 | Pyridine, Dichloromethane | Cesium bicarbonate, Acetonitrile |
| 15 | Pyridine, Dichloromethane | Sodium hydroxide, Dichloromethane |

IV-1 was obtained in moderate to high yield.

### Example 18:

### Synthesis of mixture of 1-(3-chloropyridin-2-yl)-5-(trichloromethyl)-3-((5-(trifluoromethyl)-2H-tetrazol-2-yl)methyl)-4,5-dihydro-1H-pyrazol-5-ol (IIA-1) and 1-(3-chloropyridin-2-yl)-5-(trichloromethyl)-3-((5-(trifluoromethyl)-1H-tetrazol-1-yl)methyl)-4,5-dihydro-1H-pyrazol-5-ol (IIA-2)

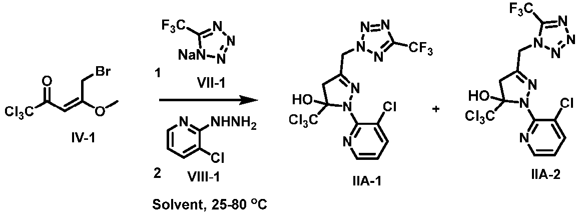

(E)-5-bromo-1,1,1-trichloro-4-methoxypent-3-en-2-one (IV-1, 158 g, 533 mmol) was added to a solution of sodium 5-(trifluoromethyl)-2H-tetrazole (VII-1, 85 g, 533 mmol) in a suitable solvent (460 mL) (see Table No. 4 herein below) at 60 °C with stirring for 5 h. The resulting reaction mixture was cooled to 25 °C, and 3-chloro-2-hydrazineylpyridine (VIII-1, 74.0 g, 517 mmol) was added to it. The reaction mixture was stirred for 4 h at 25 °C. The results are provided in Table No. 4 herein below.

**Table No. 4: The preparation of IIA-1 and IIA-2 using different solvents**

| **Sr. No.** | **Solvent** |
|---|---|
| 1 | Acetone |
| 2 | Acetonitrile |
| 3 | Methyl tert-butyl ether |
| 4 | Chlorobenzene |
| 5 | Dichloroethane |
| 6 | Dichloromethane |
| 7 | Dioxane |
| 8 | Ethyl acetate |
| 9 | Dimethylformamide |
| 10 | Dimethylacetamide |
| 11 | Dimethyl sulfoxide |
| 12 | 2-Methyltetrahydrofuran |
| 13 | Tetrahydrofuran |
| 14 | 1,2-Dimethoxyether |
| 15 | Toluene |
| 16 | Xylene |
| 17 | N-methyl-2-pyrrolidone |

A mixture of IIA-1 and IIA-2 was obtained in low to high yield.

### Example 19:

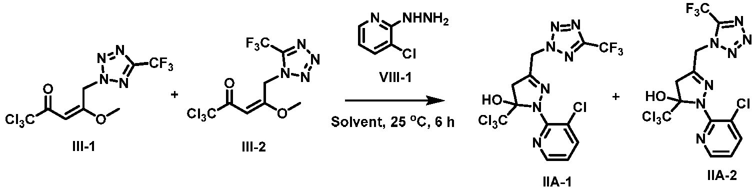

The above reaction with 1:1 molar ratio of III-1+III-2:VIII-1 was carried out using different solvents as mentioned in Table No. 5 herein below.

**Table No. 5: The preparation of IIA-1 and IIA-2 using different solvents**

| **Sr. No.** | **Solvent** |
|---|---|
| 1 | Acetone |
| 2 | Acetonitrile |
| 3 | Methyl tert-butyl ether |
| 4 | Chlorobenzene |
| 5 | Dichloroethane |
| 6 | Dichloromethane |
| 7 | Dioxane |
| 8 | Dimethylformamide |
| 9 | Dimethylacetamide |
| 10 | Dimethyl sulfoxide |
| 11 | Ethyl acetate |
| 12 | 2-Metyltetrahedrafuran |
| 13 | Tetrahydrofuran |
| 14 | 1,2-Dimethoxyether |
| 15 | Toluene |
| 16 | Xylene |
| 17 | N-methyl-2-pyrrolidone |

A mixture of IIA-1 and IIA-2 was obtained in low to high yield.

### Example 20:

### Synthesis of mixture of 3-chloro-2-(5-(trichloromethyl)-3-((5-(trifluoromethyl)-2H-tetrazol-2-yl)methyl)-1H-pyrazol-1-yl)pyridine (IIB-1) and 3-chloro-2-(5-(trichloromethyl)-3-((5-(trifluoromethyl)-1H-tetrazol-1-yl)methyl)-1H-pyrazol-1-yl)pyridine (IIB-2)

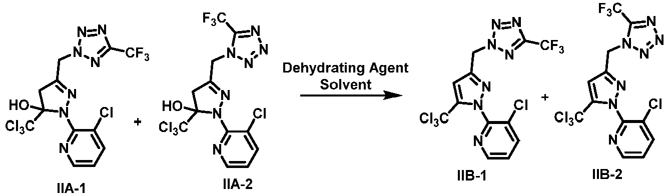

Hydrochloride gas (109 mg, 3 mmol) was bubbled into a solution of a mixture containing 1-(3-chloropyridin-2-yl)-5-(trichloromethyl)-3-((5-(trifluoromethyl)-2H-tetrazol-2-yl)methyl)-4,5-dihydro-1H-pyrazol-5-ol and 1-(3-chloropyridin-2-yl)-5-(trichloromethyl)-3-((5-(trifluoromethyl)-1H-tetrazol-1-yl)methyl)-4,5-dihydro-1H-pyrazol-5-ol (930 mg, 2 mmol) in a suitable solvent and dehydrating agent at 25 °C (see Table No. 6 herein below). The reaction mixture was heated at 65 °C with stirring for 6 h.

**Table No. 6: The preparation of IIB-1 and IIB-2 using different dehydrating agents and solvents**

| **Sr. No.** | **Dehydrating agent** | **Solvent** |
|---|---|---|
| 1 | Oxalyl chloride | Acetonitrile |
| 2 | Oxalyl chloride | Methyl tert-butyl ether |
| 3 | Oxalyl chloride | Dichloromethane |
| 4 | Oxalyl chloride | Dioxane |
| 5 | Thionyl chloride | Thionyl Chloride |
| 6 | Thionyl chloride | Dichloromethane |
| 7 | Thionyl chloride | Methyl tert-butyl ether |
| 8 | Thionyl chloride | Acetic acid |
| 9 | Methanolic hydrochloric acid | Methyl alcohol |
| 10 | Hydrogen chloride gas | Methyl alcohol |
| 11 | Conc. Sulphuric acid (cat) | Ethyl alcohol |
| 12 | Conc. Sulphuric acid (cat) | Tetrahydrofuran |
| 13 | Conc. Sulphuric acid (cat) | Methyl alcohol |
| 14 | Conc. Sulphuric acid (cat) | Dioxane |
| 15 | Conc. Sulphuric acid (cat) | Isopropyl alcohol |
| 16 | Conc. Sulphuric acid (cat) | tert-Butyl alcohol |
| 17 | Conc. Sulphuric acid (cat) | Acetonitrile |
| 18 | Conc. Sulphuric acid (cat) | Acetic acid |
| 19 | Acetic Acid | Acetonitrile |
| 20 | Acetic acid | Acetic acid |
| 21 | Hydrogen chloride-1,4-dioxane | Acetic acid |
| 22 | Hydrogen chloride gas | Acetic acid |
| 23 | Hydrogen bromide (30% in Acetic Acid) | Acetic acid |
| 24 | Conc. Sulphuric acid (cat) | Acetic acid |
| 25 | Triflic acid(cat) | Acetic acid |
| 26 | Trifluoroacetic acid (cat) | Acetic acid |
| 27 | Methanesulfonic acid | Acetic acid |
| 28 | p-Toluenesulfonic acid | Acetonitrile |
| 29 | Methanesulfonic acid | Acetonitrile |
| 30 | Silica gel | Acetonitrile |
| 31 | Silica gel | Acetic acid |

A mixture of IIB-1+IIB-2 was obtained in moderate to high yield.

### Example 21:

### Synthesis of mixture of 1-(3-chloropyridin-2-yl)-3-((5-(trifluoromethyl)-2H-tetrazol-2-yl)methyl)-1H-pyrazole-5-carboxylic acid (II-1) and 1-(3-chloropyridin-2-yl)-3-((5-(trifluoromethyl)-1H-tetrazol-1-yl)methyl)-1H-pyrazole-5-carboxylic acid (II-2)

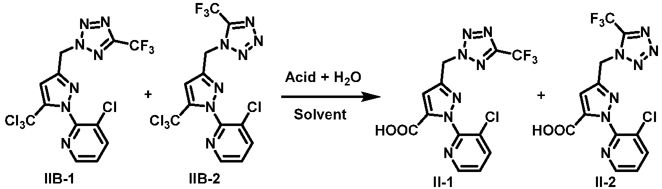

A mixture (1.3 g, 3 mmol) of 3-chloro-2-(5-(trichloromethyl)-3-((5-(trifluoromethyl)-2H-tetrazol-2-yl)methyl)-1H-pyrazol-1-yl)pyridine (IIB-1) and 3-chloro-2-(5-(trichloromethyl)-3-((5-(trifluoromethyl)-1H-tetrazol-1-yl)methyl)-1H-pyrazol-1-yl)pyridine (IIB-2) was dissolved in a suitable acid and solvent (see Table No. 7 herein below). The reaction mixture was heated at 100 °C with stirring. Water (4 mL) was added drop wise to reaction mixture at 100 °C and continued stirring for 8 h.

**Table No. 7: The preparation of II-1 and II-2 different acids and solvents**

| **Sr. No.** | **Acid** | **Solvent** |
|---|---|---|
| 1 | 50 % sulphuric acid | Acetonitrile |
| 2 | 50 % sulphuric acid | 50 % Sulphuric acid |
| 3 | Acetic acid | Water |
| 4 | 6N Hydrochloric acid | Acetonitrile |
| 5 | 50% sulphuric acid | Ethyl alcohol |
| 6 | 50% sulphuric acid | Tetrahydrofuran |
| 7 | 50% sulphuric acid | Dioxane |
| 8 | 50% sulphuric acid | Isopropyl alcohol |
| 9 | 50% sulphuric acid | tert-Butyl alcohol |
| 10 | 10% sulphuric acid | Acetonitrile |
| 11 | 20% sulphuric acid | Acetonitrile |
| 12 | 30% Sulphuric acid | Acetonitrile |
| 13 | 40% sulphuric acid | Acetonitrile |
| 14 | 98% sulphuric acid | Acetonitrile |
| 15 | 10% sulphuric acid | Acetic Acid |
| 16 | 20% sulphuric acid | Acetic Acid |
| 17 | 30% sulphuric acid | Acetic Acid |
| 18 | 40% sulphuric acid | Acetic Acid |
| 19 | 6N Hydrochloric Acid | Methyl alcohol |
| 20 | 6N Hydrochloric Acid | Ethyl alcohol |
| 21 | 6N Hydrochloric Acid | tert-Butyl alcohol |
| 22 | 6N Hydrochloric Acid | Isopropyl alcohol |

A mixture of II-1+II-2 was obtained in moderate to high yield.

### Example 22:

### One pot synthesis of 3-chloro-2-methoxyprop-1-ene (3a)

To a solution of 2-methoxyprop-1-ene (670 mg, 9.3 mmol) in dichloroethane (10 mL) was added *N-*chlorosuccinimide (225 mg, 9.3 mmol) at 0 °C and stirred for 1 h at 0 °C. The temperature of the reaction mixture was allowed to rise to 25 °C and filtered to remove precipitated succinimide. The filtrate was taken in a round bottom flask and to it pyridine and a solution of 2,2,2-trichloroacetyl chloride (1.69 g, 9.3 mmol) in dichloroethane (10 mL) was added drop wise over 30 min. The reaction mixture was stirred at 25 °C for 16 h. Then, the reaction mixture was washed subsequently with 0.1 M hydrochloric acid (10 mL) and twice with water (10 mL). The dichloroethane layer was dried over anhydrous sodium sulphate and concentrated under reduced pressure to obtain a crude product, which was purified by flash chromatography to obtain pure product 1,1,1,5-tetrachloro-4-methoxypent-3-en-2-one (1.0 g, 1.34 mmol, 15% yield).

¹H-NMR (400 MHz, CDCl₃) δ 6.08 (s, 1H), 4.62 (s, 2H), 3.88 (s, 3H).

## Claims

1. A process for preparing a compound of formula II 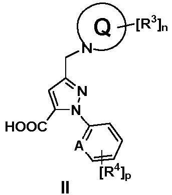 wherein,
A is N or C;
R³ and R⁴ are independently selected from the group consisting of hydrogen, halogen, cyano, nitro, hydroxy, C₁-C₆-alkyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, C₁-C₆-alkylsulphinyl, C₁-C₆-alkylsulphonyl, C₁-C₆-haloalkylthio, C₁-C₆-haloalkylsulphinyl, C₁-C₆-haloalkylsulphonyl and NR^{a}R^{b}; R^{a} and R^{b} are independently selected from the group consisting of hydrogen, C₁-C₆-alkyl, C₃-C₆-cycloalkyl; or R^{a} and R^{b} together with the N atom to which they are attached form a substituted or unsubstituted 3- to 6-membered heterocyclic ring,
wherein, the substituents on the 3- to 6- membered heterocyclic ring are selected from the group consisting of halogen, cyano, nitro, hydroxy, C₁-C₆-alkyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, C₁-C₆-alkylsulphinyl, C₁-C₆-alkylsulphonyl, C₁-C₆-haloalkylthio, C₁-C₆-haloalkylsulphinyl and C₁-C₆-haloalkylsulphonyl;
Q is a 3-, 4- or 5 membered heterocyclic ring;
n is an integer 0 to 4; and
p is an integer 0 to 5;
said process comprising the steps of:
a. reacting a compound of formula IV with a compound of formula VII in the presence of one or more suitable solvent and optionally, one or more suitable catalyst and or reagent to obtain a compound of formula III, 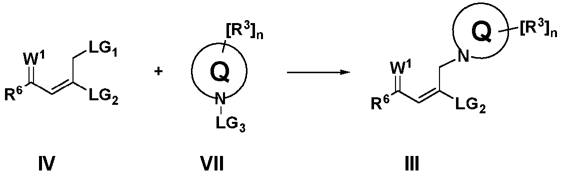 wherein,
R⁶ is selected from the group consisting of CX₃, COW²(R⁷), C(W⁴R⁷)₃, CH(W⁴R⁷)₂, allylic group, substituted or unsubstituted furanyl, 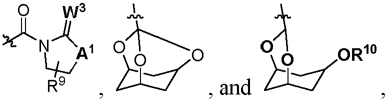 wherein, the substitution on furanyl group is selected from the group consisting of halogen, cyano, nitro, hydroxy, C₁-C₆-alkyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, C₁-C₆-alkylsulphinyl, C₁-C₆-alkylsulphonyl, C₁-C₆-haloalkylthio, C₁-C₆-haloalkylsulphinyl, or C₁-C₆-haloalkylsulphonyl;
W¹, W², W³, W⁴ and A¹ are independently O, S or NR^{1c}; wherein R^{1c} is hydrogen, C₁-C₆-alkyl, or C₃-C₆-cycloalkyl;
R⁷ is selected from the group consisting of hydrogen, substituted or unsubstituted C₁-C₆-alkyl, substituted or unsubstituted C₃-C₆-cycloalkyl, substituted or unsubstituted aryl and substituted or unsubstituted arylalkyl, or
two R⁷ together with the atom to which they are attached form a substituted or unsubstituted 3- to 6- membered carbocyclic or heterocyclic ring,
wherein, the substitution on C₁-C₆-alkyl, C₃-C₆-cycloalkyl, aryl, and arylalkyl of R⁷ and carbocyclic or heterocyclic ring formed by two R⁷ are independently selected from the group consisting of halogen, cyano, nitro, hydroxy, C₁-C₆-alkyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, C₁-C₆-alkylsulphinyl, C₁-C₆-alkylsulphonyl, C₁-C₆-haloalkylthio, C₁-C₆-haloalkylsulphinyl, and C₁-C₆-haloalkylsulphonyl;
R⁹ and R¹⁰ are independently selected from the group consisting of hydrogen, halogen, cyano, substituted or unsubstituted C₁-C₆-alkyl, substituted or unsubstituted C₁-C₆-alkoxy, substituted or unsubstituted C₃-C₆-cycloalkyl, substituted or unsubstituted C₁-C₆-alkylthio, substituted or unsubstituted C₁-C₆-alkylsulphinyl, substituted or unsubstituted C₁-C₆-alkylsulphonyl, substituted or unsubstituted aryl and substituted or unsubstituted arylalkyl,
wherein, the substitution on C₁-C₆-alkyl, C₁-C₆-alkoxy, C₃-C₆-cycloalkyl, C₁-C₆-alkylthio, C₁-C₆-alkylsulphinyl, C₁-C₆-alkylsulphonyl, aryl, and arylalkyl of R⁹ and R¹⁰ is selected from the group consisting of halogen, cyano, nitro, hydroxy, C₁-C₆-alkyl, C₁-C₆-haloalkyl, C₃-C₆-cycloalkyl, C₃-C₆-halocycloalk-yl, C₁-C₆-alkoxy, C₁-C₆-haloalkoxy, C₁-C₆-alkylthio, C₁-C₆-alkylsulphinyl, C₁-C₆-alkylsulphonyl, C₁-C₆-haloalkylthio, C₁-C₆-haloalkylsulphinyl, and C₁-C₆-haloalkylsulphonyl;
LG₁ is selected from the group consisting of X, OR⁵, and OSi(R¹¹)₃,
wherein, R⁵ is selected from the group consisting of hydrogen, substituted or unsubstituted C₁-C₆-alkyl, substituted or unsubstituted aryl-C₁-C₆-alkyl, substituted or unsubstituted aryl, -(C=O)-C₁-C₆-alkyl, -(C=O)-C₁-C₆-haloalkyl, -(C=O)O-C₁-C₆-alkyl, -(C=O)O-haloC₁-C₆-alkyl, SO₂-C₁-C₆-alkyl, SO₂-C₁-C₆-haloalkyl and substituted or unsubstituted SO₂-aryl,
R¹¹ is selected from the group consisting of hydrogen, halogen, substituted or unsubstituted C₁-C₆-alkyl, substituted or unsubstituted aryl-C₁-C₆-alkyl, substituted or unsubstituted aryl, -(C=O)-C₁-C₆-alkyl, -(C=O)-C₁-C₆-haloalkyl, -(C=O)O-C₁-C₆-alkyl, -(C=O)O-haloC₁-C₆-alkyl, SO₂-C₁-C₆-alkyl, SO₂-C₁-C₆-haloalkyl and substituted or unsubstituted SO₂-aryl;
LG₂ is X, OR¹²,
wherein, R¹² is selected from the group consisting of hydrogen, substituted or unsubstituted C₁-C₆-alkyl, substituted or unsubstituted aryl-C₁-C₆-alkyl, substituted or unsubstituted aryl, -(C=O)-C₁-C₆-alkyl, -(C=O)-C₁-C₆-haloalkyl, -(C=O)O-C₁-C₆-alkyl, -(C=O)O-haloC₁-C₆-alkyl, SO₂-C₁-C₆-alkyl, SO₂-C₁-C₆-haloalkyl, substituted or unsubstituted SO₂-aryl, alkylthio, and NR^{a}R^{b}; R^{a} and R^{b} are independently selected from the group consisting of hydrogen, C₁-C₆-alkyl, C₃-C₆-cycloalkyl; or R^{a} and R^{b} together with the N atom to which they are attached form a substituted or unsubstituted 3- to 6- membered heterocyclic ring, wherein, the substitution on C₁-C₆-alkyl, aryl-C₁-C₆-alkyl, aryl, and SO₂-aryl of LG₁ and LG₂ group is selected from the group consisting of halogen, cyano, nitro, hydroxy, C₁-C₆-alkyl, C₁-C₆-haloalkyl, C₃-C₆-cycloalkyl, C₃-C₆-halocycloalkyl, C₁-C₆-alkoxy, C₁-C₆-haloalkoxy, C₁-C₆-alkylthio, C₁-C₆-alkylsulphinyl, C₁-C₆-alkylsulphonyl, C₁-C₆-haloalkylthio, C₁-C₆-haloalkylsulphinyl, and C₁-C₆-haloalkylsulphonyl;
LG₃ is selected from the group consisting of hydrogen, alkali metal, halogen and Si(R¹¹)₃,
each X is independently hydrogen, F, Cl, Br or I;
R³, n, and Q are each as defined above;
b. cyclizing the compound of formula III with a hydrazine of formula VIII in one or more suitable solvent and optionally, one or more suitable reagent to obtain a compound of formula IIA, 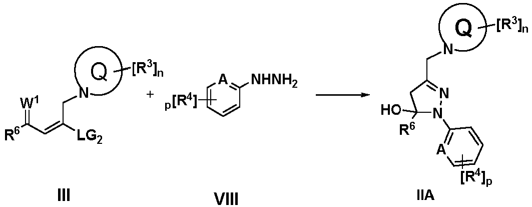 wherein, R³, R⁴, R⁶, A, n, p, Q, W¹, and LG₂ are each as defined herein above;
c. eliminating water from the compound of formula IIA by using one or more suitable dehydrating reagent in one or more suitable solvent to obtain a compound of formula IIB, 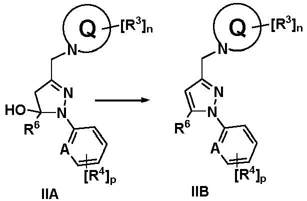 wherein, R³, R⁴, R⁶, A, n, p, and Q are each as defined herein above; and
d. converting the compound of formula IIB into the compound of formula II using one or more suitable reagent in one or more suitable solvent and optionally, one or more suitable catalyst, 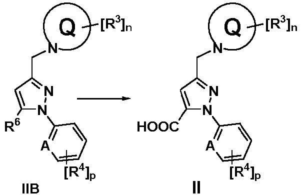
wherein, R³, R⁴, R⁶, A, n, p, and Q are each as defined herein above; and
wherein, the compound of formula III obtained in step (a), the compound of formula IIA obtained in step (b) and the compound of formula IIB obtained in step (c) may or may not be isolated.

2. The process as claimed in claim 1, wherein
i. the suitable solvent in the process step (a) is selected from the group consisting of acetone, acetonitrile, methyl tert-butyl ether, chlorobenzene, dichloroethane, dichloromethane, dioxane, ethyl acetate, dimethylformamide, dimethylacetamide, dimethyl sulfoxide, 2-methyltetrahydrofuran, tetrahydrofuran, 1,2-dimethoxyether, toluene, p-xylene and N-methyl-2-pyrrolidone;
ii. the suitable catalyst in the process step (a) is selected from the group consisting of potassium iodide, sodium iodide, copper iodide and cupric iodide. and
iii. the process step (a) is performed within a temperature range from 20 °C to 150 °C.

3. The process as claimed in claim 1, wherein
i. the suitable solvent in the process step (b) is selected from the group consisting of acetone, acetonitrile, ethyl alcohol, acetic acid, methyl tert-butyl ether, chlorobenzene, dichloroethane, dichloromethane, dioxane, dimethylformamide, dimethylacetamide, dimethyl sulfoxide, ethyl acetate, 2-metyltetrahedrafuran, tetrahydrofuran, 1,2-dimethoxyether, toluene, xylene and N-methyl-2-pyrrolidone;
ii. the suitable reagent in the process step (b) is selected from the group consisting of acetic acid, trifluoroacetic acid, hydrochloric acid, hydrobromic acid, sulphuric acid, methanesulfonic acid, triflic acid, phosphoric acid and p-toluenesulfonic acid; and
iii. the process step (b) is performed within a temperature range from 20 °C to 150 °C.

4. The process as claimed in claim 1, wherein
i. the suitable solvent in the process step (c) is selected from the group consisting of acetonitrile, methyl tert-butyl ether, dichloromethane, dioxane, thionyl chloride, acetic acid, methyl alcohol, ethyl alcohol, tetrahydrofuran, isopropyl alcohol and tert-butyl alcohol;
ii. the suitable dehydrating reagent in the process step (c) is selected from the group consisting of sulphuric acid, trifluoroacetic acid, phosphorous trichloride, phosphorous oxychloride, thionyl chloride, acetic anhydride, trifluoroacetic anhydride, oxalyl chloride, phosgene, diphosgene, methanolic hydrochloric acid, hydrogen chloride gas, acetic acid, hydrogen bromide, triflic acid, methanesulfonic acid, p-toluenesulfonic acid, hydrogen chloride-1,4-dioxane and silica gel; and
iii. the process step (c) is performed within a temperature range from 20 °C to 150 °C.

5. The process as claimed in claim 1, wherein
i. the suitable solvent in the process step (d) is selected from the group consisting of water, acetonitrile, dioxane, sulphuric acid, acetic acid, methyl alcohol, ethyl alcohol, tetrahydrofuran, isopropyl alcohol and tert-butyl alcohol;
ii. the suitable reagent in the process step (d) is an acid selected from the group consisting of sulphuric acid, chlorosulphuric acid, hydrochloric acid, hydrofluoric acid, hydroboric acid, phosphoric acid, acetic acid, trifluoroacetic acid, p-toluenesulphonic acid, methanesulphonic acid, and trifluoromethanesulphonic acid; or
iii. the suitable reagent in the process step (d) is a base selected from the group consisting of trialkylamines, pyridine, alkylpyridines, phosphazines, 1,8-diazabicyclo[5.4.0]undecene (DBU), lithium hydroxide, sodium hydroxide, potassium hydroxide, sodium carbonate, potassium carbonate, sodium acetate, potassium acetate, lithium acetate, sodium methoxide, sodium ethoxide, sodium tert-butoxide, and potassium tert-butoxide;
iv. the catalyst in the process step (d) is selected from the group consisting of ferric chloride (FeCl₃), aluminum chloride (AlCl₃), boron trifluoride (BF₃), antimony trichloride (SbCl₃) and monosodium phosphate (NaH₂PO₄); and
v. the process step (d) is performed within a temperature range from 20 °C to 150 °C.

6. The process as claimed in claim 1, wherein the compound of formula IIB is alternatively obtained by the process comprising the steps of:
i. cyclizing a compound of formula XVII with the hydrazine of formula VIII in one or more suitable solvent and optionally, one or more suitable reagent to obtain a compound of formula XVI, 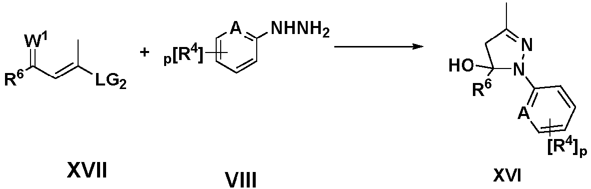
wherein, R⁶ is CX₃, X is F, Cl, Br and I; R⁴, A, p, W¹, and LG₂ are each as defined in claim 1;
or
cyclizing the compound of formula IV and the hydrazine of formula VIII in one or more suitable solvent and optionally, one or more suitable reagent to obtain a compound of formula XVIII, 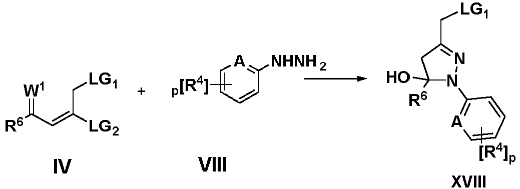
wherein, R⁶ is CX₃, LG₁ is Cl; X is F, Cl, Br and I, preferably Cl; R⁴, A, p, W¹ and LG₂ are each as defined in claim 1;
ii. eliminating water from the compound of formula XVI by using one or more suitable dehydrating reagent in one or more suitable solvent to obtain a compound of formula XV; 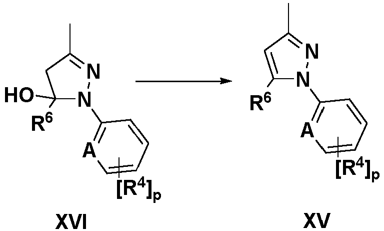
wherein, R⁶ is CX₃, X is F, Cl, Br and I; R⁴, A, and p are each as defined in claim 1;
or
eliminating water from the compound of formula XVIII by using one or more suitable dehydrating reagent in one or more suitable solvent to obtain a compound of formula XIV, 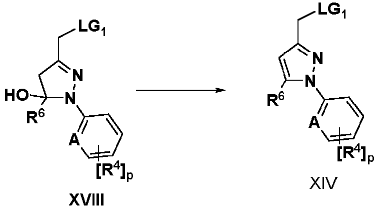
wherein, R⁶ is CX₃, LG₁ is Cl, X is F, Cl, Br and I; R⁴, A, and p, are each as defined in claim 1;
iii. halogenating the compound of formula XV using a suitable halogenating agent in one or more suitable solvent and optionally, one or more radical initiator to obtain a compound of formula XIV, 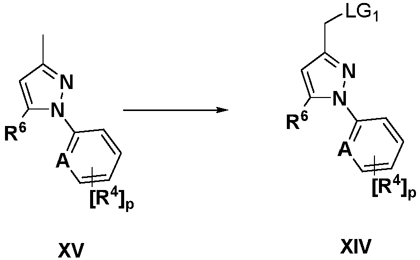 wherein, R⁶ is CX₃, LG₁ is X, X is F, Cl, Br and I; R⁴, A, and p, are each as defined in claim 1; and
iv. obtaining the compound of formula IIB by reacting the compound of formula XIV with the compound of formula VII, 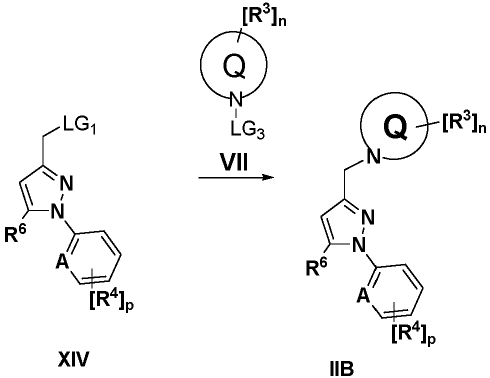
wherein, R⁶ is CX₃, LG₁ is X, X is F, Cl, Br and I; R³, R⁴, A, Q, n, p, and LG₃ are each as defined in claim 1; and
the compounds of formula XVI and XVIII obtained in step (i), the compounds of formula XV and XIV obtained in step (ii) and the compound XIV obtained in step (iii) may or may not be isolated.

7. The process as claimed in claim 6, wherein
i. the suitable solvent in the step (i) is selected from the group consisting of acetone, acetonitrile, ethyl alcohol, methyl tert-butyl ether, chlorobenzene, dichloroethane, dichloromethane, dioxane, dimethylformamide, dimethylacetamide, dimethyl sulfoxide, ethyl acetate, 2-metyltetrahedrafuran, tetrahydrofuran, 1,2-dimethoxyether, toluene, xylene and N-methyl-2-pyrrolidone;
ii. the suitable reagent in the step (i) is selected from the group consisting of acetic acid, trifluoroacetic acid, hydrochloric acid, hydrobromic acid, sulphuric acid, methanesulfonic acid, triflic acid, phosphoric acid and p-toluenesulfonic acid; and
iii. the step (i) is performed within a temperature range from 20 °C to 150 °C.

8. The process as claimed in claim 6, wherein
i. the suitable solvent in the step (ii) is selected from the group consisting of water, acetonitrile, methyl tert-butyl ether, dichloromethane, dioxane, thionyl chloride, acetic acid, methyl alcohol, ethyl alcohol, tetrahydrofuran, isopropyl alcohol and tert-butyl alcohol;
ii. the suitable dehydrating reagent in the step (ii) is selected from the group consisting of sulphuric acid, trifluoroacetic acid, phosphorous trichloride, phosphorous oxychloride, thionyl chloride, acetic anhydride, trifluoroacetic anhydride, oxalyl chloride, phosgene, diphosgene, methanolic hydrochloric acid, hydrogen chloride gas, acetic acid, hydrogen bromide, triflic acid, methanesulfonic acid, p-toluenesulfonic acid and silica gel; and
iii. the step (ii) is performed within a temperature range from 20 °C to 150 °C.

9. The process as claimed in claim 6, wherein
i. the suitable solvent in the step (iii) is selected from the group consisting of acetone, acetonitrile, methyl tert-butyl ether, chlorobenzene, dichloroethane, dichloromethane, dioxane, ethyl acetate, dimethylformamide, dimethylacetamide, dimethyl sulfoxide, 2-methyltetrahydrofuran, tetrahydrofuran, 1,2-dimethoxyether, toluene, p-xylene and N-methyl-2-pyrrolidone;
ii. the halogenating agent in the step (iii) is selected from the group consisting of N-halosuccinimide: N-chlorosuccinimide, N-bromosuccinimide and N-iodosuccinimide; X₂: Cl₂, Br₂ or I₂; X₂/hν: Cl₂/hν Br₂/hν or I₂/hν; N-halosaccharine: N-chlorosaccharine, N-bromosaccharine and N-iodosaccharine; and halohydantoine: N-chlorohydantoine, N-bromohydantoine and N-iodohydantoine;
iii. the radical initiator in the step (iii) is selected from the group consisting of dibenzoyl peroxide, hydrogen peroxide, di(n-propyl)peroxydicarbonate, t-butyl peroxybenzoate, methyl ethyl ketone peroxide, 2,5-dimethyl-2,5-di(t-butylperoxy)-3-hexyne, di(t-butyl)peroxide, acetone peroxide, dicumyl peroxide, azobisisobutyronitrile, bis(2-ethylhexyl)peroxydicarbonate, (peroxybis(propane-2,2-diyl))dibenzene, peracetic acid, metachloroperbenzoic acid, Payne's reagent, magnesium monoperphthalate, trifluoroperacetic acid, trichloroperacetic acid, 2, 4-dinitorperbenzoic acid, Caro's Acid and potassium caroate; and
iv. the step (iii) is performed within a temperature range from 20 °C to 150 °C.

10. The process as claimed in claim 6, wherein
i. the suitable solvent in the step (iv) is selected from the group consisting of acetone, acetonitrile, methyl tert-butyl ether, chlorobenzene, dichloroethane, dichloromethane, dioxane, ethyl acetate, dimethylformamide, dimethylacetamide, dimethyl sulfoxide, 2-methyltetrahydrofuran, tetrahydrofuran, 1,2-dimethoxyether, toluene, p-xylene and N-methyl-2-pyrrolidone;
ii. the catalyst in the step (iv) is selected from the group consisting of potassium iodide, sodium iodide, copper iodide and cupric iodide; and
iii. the step (iv) is performed within a temperature range from 20 °C to 150 °C.

11. The process as claimed in claim 1, wherein the compound of formula IV is obtained by the process steps of:
i. converting a compound of formula IV-A into a compound of formula IV-B using a suitable reagent in one or more suitable solvent, 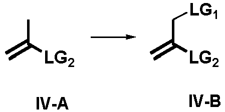 wherein, LG₁ and LG₂ are each as defined in claim 1;
ii. reacting the compound of formula IV-B with a compound of formula IV-C in a suitable solvent and optionally using suitable reagent to obtain the compound of formula IV, 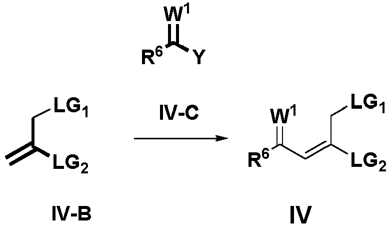
wherein, Y is OR⁵, X, or -O(C=O)CX₃; X is F, Cl, Br or I; R⁵, R⁶, W¹, LG₁ and LG₂ are each as defined in claim 1,
and
wherein, the compound of formula IV-B may or may not be isolated.

12. The process as claimed in claim 11, wherein
i. the suitable reagent in the step (i), is selected from the group consisting of HX, NaX, KX, CuX₂, MgX₂, CsX, ZnX₂, SOCl₂, SO₂Cl₂, COCl₂, X₂, C(=O)(OCl₃)₂, *t*-BuOCl, NaOCl, chloramine-T, N-halosuccinimides, N-halosaccharine, halohydantoine, POX₃, PX₃ and PX₅; wherein, X or halo is Cl, Br, I or F;
ii. the suitable solvent in the step (i) is selected from the group consisting of acetone, acetonitrile, methyl tert-butyl ether, chlorobenzene, dichloroethane, dichloromethane, dioxane, ethyl acetate, dimethylformamide, dimethylacetamide, dimethyl sulfoxide, 2-methyltetrahydrofuran, tetrahydrofuran, 1,2-dimethoxyether, toluene, p-xylene and N-methyl-2-pyrrolidone; and
iii. the step (i) is performed within a temperature range from 20 °C to 100 °C.

13. The process as claimed in claim 11, wherein
i. the suitable reagent in the step (ii), is selected from the group consisting of pyridine, triethyl amine, *N,N*-Diisopropylethylamine, 2,6-Di-tert-butylpyridine, 1,5-Diazabicyclo(4.3.0)non-5-ene, 1,8-Diazabicycloundec-7-ene, Lithium diisopropylamide, sodium bis(trimethylsilyl)amide, and potassium bis(trimethylsilyl)amide;
ii. the suitable solvent in the step (ii) is selected from the group consisting of acetone, acetonitrile, methyl tert-butyl ether, chlorobenzene, dichloroethane, dichloromethane, dioxane, ethyl acetate, dimethylformamide, dimethylacetamide, dimethyl sulfoxide, 2-methyltetrahydrofuran, tetrahydrofuran, 1,2-dimethoxyether, toluene, p-xylene and N-methyl-2-pyrrolidone; and
iii. the step (ii) is performed within a temperature range from 20 °C to 100 °C.

14. The process as claimed in claim 1, wherein said process further comprises the step of:
reacting the compound of formula II optionally after converting into a compound of formula X with a compound of formula IX to obtain the compound of formula I, 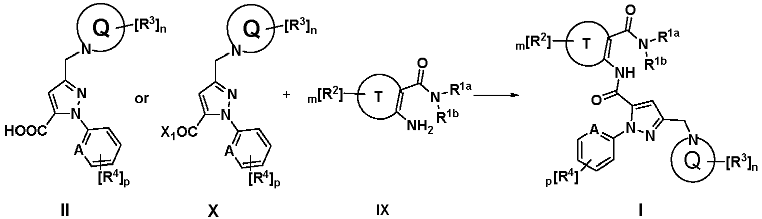 wherein,
X₁ is Cl or Br;
R^{1a} and R^{1b} are independently selected from the group consisting of hydrogen, C₁-C₆-alkyl, C₁-C₆-haloalkyl, C₃-C₆-cycloalkyl, (C₁-C₆-alkyl)-C₃-C₆-cycloalkyl, and (C₃-C₆-cycloalkyl)-C₁-C₆-alkyl; or
R^{1a} and R^{1b} together with the N atom to which they are attached form N=S(=O)₀₋₂(C₁-C₆-alkyl)₂;
T is an aryl or a heteroaryl ring or a fused or a bicyclic aryl or heteroaryl ring or ring system;
R² is selected from the group consisting of hydrogen, halogen, cyano, nitro, C₁-C₆-alkyl, C₁-C₆-haloalkyl, and C₃-C₆-cycloalkyl;
m is an integer 0 to 6; and
R³, R⁴, A, n, p, and Q are each as defined in claim 1;
or
reacting the compound of formula II optionally after converting into a compound of formula X with a compound of formula XI to obtain a compound of formula XII and reacting the compound of formula XII with suitable amine XIII to obtain the compound of formula I, 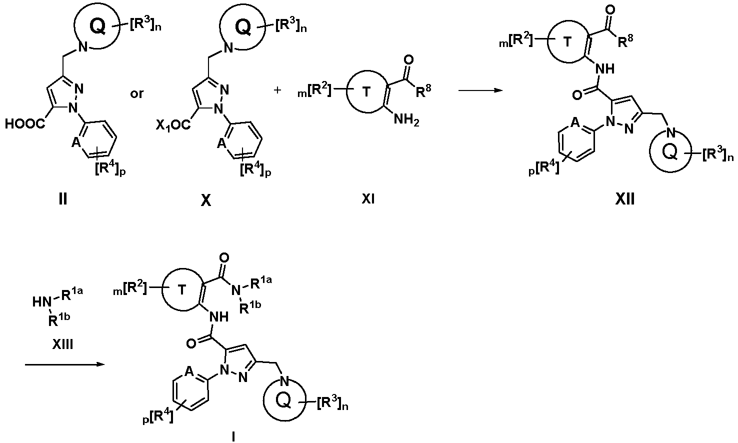 wherein,
X₁ is Cl or Br;
R^{1a} and R^{1b} are independently selected from the group consisting of hydrogen, C₁-C₆-alkyl, C₁-C₆-haloalkyl, C₃-C₆-cycloalkyl, (C₁-C₆-alkyl)-C₃-C₆-cycloalkyl, and (C₃-C₆-cycloalkyl)-C₁-C₆-alkyl; or
R^{1a} and R^{1b} together with the N atom to which they are attached form N=S(=O)₀₋₂(C₁-C₆-alkyl)₂;
T is an aryl or a heteroaryl ring or a fused or a bicyclic aryl or heteroaryl ring or ring system;
R² is selected from the group consisting of hydrogen, halogen, cyano, nitro, C₁-C₆-alkyl, C₁-C₆-haloalkyl, and C₃-C₆-cycloalkyl;
R⁸ is selected from the group consisting of the group consisting of hydroxy, Cl and OR⁷; m is an integer 0 to 6; and
R³, R⁴, R⁷, A, n, p, and Q are each as defined in claim 1.

15. The process as claimed in claims 1 and 14, wherein
R^{1a} is hydrogen; R^{1b} is selected from the group consisting of hydrogen, C₁-C₆-alkyl and (C₃-C₆-cycloalkyl)-C₁-C₆-alkyl;
R² is selected from the group consisting of halogen, cyano, and C₁-C₆-alkyl;
R³ is C₁-C₆-haloalkyl;
R⁴ is X;
R⁶ is CX₃ or C(=O)W²R⁷,
wherein, R⁷ is selected from the group consisting of hydrogen, C₁-C₆-alkyl, C₃-C₆-cycloalkyl, aryl and arylalkyl groups;
W¹ and W² are O;
LG₁ is X;
LG₂ is X or OR¹²; R¹² is C₁-C₆-alkyl;
LG₃ is hydrogen or alkali metal;
A is N;
Q is a 3-, 4- or 5 membered heterocyclic ring;
T is a phenyl ring;
Y is X;
n is an integer 1;
m is an integer 2;
p is an integer 1 or 2; and
each X is independently F, Cl, Br or I.

16. The process as claimed in claims 1 and 14, wherein
R^{1a} is hydrogen; R^{1b} is C₁-C₆-alkyl;
R² is selected from the group consisting of Cl, cyano, and methyl;
R³ is trifluoro methyl;
R⁴ is Cl;
R⁶ is CCl₃, CBr₃, C(=O)W² CH₃, or C(=O)W²C₂H₅,
W¹ and W² are O;
LG₁ is Cl, Br or I;
LG₂ is Cl, Br, I, OCH₃ or OC₂H₅;
LG₃ is alkali metal;
A is N;
Q is a tetrazole ring;
T is a phenyl ring;
Y is Cl;
n is an integer 1;
m is an integer 2; and
p is an integer 1.

17. The process as claimed in claim 6, wherein
R³ is C₁-C₆-haloalkyl;
R⁴ is X;
R⁶ is CX₃ or C(=O)W²R⁷,
wherein, R⁷ is selected from the group consisting of hydrogen, C₁-C₆-alkyl, C₃-C₆-cycloalkyl, aryl and arylalkyl;
W¹ and W² are O;
LG₁ is X;
LG₂ is X or OR¹²; R¹² is C₁-C₆-alkyl;
LG₃ is hydrogen or alkali metal;
A is N;
Q is a 3-, 4- or 5 membered heterocyclic ring;
n is an integer 1;
p is an integer 1 or 2; and
each X is independently F, Cl, Br or I.

18. The process as claimed in claim 6, wherein
R³ is trifluoro methyl;
R⁴ is Cl;
R⁶ is CCl₃, CBr₃, C(=O)W² CH₃, or C(=O)W²C₂H₅,
W¹ and W² are O;
LG₁ is Cl, Br or I;
LG₂ is Cl, Br, I, OCH₃ or OC₂H₅;
LG₃ is alkali metal;
A is N;
Q is a tetrazole ring;
n is an integer 1; and
p is an integer 1.

19. A compound of formula XIX, 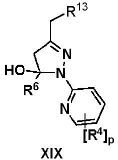 wherein, R¹³ is 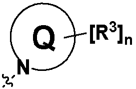 or LG₁; R³, R⁴, R⁶, n, p, and Q are each as defined in claim 1,
with the proviso that when R¹³ is LG₁ then R⁶ is CX₃, wherein LG₁ is Cl, Br or I; and X is F, Cl, Br, or I.

20. The compound as claimed in claim 19, wherein
R⁶ is CX₃ or C(=O)W²R⁷,
wherein, R⁷ is selected from the group consisting of hydrogen, C₁-C₆-alkyl, C₃-C₆-cycloalkyl, aryl and arylalkyl;
W² is O; R³ is C₁-C₆-haloalkyl; R⁴ is X; n is an integer 1; Q is a 5 membered heterocyclic ring; A is N; p is an integer 1 or 2; and each X is independently F, Cl, Br or I.

21. The compound as claimed in claim 19, wherein
R⁶ is CCl₃, CBr₃, C(=O)W²CH₃, C(=O)W²C₂H₅; W² is O; R³ is trifluoro alkyl; R⁴ is Cl; n is an integer 1; Q is a tetrazole ring; A is N; p is an integer 1 and LG₁ is Cl or Br.

22. The compound as claimed in claim 19, wherein the compound of formula XIX is selected from the group consisting of:

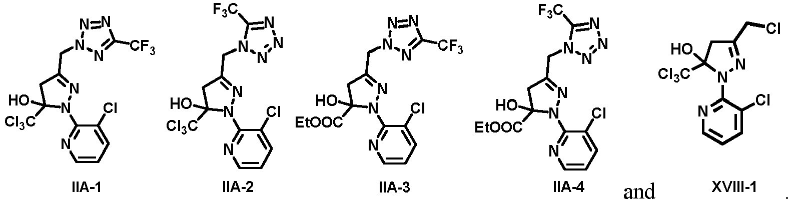

23. A compound of formula XX, 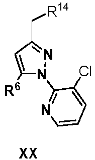
wherein, R¹⁴ is 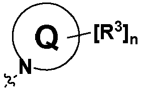 or LG₁ or hydrogen;
R⁶ is CX₃, C(W⁴R⁷)₃, CH(W⁴R⁷)₂, allylic group, substituted or unsubstituted furanyl, 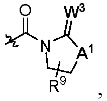 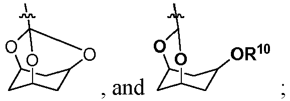
R³, R⁴, R⁷, R⁹, R¹⁰, LG₁, A, A¹, n, p, Q, W³, W⁴, X and the substituents on furanyl are each as defined in claim 1,
with the proviso that when R¹⁴ is LG₁ or hydrogen then R⁶ is CX₃, wherein LG₁ and X are Cl, Br, or I.

24. The compound as claimed in claim 23, wherein
R⁶ is CX₃, R³ is C₁-C₆-haloalkyl; n is an integer 1; Q is a 5 membered heterocyclic ring; A is N; R⁴ is X; p is an integer 1 or 2;

25. The compound as claimed in claim 23, wherein
R⁶ is CCl₃ or CBr₃; R³ is trifluoro alkyl; n is an interger 1; Q is a tetrazole ring; A is N; R⁴ is Cl; p is an integer 1, and LG₁ is Cl or Br.

26. The compound as claimed in claim 23, wherein the compound of formula XX is selected from the group consisting of:

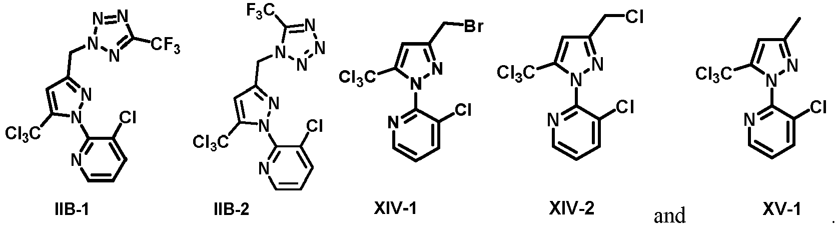

27. A compound of formula III, 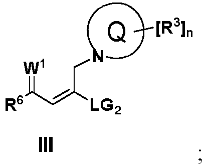 wherein, Q is a 3-, 4- or 5 membered heterocyclic ring excluding triazole ring; R³, R⁶, n, W¹, and LG₂ are each as defined in claim 1.

28. The compounds of formula III as claimed in claim 27, wherein
R⁶ is CX₃ or C(=O)W²R⁷,
wherein, R⁷ is selected from the group consisting of hydrogen, C₁-C₆-alkyl, C₃-C₆-cycloalkyl, aryl and arylalkyl;
W¹ and W² are O; LG₂ is X or OR¹²; R¹² is C₁-C₆-alkyl; R³ is C₁-C₆-haloalkyl; n is an integer 1; Q is a 5 membered heterocyclic ring excluding triazole ring; and each X is independently F, Cl, Br or I.

29. The compounds of formula III as claimed in claim 27, wherein
R⁶ is CCl₃, CBr₃, C(=O)W²CH₃, C(=O)W²C₂H₅; W¹ and W² are O; LG₂ is Cl, Br, I, OCH₃, or OC₂H₅; R³ is trifluoro alkyl; n is an integer 1; and Q is a tetrazole ring.

30. The compound as claimed in claim 27, wherein the compound of formula III is selected from the group consisting of:

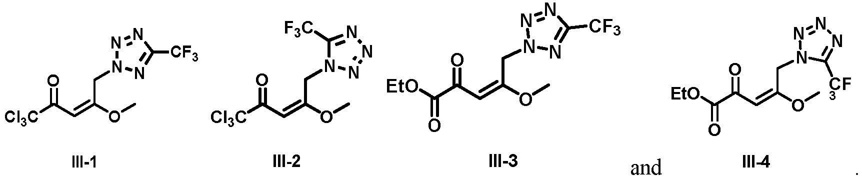

31. A compound of formula IV-2:

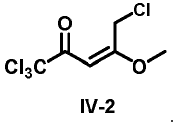

32. A compound of formula XVI, 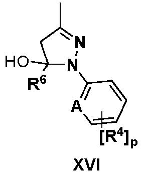 wherein, R⁶ is CX₃, C(W⁴R⁷)₃, CH(W⁴R⁷)₂, allylic group, substituted or unsubstituted furanyl, 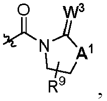 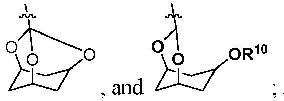 A is N; and R⁴, R⁷, R⁹, R¹⁰, A¹, p, W³, W⁴, and X are each as defined in claim 1.

33. The compound of formula XVI as claimed in claim 32, wherein
R⁶ is CX₃; A is N; R⁴ is X; p is an integer 1 or 2; and each X is independently F, Cl, Br or I.

34. The compound of formula XVI as claimed in claim 32, wherein
R⁶ is CCl₃ or CBr₃; A is N; R⁴ is Cl; and p is an integer 1.

35. The compound as claimed in claim 32, wherein the compound of formula XVI is XVI-1:

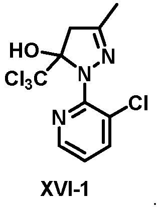

36. A process for preparing a compound of formula II-1 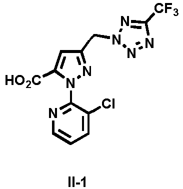 said process comprising the steps of:
a. reacting a compound of formula IV-1 or IV-2 with a compound of formula VII-1 in the presence of one or more suitable solvent and optionally, one or more suitable catalyst and or reagent to obtain a mixture of a compound of formula III-1 and a compound of formula III-2, 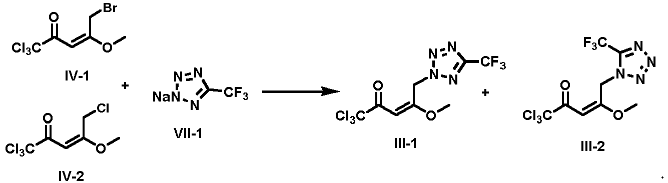
b. cyclizing the compound of formula III-1, or the compound of formula III-1 and the compound of formula III-2 in the mixture, with a hydrazine of formula VIII-1 in one or more suitable solvent and optionally, one or more suitable reagent to obtain a compound of formula IIA-1 or the mixture of the compound of formula IIA-1 and a compound of formula IIA-2 respectively, 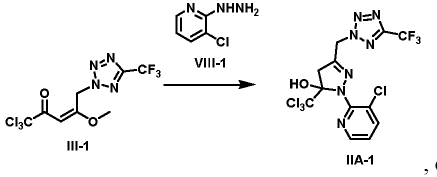 or 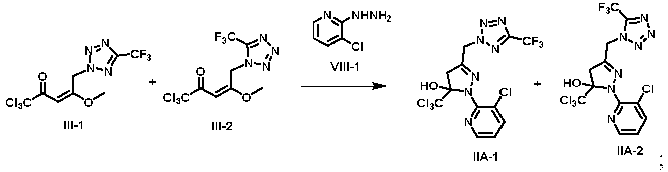
c. eliminating water from the compound of formula IIA-1, or the compound of formula IIA-1 and the compound of formula IIA-2 in the mixture, by using one or more suitable dehydrating reagent in one or more suitable solvent to obtain a compound of formula IIB-1 or the mixture of the compound of formula IIB-1 and a compound of formula IIB-2 respectively, 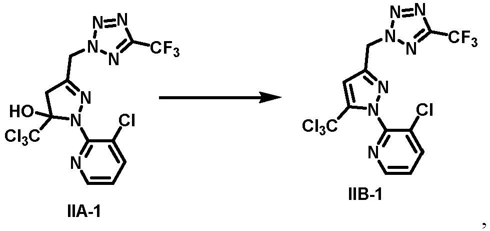 or 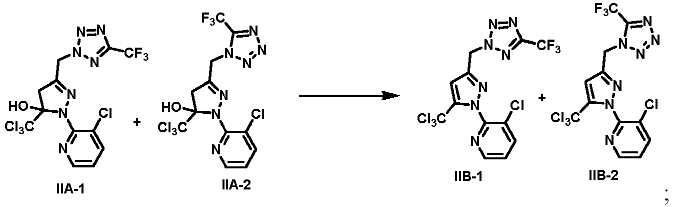 and
d. converting the compound of formula IIB-1, or the compound of formula IIB-1 and the compound of formula IIB-2 in the mixture, into the compound of formula II-1 or the mixture of the compound of formula II-1 and a compound of formula II-2 respectively, using one or more suitable reagent in one or more suitable solvent and optionally, one or more suitable catalyst, 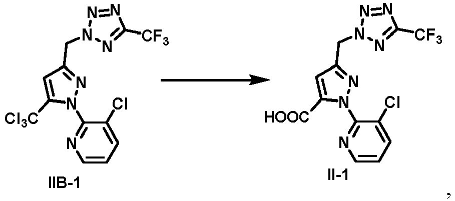 or 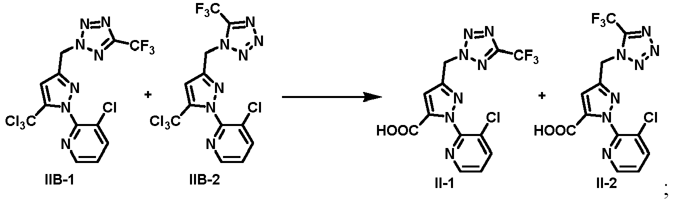 and
wherein, the compound of formula III-1 or the mixture of the compounds of formula III-1 and III-2 obtained in step (a), the compound of formula IIA-1 or the mixture of the compounds of formula IIA-1 and IIA-2 obtained in step (b) and the compound of formula IIB-1 or the mixture of the compounds of formula IIB-1 and IIB-2 obtained in step (c) may or may not be isolated.

37. The process as claimed in claim 36, wherein
i. the suitable solvent in the process step (a) is selected from the group consisting of acetone, acetonitrile, methyl tert-butyl ether, ethyl acetate, dioxane, tetrahydrofuran, 1,2-dimethoxyether;
ii. the suitable catalyst in the process step (a) is potassium iodide; and
iii. the process step (a) is performed within a temperature range from 50 °C to 80 °C.

38. The process as claimed in claim 36, wherein
i. the suitable solvent in the process step (b) is selected from the group consisting of acetone, acetonitrile, ethyl alcohol, acetic acid, methyl tert-butyl ether, dichloroethane, dichloromethane, dioxane, dimethylformamide, dimethylacetamide, dimethyl sulfoxide, ethyl acetate, tetrahydrofuran, 1,2-dimethoxyether, toluene, xylene and N-methyl-2-pyrrolidone;;
ii. the suitable reagent in the process step (b) is selected from the group consisting of acetic acid, trifluoroacetic acid, and hydrochloric acid; and
iii. the process step (b) is performed within a temperature range from 20 °C to 50 °C.

39. The process as claimed in claim 36, wherein
i. the suitable solvent in the process step (c) is selected from the group consisting of acetonitrile, methyl tert-butyl ether, dichloromethane, dioxane, thionyl chloride, acetic acid, methyl alcohol, ethyl alcohol, tetrahydrofuran, isopropyl alcohol and tert-butyl alcohol;
ii. the suitable dehydrating reagent in the process step (c) is selected from the group consisting of sulphuric acid, trifluoroacetic acid, thionyl chloride, oxalyl chloride, methanolic hydrochloric acid, hydrogen chloride gas, acetic acid, hydrogen bromide, triflic acid, methanesulfonic acid, p-toluenesulfonic acid, hydrogen chloride-1,4-dioxane and silica gel; and
iii. the process step (c) is performed within a temperature range from 20 °C to 80 °C.

40. The process as claimed in claim 36, wherein
i. the suitable solvent in the process step (d) is selected from the group consisting of water, acetonitrile, dioxane, acetic acid, sulphuric acid, methyl alcohol, ethyl alcohol, tetrahydrofuran, isopropyl alcohol and tert-butyl alcohol;
ii. the suitable reagent in the process step (d) is an acid selected from the group consisting of sulphuric acid, hydrochloric acid, and acetic acid; and
iii. the process step (d) is performed within a temperature range from 50 °C to 120 °C.

41. The process as claimed in claim 36, wherein the compound of formula IIB-1 is alternatively obtained by the process comprising the steps of:
i. cyclizing a compound of formula XVII-1 with the hydrazine of formula VIII-1 in one or more suitable solvent and optionally, one or more suitable reagent to obtain a compound of formula XVI-1, 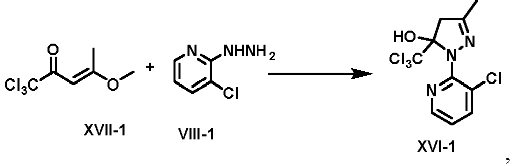 or
cyclizing the compound of formula IV-2 and the hydrazine of formula VIII-1 in one or more suitable solvent and optionally, one or more suitable reagent to obtain a compound of formula XVIII-1, 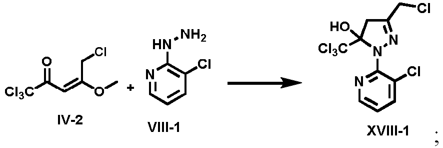
ii. eliminating water from the compound of formula XVI-1 by using one or more suitable dehydrating reagent in one or more suitable solvent to obtain a compound of formula XV-1; 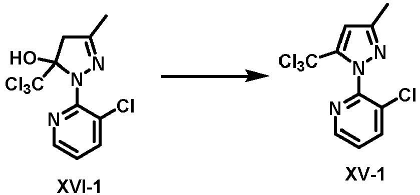 or
eliminating water from the compound of formula XVIII by using one or more suitable dehydrating reagent in one or more suitable solvent to obtain a compound of formula XIV, 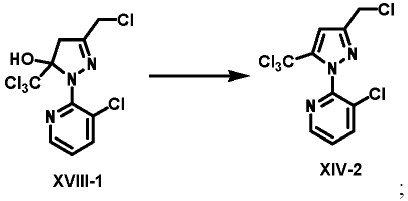
iii. halogenating the compound of formula XV-1 using a suitable halogenating agent in one or more suitable solvent and optionally, one or more radical initiator to obtain a compound of formula XIV-1, 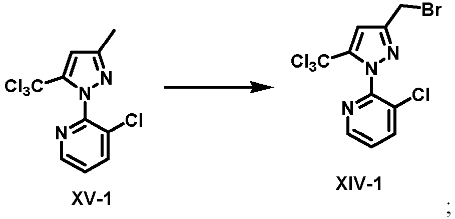 and
iv. obtaining the compound of formula IIB-1 or a mixture of the compound of formula IIB-1 and a compound of formula IIB-2 by reacting the compound of formula XIV-1 or XIV-2 with the compound of formula VII-1, 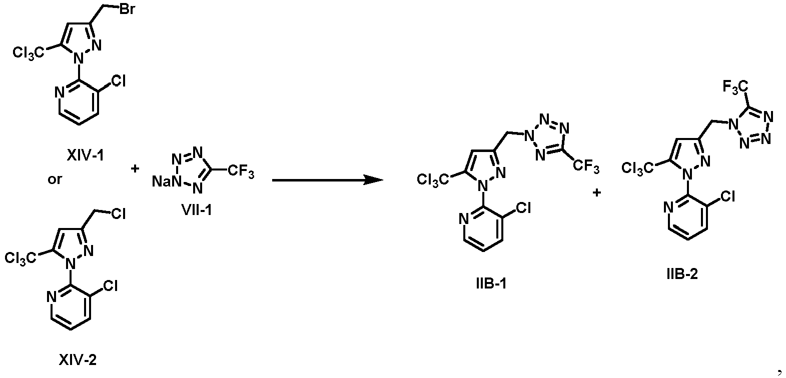 wherein, the compounds of formula XVI-1 and XVIII-1 obtained in step (i), the compounds of formula XV-1 and XIV-2 obtained in step (ii) and the compound XIV-1 obtained in step (iii) may or may not be isolated.

42. The process as claimed in claim 41, wherein
i. the suitable solvent in the process step (i) is selected from the group consisting of acetone, acetonitrile, ethyl alcohol, acetic acid, methyl tert-butyl ether, dichloroethane, dichloromethane, dioxane, dimethylformamide, dimethylacetamide, dimethyl sulfoxide, ethyl acetate, tetrahydrofuran, 1,2-dimethoxyether, toluene, xylene and N-methyl-2-pyrrolidone;;
ii. the suitable reagent in the process step (i) is selected from the group consisting of acetic acid, trifluoroacetic acid, and hydrochloric acid; and
iii. the process step (i) is performed within a temperature range from 20 °C to 50 °C.

43. The process as claimed in claim 41, wherein
i. the suitable solvent in the process step (ii) is selected from the group consisting of acetonitrile, methyl tert-butyl ether, dichloromethane, dioxane, thionyl chloride, acetic acid, methyl alcohol, ethyl alcohol, tetrahydrofuran, isopropyl alcohol and tert-butyl alcohol;
ii. the suitable dehydrating reagent in the process step (ii) is selected from the group consisting of sulphuric acid, trifluoroacetic acid, thionyl chloride, oxalyl chloride, methanolic hydrochloric acid, hydrogen chloride gas, acetic acid, hydrogen bromide, triflic acid, methanesulfonic acid, p-toluenesulfonic acid, hydrogen chloride-1,4-dioxane and silica gel; and
iv. the process step (ii) is performed within a temperature range from 20 °C to 80 °C.

44. The process as claimed in claim 41, wherein
i. the suitable solvent in the step (iii) is selected from the group consisting of acetonitrile, and ethanol;
ii. the halogenating agent in the step (iii) is selected from the group consisting of N-chlorosuccinimide, N-bromosuccinimide and Br₂;
iii. the radical initiator in the step (iii) is selected from the group consisting of dibenzoyl peroxide, hydrogen peroxide, azobisisobutyronitrile, peracetic acid, metachloroperbenzoic acid, trifluoroperacetic acid, trichloroperacetic acid; and
iv. the step (iii) is performed within a temperature range from 30 °C to 100 °C.

45. The process as claimed in claim 41, wherein
i. the suitable solvent in the step (iv) is selected from the group consisting of acetonitrile, and ethanol;
ii. the catalyst in the step (iv) is potassium iodide; and
iii. the step (iv) is performed within a temperature range from 30 °C to 100 °C.

46. A process for preparing a compound of formula II-1 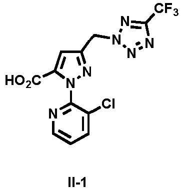 said process comprising the steps of:
a. reacting a compound of formula IV-3 or IV-4 with a compound of formula VII-1 in the presence of one or more suitable solvent and optionally, one or more suitable catalyst and or reagent to obtain a mixture of a compound of formula III-3 and a compound of formula III-4 , 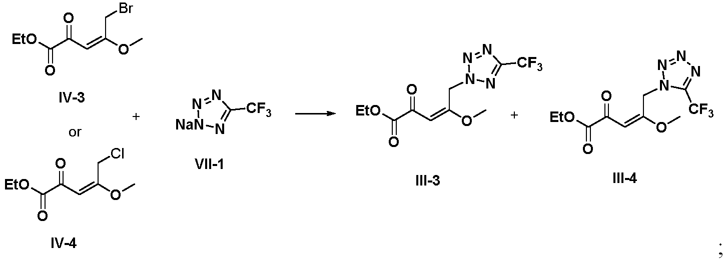
b. cyclizing the compound of formula III-3, or the compound of formula III-3 and the compound of formula III-4 in the mixture, with a hydrazine of formula VIII-1 in one or more suitable solvent and optionally, one or more suitable reagent to obtain a compound of formula IIA-3 or the mixture of the compound of formula IIA-3 and a compound of formula IIA-4 respectively, 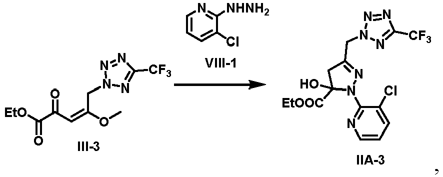 or 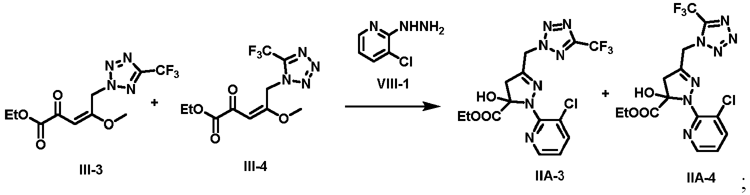
c. eliminating water from the compound of formula IIA-3, or the compound of formula IIA-3 and the compound of formula IIA-4 in the mixture, by using one or more suitable dehydrating reagent in one or more suitable solvent to obtain a compound of formula IIB-3 or the mixture of the compound of formula IIB-3 and a compound of formula IIB-4 respectively, 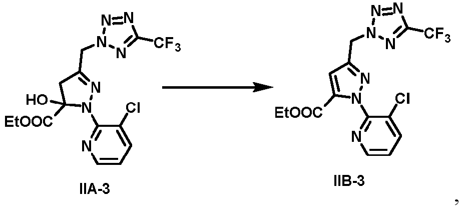 or 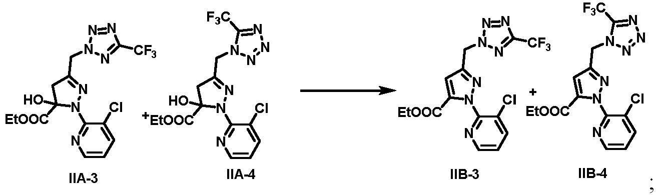 and
d. converting the compound of formula IIB-3, or the compound of formula IIB-3 and the compound of formula IIB-4 in the mixture, into the compound of formula II-1 or the mixture of the compound of formula II-1 and a compound of formula II-2 respectively, using one or more suitable reagent in one or more suitable solvent and optionally, one or more suitable catalyst, 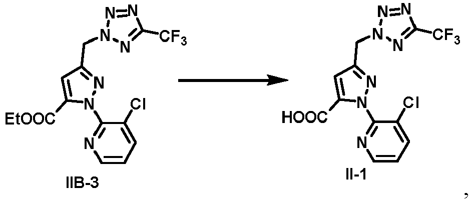 or 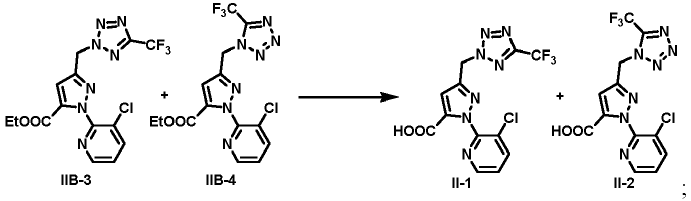 and
wherein, the compound of formula III-3 or the mixture of the compounds of formula III-3 and III-4 obtained in step (a), the compound of formula IIA-3 or the mixture of the compounds of formula IIA-3 and IIA-4 obtained in step (b) and the compound of formula IIB-3 or the mixture of the compounds of formula IIB-3 and IIB-4 obtained in step (c) may or may not be isolated.

47. The process as claimed in claim 46, wherein
i. the suitable solvent in the process step (a) is selected from the group consisting of acetone, acetonitrile, methyl tert-butyl ether, ethyl acetate, dioxane, tetrahydrofuran, 1,2-dimethoxyether;
ii. the suitable catalyst in the process step (a) is potassium iodide; and
iii. the process step (a) is performed within a temperature range from 50 °C to 80 °C.

48. The process as claimed in claim 46, wherein
i. the suitable solvent in the process step (b) is selected from the group consisting of acetone, acetonitrile, ethyl alcohol, acetic acid, methyl tert-butyl ether, dichloroethane, dichloromethane, dioxane, dimethylformamide, dimethylacetamide, dimethyl sulfoxide, ethyl acetate, tetrahydrofuran, 1,2-dimethoxyether, toluene, xylene and N-methyl-2-pyrrolidone;;
ii. the suitable reagent in the process step (b) is selected from the group consisting of acetic acid, trifluoroacetic acid, and hydrochloric acid; and
iii. the process step (b) is performed within a temperature range from 20 °C to 50 °C.

49. The process as claimed in claim 46, wherein
i. the suitable solvent in the process step (c) is selected from the group consisting of acetonitrile, methyl tert-butyl ether, dichloromethane, dioxane, thionyl chloride, acetic acid, methyl alcohol, ethyl alcohol, tetrahydrofuran, isopropyl alcohol and tert-butyl alcohol;
ii. the suitable dehydrating reagent in the process step (c) is selected from the group consisting of sulphuric acid, trifluoroacetic acid, thionyl chloride, oxalyl chloride, methanolic hydrochloric acid, hydrogen chloride gas, acetic acid, hydrogen bromide, triflic acid, methanesulfonic acid, p-toluenesulfonic acid, hydrogen chloride-1,4-dioxane and silica gel; and
iii. the process step (c) is performed within a temperature range from 20 °C to 80 °C.

50. The process as claimed in claim 46, wherein
i. the suitable solvent in the process step (d) is selected from the group consisting of water, acetonitrile, dioxane, acetic acid, sulphuric acid, methyl alcohol, ethyl alcohol, tetrahydrofuran, isopropyl alcohol and tert-butyl alcohol;
ii. the suitable reagent in the process step (d) is an acid selected from the group consisting of sulphuric acid, hydrochloric acid, and acetic acid; and
iii. the process step (d) is performed within a temperature range from 50 °C to 120 °C.

51. The process as claimed in claim 36, wherein said process further comprises the step of:
reacting the compound of formula II-1 optionally after converting into a compound of formula X-1 with a compound of formula IX-1 to obtain the compound of formula I-1, 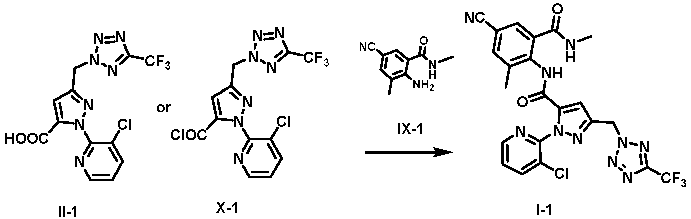 or
reacting the compound of formula II-1 optionally after converting into a compound of formula X-1 with a compound of formula XI to obtain a compound of formula XII and reacting the compound of formula XII with amine XIII-1 to obtain the compound of formula I-1, 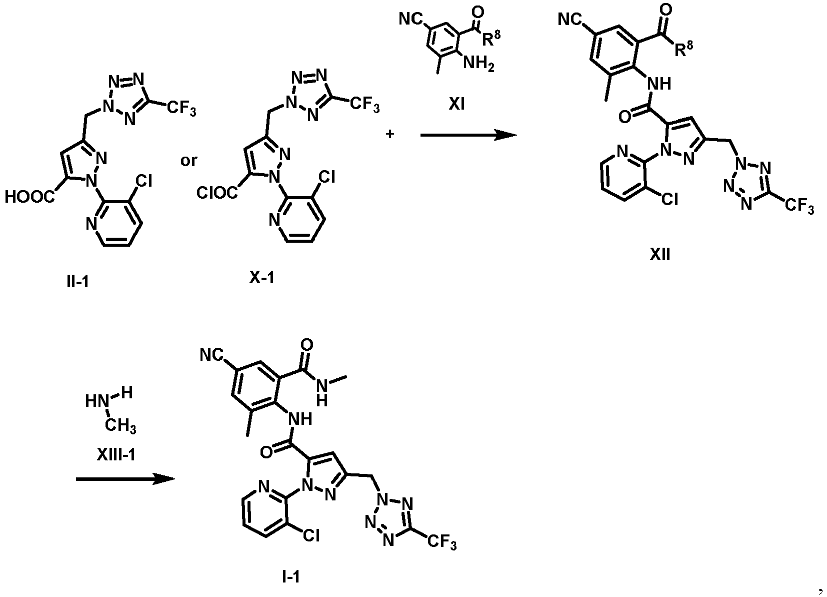
wherein, R⁸ is hydroxy or Cl.

52. The process as claimed in claim 36, wherein said process further comprises the step of:
reacting the compound of formula II-1 optionally after converting into a compound of formula X-1 with a compound of formula IX-1 to obtain the compound of formula I-1, 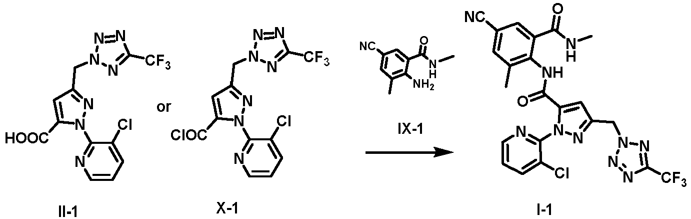 or
reacting the compound of formula II-1 optionally after converting into a compound of formula X-1 with a compound of formula XI to obtain a compound of formula XII and reacting the compound of formula XII with amine XIII-1 to obtain the compound of formula I-1, 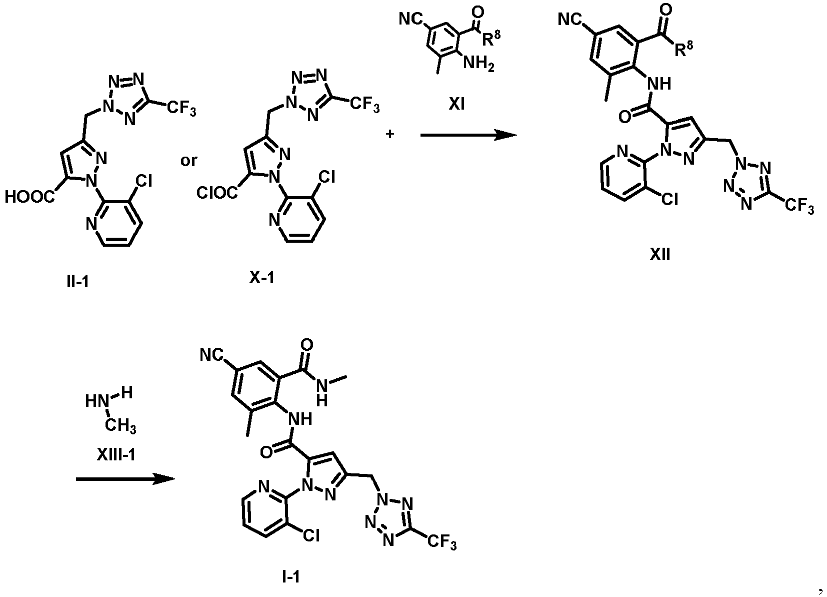
wherein, R⁸ is hydroxy or Cl.

## Patentansprüche

1. Verfahren zur Herstellung einer Verbindung der Formel II 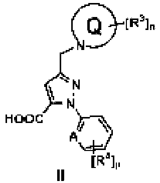 wobei
A N oder C ist;
R³ und R⁴ unabhängig ausgewählt sind aus der Gruppe bestehend aus Wasserstoff, Halogen, Cyano, Nitro, Hydroxy, C₁-C₆-Alkyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthio, C₁-C₆-Alkylsulfinyl, C₁-C₆-Alkylsulfonyl, C₁-C₆-Halogenalkylthio, C₁-C₆-Halogenalkylsulfinyl, C₁-C₆-Halogenalkylsulfonyl und NR^{a}R^{b}; R^{a} und R^{b} unabhängig ausgewählt sind aus der Gruppe bestehend aus Wasserstoff, C₁-C₆-Alkyl, C₃-C₆-Cycloalkyl; oder R^{a} und R^{b} zusammen mit dem N-Atom, an das sie gebunden sind, einen substituierten oder unsubstituierten 3- bis 6-gliedrigen heterocyclischen Ring bilden,
wobei die Substituenten an dem 3- bis 6-gliedrigen heterocyclischen Ring ausgewählt sind aus der Gruppe bestehend aus Halogen, Cyano, Nitro, Hydroxy, C₁-C₆-Alkyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthio, C₁-C₆-Alkylsulfinyl, C₁-C₆-Alkylsulfonyl, C₁-C₆-Halogenalkylthio, C₁-C₆-Halogenalkylsulfinyl und C₁-C₆-Halogenalkylsulfonyl;
Q ein 3-, 4- oder 5-gliedriger heterocyclischer Ring ist;
n eine ganze Zahl von 0 bis 4 ist; und
p eine ganze Zahl von 0 bis 5 ist;
wobei das Verfahren die Schritte umfasst:
a. Umsetzen einer Verbindung der Formel IV mit einer Verbindung der Formel VII in Gegenwart von einem oder mehreren geeigneten Lösungsmitteln und gegebenenfalls einem oder mehreren geeigneten Katalysatoren und/oder Reagenzien, um eine Verbindung der Formel III zu erhalten: 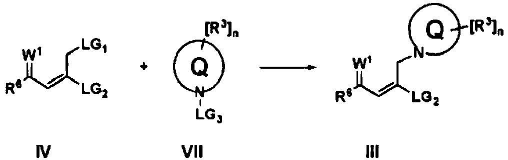 wobei
R⁶ ausgewählt ist aus der Gruppe bestehend aus CX₃, COW²(R⁷), C(W⁴R⁷)₃, CH(W⁴R⁷)₂, Allylgruppe, substituiertem oder unsubstituiertem Furanyl, 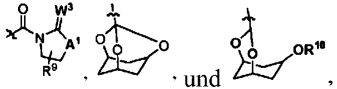 wobei die Substitution an der Furanylgruppe ausgewählt ist aus der Gruppe bestehend aus Halogen, Cyano, Nitro, Hydroxy, C₁-C₆-Alkyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthio, C₁-C₆-Alkylsulfinyl, C₁-C₆-Alkylsulfonyl, C₁-C₆-Halogenalkylthio, C₁-C₆-Halogenalkylsulfinyl oder C₁-C₆-Halogenalkylsulfonyl;
W¹, W², W³, W⁴ und A¹ unabhängig O, S oder NR^{1c} sind; wobei R^{1c} Wasserstoff, C₁-C₆-Alkyl oder C₃-C₆-Cycloalkyl ist;
R⁷ ausgewählt ist aus der Gruppe bestehend aus Wasserstoff, substituiertem oder unsubstituiertem C₁-C₆-Alkyl, substituiertem oder unsubstituiertem C₃-C₆-Cycloalkyl, substituiertem oder unsubstituiertem Aryl und substituiertem oder unsubstituiertem Arylalkyl, oder
zwei R⁷ zusammen mit dem Atom, an das sie gebunden sind, einen substituierten oder unsubstituierten 3- bis 6-gliedrigen carbocyclischen oder heterocyclischen Ring bilden,
wobei die Substitution an C₁-C₆-Alkyl, C₃-C₆-Cycloalkyl, Aryl und Arylalkyl von R⁷ und carbocyclischem oder heterocyclischem Ring, gebildet durch zwei R⁷, unabhängig ausgewählt ist aus der Gruppe bestehend aus Halogen, Cyano, Nitro, Hydroxy, C₁-C₆-Alkyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthio, C₁-C₆-Alkylsulfinyl, C₁-C₆-Alkylsulfonyl, C₁-C₆-Halogenalkylthio, C₁-C₆-Halogenalkylsulfinyl und C₁-C₆-Halogenalkylsulfonyl;
R⁹ und R¹⁰ unabhängig ausgewählt sind aus der Gruppe bestehend aus Wasserstoff, Halogen, Cyano, substituiertem oder unsubstituiertem C₁-C₆-Alkyl, substituiertem oder unsubstituiertem C₁-C₆-Alkoxy, substituiertem oder unsubstituiertem C₃-C₆-Cycloalkyl, substituiertem oder unsubstituiertem C₁-C₆-Alkylthio, substituiertem oder unsubstituiertem C₁-C₆-Alkylsulfinyl, substituiertem oder unsubstituiertem C₁-C₆-Alkylsulfonyl, substituiertem oder unsubstituiertem Aryl und substituiertem oder unsubstituiertem Arylalkyl,
wobei die Substitution an C₁-C₆-Alkyl, C₁-C₆-Alkoxy, C₃-C₆-Cycloalkyl, C₁-C₆-Alkylthio, C₁-C₆-Alkylsulfinyl, C₁-C₆-Alkylsulfonyl, Aryl und Arylalkyl von R⁹ und R¹⁰ ausgewählt ist aus der Gruppe bestehend aus Halogen, Cyano, Nitro, Hydroxy, C₁-C₆-Alkyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthio, C₁-C₆-Alkylsulfinyl, C₁-C₆-Alkylsulfonyl, C₁-C₆-Halogenalkylthio, C₁-C₆-Halogenalkylsulfinyl und C₁-C₆-Halogenalkylsulfonyl;
LG₁ ausgewählt ist aus der Gruppe bestehend aus X, OR⁵ und OSi(R¹¹)₃,
wobei R⁵ ausgewählt ist aus der Gruppe bestehend aus Wasserstoff, substituiertem oder unsubstituiertem C₁-C₆-Alkyl, substituiertem oder unsubstituiertem Aryl-C₁-C₆-alkyl, substituiertem oder unsubstituiertem Aryl, -(C=O)-C₁-C₆-Alkyl, -(C=O)-C₁-C₆-Halogenalkyl, - (C=O)O-C₁-C₆-Alkyl, -(C=O)O-Halogen-C₁-C₆-alkyl, SO₂-C₁-C₆-Alkyl, SO₂-C₁-C₆-Halogenalkyl und substituiertem oder unsubstituiertem SO₂-Aryl,
R¹¹ ausgewählt ist aus der Gruppe bestehend aus Wasserstoff, Halogen, substituiertem oder unsubstituiertem C₁-C₆-Alkyl, substituiertem oder unsubstituiertem Aryl-C₁-C₆-alkyl, substituiertem oder unsubstituiertem Aryl, -(C=O)-C₁-C₆-Alkyl, -(C=O)-C₁-C₆-Halogenalkyl, - (C=O)O-C₁-C₆-Alkyl, -(C=O)O-Halogen-C₁-C₆-alkyl, SO₂-C₁-C₆-Alkyl, SO₂-C₁-C₆-Halogenalkyl und substituiertem oder unsubstituiertem SO₂-Aryl;
LG₂ X, OR¹² ist,
wobei R¹² ausgewählt ist aus der Gruppe bestehend aus Wasserstoff, substituiertem oder unsubstituiertem C₁-C₆-Alkyl, substituiertem oder unsubstituiertem Aryl-C₁-C₆-alkyl, substituiertem oder unsubstituiertem Aryl, -(C=O)-C₁-C₆-Alkyl, -(C=O)-C₁-C₆-Halogenalkyl, - (C=O)O-C₁-C₆-Alkyl, -(C=O)O-Halogen-C₁-C₆-alkyl, SO₂-C₁-C₆-Alkyl, SO₂-C₁-C₆-Halogenalkyl, substituiertem oder unsubstituiertem SO₂-Aryl, Alkylthio und NR^{a}R^{b}; R^{a} und R^{b} unabhängig ausgewählt sind aus der Gruppe bestehend aus Wasserstoff, C₁-C₆-Alkyl, C₃-C₆-Cycloalkyl; oder R^{a} und R^{b} zusammen mit dem N-Atom, an das sie gebunden sind, einen substituierten oder unsubstituierten 3- bis 6-gliedrigen heterocyclischen Ring bilden,
wobei die Substitution an C₁-C₆-Alkyl, Aryl-C₁-C₆-alkyl, Aryl und SO₂-Aryl der Gruppe LG₁ und LG₂ ausgewählt ist aus der Gruppe bestehend aus Halogen, Cyano, Nitro, Hydroxy, C₁-C₆-Alkyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthio, C₁-C₆-Alkylsulfinyl, C₁-C₆-Alkylsulfonyl, C₁-C₆-Halogenalkylthio, C₁-C₆-Halogenalkylsulfinyl und C₁-C₆-Halogenalkylsulfonyl;
LG₃ ausgewählt ist aus der Gruppe bestehend aus Wasserstoff, Alkalimetall, Halogen und Si(R¹¹)₃;
jedes X unabhängig Wasserstoff, F, Cl, Br oder I ist;
R³, n und Q jeweils wie oben definiert sind;
b. Cyclisieren der Verbindung der Formel III mit einem Hydrazin der Formel VIII in einem oder mehreren geeigneten Lösungsmitteln und gegebenenfalls einem oder mehreren geeigneten Reagenzien, um eine Verbindung der Formel IIA zu erhalten: 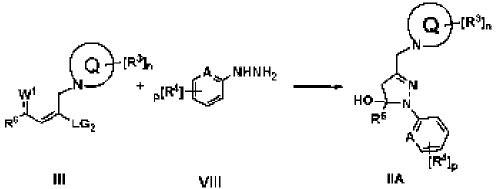 wobei R³, R⁴, R⁶, A, n, p, Q, W¹ und LG₂ jeweils wie zuvor definiert sind;
c. Eliminieren von Wasser aus der Verbindung der Formel IIA unter Verwendung von einem oder mehreren geeigneten Dehydratisierungsreagenzien in einem oder mehreren geeigneten Lösungsmitteln, um eine Verbindung der Formel IIB zu erhalten, 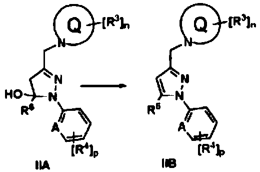 wobei R³, R⁴, R⁶, A, n, p und Q jeweils wie hier zuvor definiert sind; und
d. Konvertieren der Verbindung der Formel IIB in die Verbindung der Formel II unter Verwendung von einem oder mehreren geeigneten Reagenzien in einem oder mehreren geeigneten Lösungsmitteln und gegebenenfalls einem oder mehreren geeigneten Katalysatoren, 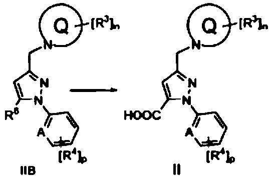
wobei R³, R⁴, R⁶, A, n, p und Q jeweils wie hier zuvor definiert sind; und
wobei die Verbindung der Formel III, die in Schritt (a) erhalten wurde, die Verbindung der Formel IIA, die in Schritt (b) erhalten wurde, und die Verbindung der Formel IIB, die in Schritt (c) erhalten wurde, isoliert werden können oder nicht.

2. Verfahren nach Anspruch 1, wobei
i. das geeignete Lösungsmittel in dem Verfahrensschritt (a) ausgewählt ist aus der Gruppe bestehend aus Aceton, Acetonitril, Methyl-tert-butylether, Chlorbenzol, Dichlorethan, Dichlormethan, Dioxan, Ethylacetat, Dimethylformamid, Dimethylacetamid, Dimethylsulfoxid, 2-Methyltetrahydrofuran, Tetrahydrofuran, 1,2-Dimethoxyether, Toluol, p-Xylol und N-Methyl-2-pyrrolidon;
ii. der geeignete Katalysator in dem Verfahrensschritt (a) ausgewählt ist aus der Gruppe bestehend aus Kaliumiodid, Natriumiodid, Kupferiodid und Kupfer(II)iodid, und
iii. der Verfahrensschritt (a) in einem Temperaturbereich von 20 °C bis 150 °C durchgeführt wird.

3. Verfahren nach Anspruch 1, wobei
i. das geeignete Lösungsmittel in dem Verfahrensschritt (b) ausgewählt ist aus der Gruppe bestehend aus Aceton, Acetonitril, Ethylalkohol, Essigsäure, Methyl-tert-butylether, Chlorbenzol, Dichlorethan, Dichlormethan, Dioxan, Dimethylformamid, Dimethylacetamid, Dimethylsulfoxid, Ethylacetat, 2-Methyltetrahydrofuran, Tetrahydrofuran, 1,2-Dimethoxyether, Toluol, Xylol und N-Methyl-2-pyrrolidon;
ii. das geeignete Reagenz in dem Verfahrensschritt (b) ausgewählt ist aus der Gruppe bestehend aus Essigsäure, Trifluoressigsäure, Salzsäure, Bromwasserstoffsäure, Schwefelsäure, Methansulfonsäure, Trifluormethansulfonsäure, Phosphorsäure und p-Toluolsulfonsäure; und
iii. der Verfahrensschritt (b) in einem Temperaturbereich von 20 °C bis 150 °C durchgeführt wird.

4. Verfahren nach Anspruch 1, wobei
i. das geeignete Lösungsmittel in dem Verfahrensschritt (c) ausgewählt ist aus der Gruppe bestehend aus Acetonitril, Methyl-tert-butylether, Dichlormethan, Dioxan, Thionylchlorid, Essigsäure, Methylalkohol, Ethylalkohol, Tetrahydrofuran, Isopropylalkohol und tert-Butylalkohol;
ii. das geeignete Dehydratisierungsreagenz in dem Verfahrensschritt (c) ausgewählt ist aus der Gruppe bestehend aus Schwefelsäure, Trifluoressigsäure, Phosphortrichlorid, Phosphoroxychlorid, Thionylchlorid, Essigsäureanhydrid, Trifluoressigsäureanhydrid, Oxalylchlorid, Phosgen, Diphosgen, methanolischer Salzsäure, Chlorwasserstoffgas, Essigsäure, Bromwasserstoff, Trifluormethansulfonsäure, Methansulfonsäure, p-Toluolsulfonsäure, Chlorwasserstoff-1,4-Dioxan und Silikagel; und
iii. der Verfahrensschritt (c) in einem Temperaturbereich von 20 °C bis 150 °C durchgeführt wird.

5. Verfahren nach Anspruch 1, wobei
i. das geeignete Lösungsmittel in dem Verfahrensschritt (d) ausgewählt ist aus der Gruppe bestehend aus Wasser, Acetonitril, Dioxan, Schwefelsäure, Essigsäure, Methylalkohol, Ethylalkohol, Tetrahydrofuran, Isopropylalkohol und tert-Butylalkohol;
ii. das geeignete Reagenz in dem Verfahrensschritt (d) ausgewählt ist aus der Gruppe bestehend aus Schwefelsäure, Chlorschwefelsäure, Salzsäure, Fluorwasserstoffsäure, Hydroborsäure, Phosphorsäure, Essigsäure, Trifluoressigsäure, p-Toluolsulfonsäure, Methansulfonsäure und Trifluormethansulfonsäure; oder
iii. das geeignete Reagenz in dem Verfahrensschritt (d) eine Base ausgewählt aus der Gruppe bestehend aus Trialkylaminen, Pyridin, Alkylpyridinen, Phosphazinen, 1,8-Diazabicyclo[5.4.0]undecen (DBU), Lithiumhydroxid, Natriumhydroxid, Kaliumhydroxid, Natriumcarbonat, Kaliumcarbonat, Natriumacetat, Kaliumacetat, Lithiumacetat, Natriummethoxid, Natriumethoxid, Natrium-tert-butoxid und Kalium-tert-butoxid ist;
iv. der Katalysator in dem Verfahrensschritt (d) ausgewählt ist aus der Gruppe bestehend aus Eisen(III)chlorid (FeCl₃), Aluminiumchlorid (AlCl₃), Bortrifluorid (BF₃), Antimontrichlorid (SbCl₃) und Mononatriumphosphat (NaH₂PO₄); und
v. der Verfahrensschritt (d) in einem Temperaturbereich von 20 °C bis 150 °C durchgeführt wird.

6. Verfahren nach Anspruch 1, wobei die Verbindung der Formel IIB alternativ nach dem Verfahren erhalten wird, welches die Schritte umfasst:
i. Cyclisieren einer Verbindung der Formel XVII mit dem Hydrazin der Formel VIII in einem oder mehreren geeigneten Lösungsmitteln und gegebenenfalls einem oder mehreren geeigneten Reagenzien, um eine Verbindung der Formel XVI zu erhalten: 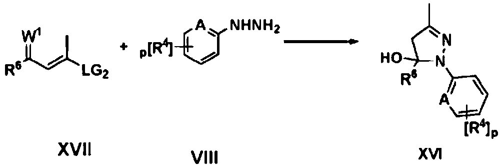
wobei R⁶ CX₃ ist, X F, Cl, Br und I ist; R⁴, A, p, W¹ und LG₂ jeweils wie in Anspruch 1 definiert sind;
oder
Cyclisieren der Verbindung der Formel IV und des Hydrazins der Formel VIII in einem oder mehreren geeigneten Lösungsmitteln und gegebenenfalls einem oder mehreren geeigneten Reagenzien, um eine Verbindung der Formel XVIII zu erhalten: 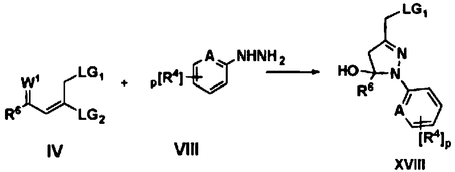
wobei R⁶ CX₃ ist, LG₁ Cl ist; X F, Cl, Br und I ist, vorzugsweise Cl; R⁴, A, p, W¹ und LG₂ jeweils wie in Anspruch 1 definiert sind;
ii. Eliminieren von Wasser aus der Verbindung der Formel XVI unter Verwendung von einem oder mehreren geeigneten Dehydratisierungsreagenzien in einem oder mehreren geeigneten Lösungsmitteln, um eine Verbindung der Formel XV zu erhalten, 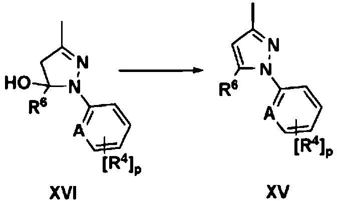
wobei R⁶ CX₃ ist, X F, Cl, Br und I ist; R⁴, A und p jeweils wie in Anspruch 1 definiert sind;
oder
Eliminieren von Wasser aus der Verbindung der Formel XVIII unter Verwendung von einem oder mehreren geeigneten Dehydratisierungsreagenzien in einem oder mehreren geeigneten Lösungsmitteln, um eine Verbindung der Formel XIV zu erhalten, 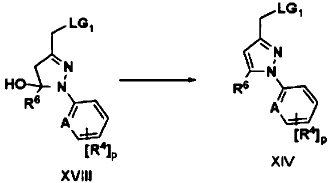
wobei R⁶ CX₃ ist, LG₁ Cl ist, X F, Cl, Br und I ist; R⁴, A und p jeweils wie in Anspruch 1 definiert sind;
iii. Halogenieren der Verbindung der Formel XV unter Verwendung eines geeigneten Halogenierungsmittels in einem oder mehreren geeigneten Lösungsmitteln und gegebenenfalls einem oder mehreren Radikalinitiatoren, um eine Verbindung der Formel XIV zu erhalten, 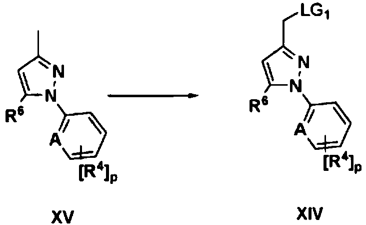 wobei R⁶ CX₃ ist, LG₁ X ist, X F, Cl, Br und I ist; R⁴, A und p jeweils wie in Anspruch 1 definiert sind; und
iv. Erhalten der Verbindung der Formel IIB durch Umsetzen der Verbindung der Formel XIV mit der Verbindung der Formel VII, 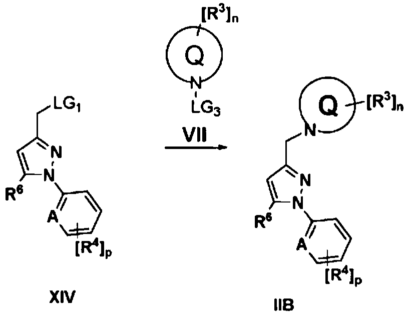
wobei R⁶ CX₃ ist, LG₁ X ist, X F, Cl, Br und I ist; R³, R⁴, A, Q, n, p und LG₃ jeweils wie in Anspruch 1 definiert sind; und
wobei die Verbindungen der Formel XVI und XVIII, die in Schritt (i) erhalten wurden, die Verbindungen der Formel XV und XIV, die in Schritt (ii) erhalten wurden, und die Verbindung der Formel XIV, die in Schritt (iii) erhalten wurde, isoliert werden können oder nicht.

7. Verfahren nach Anspruch 6, wobei
i. das geeignete Lösungsmittel in Schritt (i) ausgewählt ist aus der Gruppe bestehend aus Aceton, Acetonitril, Ethylalkohol, Methyl-tert-butylether, Chlorbenzol, Dichlorethan, Dichlormethan, Dioxan, Dimethylformamid, Dimethylacetamid, Dimethylsulfoxid, Ethylacetat, 2-Methyltetrahydrofuran, Tetrahydrofuran, 1,2-Dimethoxyether, Toluol, Xylol und N-Methyl-2-pyrrolidon;
ii. das geeignete Reagenz in dem Schritt (i) ausgewählt ist aus der Gruppe bestehend aus Essigsäure, Trifluoressigsäure, Salzsäure, Bromwasserstoffsäure, Schwefelsäure, Methansulfonsäure, Trifluormethansulfonsäure, Phosphorsäure und p-Toluolsulfonsäure; und
iii. der Schritt (i) in einem Temperaturbereich von 20 °C bis 150 °C durchgeführt wird.

8. Verfahren nach Anspruch 6, wobei
i. das geeignete Lösungsmittel in dem Schritt (ii) ausgewählt ist aus der Gruppe bestehend aus Wasser, Acetonitril, Methyl-tert-butylether, Dichlormethan, Dioxan, Thionylchlorid, Essigsäure, Methylalkohol, Ethylalkohol, Tetrahydrofuran, Isopropylalkohol und tert-Butylalkohol;
ii. das geeignete Dehydratisierungsreagenz in dem Schritt (ii) ausgewählt ist aus der Gruppe bestehend aus Schwefelsäure, Trifluoressigsäure, Phosphortrichlorid, Phosphoroxychlorid, Thionylchlorid, Essigsäureanhydrid, Trifluoressigsäureanhydrid, Oxalylchlorid, Phosgen, Diphosgen, methanolischer Salzsäure, Chlorwasserstoffgas, Essigsäure, Bromwasserstoff, Trifluormethansulfonsäure, Methansulfonsäure, p-Toluolsulfonsäure und Silikagel; und
iii. der Schritt (ii) in einem Temperaturbereich von 20 °C bis 150 °C durchgeführt wird.

9. Verfahren nach Anspruch 6, wobei
i. das geeignete Lösungsmittel in dem Schritt (iii) ausgewählt ist aus der Gruppe bestehend aus Aceton, Acetonitril, Methyl-tert-butylether, Chlorbenzol, Dichlorethan, Dichlormethan, Dioxan, Ethylacetat, Dimethylformamid, Dimethylacetamid, Dimethylsulfoxid, 2-Methyltetrahydrofuran, Tetrahydrofuran, 1,2-Dimethoxyether, Toluol, p-Xylol und N-Methyl-2-pyrrolidon;
ii. das Halogenierungsmittel in dem Schritt (iii) ausgewählt ist aus der Gruppe bestehend aus N-Halogensuccinimid: N-Chlorsuccinimid, N-Bromsuccinimid und N-Iodsuccinimid; X₂: Cl₂, Br₂ oder I₂; X₂/hν: Cl₂/hν, Br₂/hν oder I₂/hν; N-Halogensaccharin: N-Chlorsaccharin, N-Bromsaccharin und N-Iodsaccharin; und Halogenhydantoin: N-Chlorhydantoin, N-Bromhydantoin und N-Iodhydantoin;
iii. der Radikalinitiator in dem Schritt (iii) ausgewählt ist aus der Gruppe bestehend aus Dibenzoylperoxid, Wasserstoffperoxid, Di(n-propyl)-peroxydicarbonat, t-Butylperoxybenzoat, Methylethylketonperoxid, 2,5-Dimethyl-2,5-di(t-butylperoxy)-3-hexin, Di(t-butyl)peroxid, Acetonperoxid, Dicumylperoxid, Azobisisobutyronitril, Bis(2-ethylhexyl)-peroxydicarbonat, (Peroxybis(propan-2,2-diyl))dibenzol, Peressigsäure, Metachlorperbenzoesäure, Payne-Reagenz (Peroxybenzimidsäure), Magnesiummonoperphthalat, Trifluorperessigsäure, Trichlorperessigsäure, 2,4-Dinitroperbenzoesäure, Carosche Säure und Kaliumcaroat (Kaliummonopersulfat); und
iv. der Schritt (iii) in einem Temperaturbereich von 20 °C bis 150 °C durchgeführt wird.

10. Verfahren nach Anspruch 6, wobei
i. das geeignete Lösungsmittel in dem Schritt (iv) ausgewählt ist aus der Gruppe bestehend aus Aceton, Acetonitril, Methyl-tert-butylether, Chlorbenzol, Dichlorethan, Dichlormethan, Dioxan, Ethylacetat, Dimethylformamid, Dimethylacetamid, Dimethylsulfoxid, 2-Methyltetrahydrofuran, Tetrahydrofuran, 1,2-Dimethoxyether, Toluol, p-Xylol und N-Methyl-2-pyrrolidon;
ii. der Katalysator in dem Schritt (iv) ausgewählt ist aus der Gruppe bestehend aus Kaliumiodid, Natriumiodid, Kupferiodid und Kupfer(II)iodid, und
iii. der Schritt (iv) in einem Temperaturbereich von 20 °C bis 150 °C durchgeführt wird.

11. Verfahren nach Anspruch 1, wobei die Verbindung der Formel IV nach den folgenden Verfahrensschritten erhalten wird:
i. Konvertieren einer Verbindung der Formel IV-A in eine Verbindung der Formel IV-B unter Verwendung eines geeigneten Reagenzes in einem oder mehreren geeigneten Lösungsmitteln, 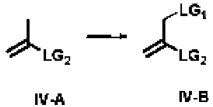 wobei LG₁ und LG₂ jeweils wie in Anspruch 1 definiert sind;
ii. Umsetzen der Verbindung der Formel IV-B mit einer Verbindung der Formel IV-C in einem geeigneten Lösungsmittel und gegebenenfalls unter Verwendung eines geeigneten Reagenzes, um die Verbindung der Formel IV zu erhalten, 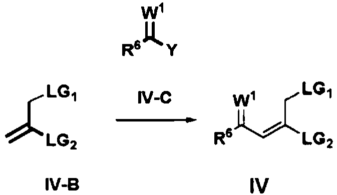
wobei Y OR⁵, X oder -O(C=O)CX₃ ist; X F, Cl, Br oder I ist; R⁵, R⁶, W¹, LG₁ und LG₂ jeweils wie in Anspruch 1 definiert sind,
und
wobei die Verbindung der Formel IV-B isoliert werden kann oder nicht.

12. Verfahren nach Anspruch 11, wobei
i. das geeignete Reagenz in dem Schritt (i) ausgewählt ist aus der Gruppe bestehend aus HX, NaX, KX, CuX₂, MgX₂, CsX, ZnX₂, SOCl₂, SO₂Cl₂, COCl₂, X₂, C(=O)(OCl₃)₂, t-BuOCl, NaOCl, Chloramin-T, N-Halogensuccinimiden, N-Halogensaccharin, Halogenhydantoin, POX₃, PX₃ und PX₅; wobei X oder Halogen Cl, Br, I oder F ist;
ii. das geeignete Lösungsmittel in dem Schritt (i) ausgewählt ist aus der Gruppe bestehend aus Aceton, Acetonitril, Methyl-tert-butylether, Chlorbenzol, Dichlorethan, Dichlormethan, Dioxan, Ethylacetat, Dimethylformamid, Dimethylacetamid, Dimethylsulfoxid, 2-Methyltetrahydrofuran, Tetrahydrofuran, 1,2-Dimethoxyether, Toluol, p-Xylol und N-Methyl-2-pyrrolidon; und
iii. der Schritt (i) in einem Temperaturbereich von 20 °C bis 100 °C durchgeführt wird.

13. Verfahren nach Anspruch 11, wobei
i. das geeignete Reagenz in dem Schritt (ii) ausgewählt ist aus der Gruppe bestehend aus Pyridin, Triethylamin, N,N-Diisopropylethylamin, 2,6-Di-tert-butylpyridin, 1,5-Diazabicyclo(4.3.0)non-5-en, 1,8-Diazabicycloundec-7-en, Lithiumdiisopropylamid, Natriumbis(trimethylsilyl)-amid und Kaliumbis(trimethylsilyl)amid;
ii. das geeignete Lösungsmittel in dem Schritt (ii) ausgewählt ist aus der Gruppe bestehend aus Aceton, Acetonitril, Methyl-tert-butylether, Chlorbenzol, Dichlorethan, Dichlormethan, Dioxan, Ethylacetat, Dimethylformamid, Dimethylacetamid, Dimethylsulfoxid, 2-Methyltetrahydrofuran, Tetrahydrofuran, 1,2-Dimethoxyether, Toluol, p-Xylol und N-Methyl-2-pyrrolidon; und
iii. der Schritt (ii) in einem Temperaturbereich von 20 °C bis 100 °C durchgeführt wird.

14. Verfahren nach Anspruch 1, wobei das Verfahren ferner den Schritt umfasst:
Umsetzen der Verbindung der Formel II, gegebenenfalls nach Konvertieren in eine Verbindung der Formel X, mit einer Verbindung der Formel IX, um die Verbindung der Formel I zu erhalten, 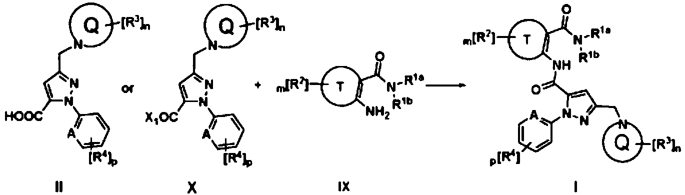 wobei
X₁ Cl oder Br ist;
R^{1a} und R^{1b} unabhängig ausgewählt sind aus der Gruppe bestehend aus Wasserstoff, C₁-C₆-Alkyl, C₁-C₆-Halogenalkyl, C₃-C₆-Cycloalkyl, (C₁-C₆-Alkyl)-C₃-C₆-cycloalkyl und (C₃-C₆-Cycloalkyl)-C₁-C₆-alkyl; oder R^{1a} und R^{1b} zusammen mit dem N-Atom, an das sie gebunden sind, N=S(=O)₀₋₂(C₁-C₆-alkyl)₂ bilden;
T ein Aryl- oder ein Heteroarylring oder ein kondensierter oder ein bicyclischer Aryl- oder Heteroarylring oder entsprechendes Ringsystem ist;
R² ausgewählt ist aus der Gruppe bestehend aus Wasserstoff, Halogen, Cyano, Nitro, C₁-C₆-Alkyl, C₁-C₆-Halogenalkyl und C₃-C₆-Cycloalkyl; m eine ganze Zahl von 0 bis 6 ist; und
R³, R⁴, A, n, p und Q jeweils wie in Anspruch 1 definiert sind;
oder
Umsetzen der Verbindung der Formel II, gegebenenfalls nach Konvertieren in eine Verbindung der Formel X, mit einer Verbindung der Formel XI, um eine Verbindung der Formel XII zu erhalten, und Umsetzen der Verbindung der Formel XII mit geeignetem Amin XIII, um die Verbindung der Formel I zu erhalten, 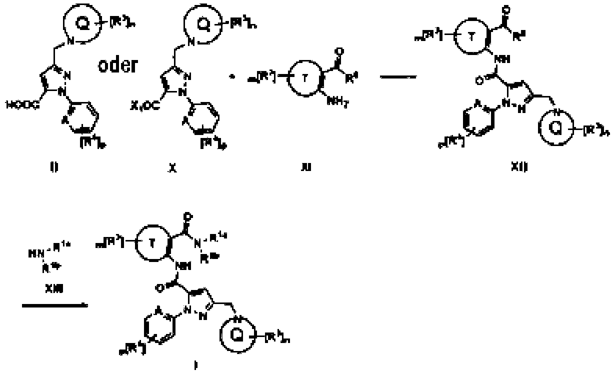 wobei
X₁ Cl oder Br ist;
R^{1a} und R^{1b} unabhängig ausgewählt sind aus der Gruppe bestehend aus Wasserstoff, C₁-C₆-Alkyl, C₁-C₆-Halogenalkyl, C₃-C₆-Cycloalkyl, (C₁-C₆-Alkyl)-C₃-C₆-cycloalkyl und (C₃-C₆-Cycloalkyl)-C₁-C₆-alkyl; oder R^{1a} und R^{1b} zusammen mit dem N-Atom, an das sie gebunden sind, N=S(=O)₀₋₂(C₁-C₆-alkyl)₂ bilden;
T ein Aryl- oder ein Heteroarylring oder ein kondensierter oder ein bicyclischer Aryl- oder Heteroarylring oder entsprechendes Ringsystem ist;
R² ausgewählt ist aus der Gruppe bestehend aus Wasserstoff, Halogen, Cyano, Nitro, C₁-C₆-Alkyl, C₁-C₆-Halogenalkyl und C₃-C₆-Cycloalkyl; R⁸ ausgewählt ist aus der Gruppe bestehend aus Hydroxy, Cl und OR⁷; m eine ganze Zahl von 0 bis 6 ist; und
R³, R⁴, R⁷, A, n, p und Q jeweils wie in Anspruch 1 definiert sind.

15. Verfahren nach Anspruch 1 und 14, wobei
R^{1a} Wasserstoff ist; R^{1b} ausgewählt ist aus der Gruppe bestehend aus Wasserstoff, C₁-C₆-Alkyl und (C₃-C₆-Cycloalkyl)-C₁-C₆-alkyl;
R² ausgewählt ist aus der Gruppe bestehend aus Halogen, Cyano und C₁-C₆-Alkyl;
R³ C₁-C₆-Halogenalkyl ist;
R⁴ X ist;
R⁶ CX₃ oder C(=O)W²R⁷ ist,
wobei R⁷ ausgewählt ist aus der Gruppe bestehend aus Wasserstoff, C₁-C₆-Alkyl-, C₃-C₆-Cycloalkyl-, Aryl- und Arylalkylgruppen;
W¹ und W² O sind;
LG₁ X ist;
LG₂ X oder OR¹² ist; R¹² C₁-C₆-Alkyl ist;
LG₃ Wasserstoff oder Alkalimetall ist;
A N ist;
Q ein 3-, 4- oder 5-gliedriger heterocyclischer Ring ist;
T ein Phenylring ist;
Y X ist;
n eine ganze Zahl von 1 ist;
m eine ganze Zahl von 2 ist;
p eine ganze Zahl von 1 oder 2 ist; und
jedes X unabhängig F, Cl, Br oder I ist.

16. Verfahren nach Anspruch 1 und 14, wobei
R^{1a} Wasserstoff ist; R^{1b} C₁-C₆-Alkyl ist;
R² ausgewählt ist aus der Gruppe bestehend aus Cl, Cyano und Methyl;
R³ Trifluormethyl ist;
R⁴ Cl ist;
R⁶ CCl₃, CBr₃, C(=O)W²CH₃ oder C(=O)W²C₂H₅ ist,
W¹ und W² O sind;
LG₁ Cl, Br oder I ist;
LG₂ Cl, Br, I, OCH₃ oder OC₂H₅ ist;
LG₃ Alkalimetall ist;
A N ist;
Q ein Tetrazolring ist;
T ein Phenylring ist;
Y Cl ist;
n eine ganze Zahl von 1 ist;
m eine ganze Zahl von 2 ist; und
p eine ganze Zahl von 1 ist.

17. Verfahren nach Anspruch 6, wobei
R³ C₁-C₆-Halogenalkyl ist;
R⁴ X ist;
R⁶ CX₃ oder C(=O)W²R⁷ ist,
wobei R⁷ ausgewählt ist aus der Gruppe bestehend aus Wasserstoff, C₁-C₆-Alkyl, C₃-C₆-Cycloalkyl, Aryl und Arylalkyl;
W¹ und W² O sind;
LG₁ X ist;
LG₂ X oder OR¹² ist; R¹² C₁-C₆-Alkyl ist;
LG₃ Wasserstoff oder Alkalimetall ist;
A N ist;
Q ein 3-, 4- oder 5-gliedriger heterocyclischer Ring ist;
n eine ganze Zahl von 1 ist;
p eine ganze Zahl von 1 oder 2 ist; und
jedes X unabhängig F, Cl, Br oder I ist.

18. Verfahren nach Anspruch 6, wobei
R³ Trifluormethyl ist;
R⁴ Cl ist;
R⁶ CCl₃, CBr₃, C(=O)W²CH₃ oder C(=O)W²C₂H₅ ist,
W¹ und W² O sind;
LG₁ Cl, Br oder I ist;
LG₂ Cl, Br, I, OCH₃ oder OC₂H₅ ist;
LG₃ Alkalimetall ist;
A N ist;
Q ein Tetrazolring ist;
n eine ganze Zahl von 1 ist; und
p eine ganze Zahl von 1 ist.

19. Verbindung der Formel XIX, 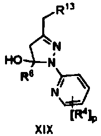
wobei R¹³ 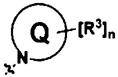 oder LG₁ ist; R³, R⁴, R⁶, n, p und Q jeweils wie in Anspruch 1 definiert sind,
mit der Maßgabe, dass, wenn R¹³ LG₁ ist, dann R⁶ CX₃ ist, wobei LG₁ Cl, Br oder I ist; und X F, Cl, Br oder I ist.

20. Verbindung nach Anspruch 19, wobei
R⁶ CX₃ oder C(=O)W²R⁷ ist,
wobei R⁷ ausgewählt ist aus der Gruppe bestehend aus Wasserstoff, C₁-C₆-Alkyl, C₃-C₆-Cycloalkyl, Aryl und Arylalkyl;
W² O ist; R³ C₁-C₆-Halogenalkyl ist; R⁴ X ist; n eine ganze Zahl 1 ist; Q ein 5-gliedriger heterocyclischer Ring ist; A N ist; p eine ganze Zahl von 1 oder 2 ist; und jedes X unabhängig F, Cl, Br oder I ist.

21. Verbindung nach Anspruch 19, wobei
R⁶ CCl₃, CBr₃, C(=O)W²CH₃, C(=O)W²C₂H₅ ist; W² O ist; R³ Trifluoralkyl ist; R⁴ Cl ist; n eine ganze Zahl von 1 ist; Q ein Tetrazolring ist; A N ist; p eine ganze Zahl von 1 ist, und LG₁ Cl oder Br ist.

22. Verbindung nach Anspruch 19, wobei die Verbindung der Formel XIX ausgewählt ist aus der Gruppe bestehend aus:

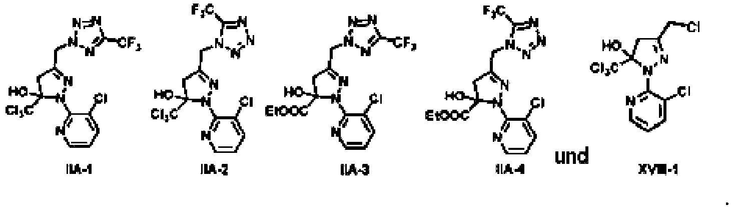

23. Verbindung der Formel XX, 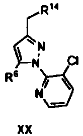
wobei R¹⁴ 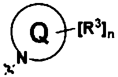 oder LG₁ oder Wasserstoff ist;
R⁶ CX₃, C(W⁴R⁷)₃, CH(W⁴R⁷)₂, Allylgruppe, substituiertes oder unsubstituiertes Furanyl, 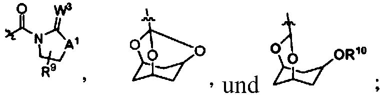 ist;
R³, R⁴, R⁷, R⁹, R¹⁰ LG₁, A, A¹, n, p, Q, W³, W⁴, X und die Substituenten an dem Furanyl jeweils wie in Anspruch 1 definiert sind;
mit der Maßgabe, dass, wenn R¹⁴ LG₁ oder Wasserstoff ist, dann R⁶ CX₃ ist, wobei LG₁ und X F, Cl, Br oder I sind.

24. Verbindung nach Anspruch 23, wobei
R⁶ CX₃ ist, R³ C₁-C₆-Halogenalkyl ist; n eine ganze Zahl von 1 ist; Q ein 5-gliedriger heterocyclischer Ring ist; A N ist; R⁴ X ist; p eine ganze Zahl von 1 oder 2 ist.

25. Verbindung nach Anspruch 23, wobei
R⁶ CCl₃ oder CBr₃ ist; R³ Trifluoralkyl ist; n eine ganze Zahl von 1 ist; Q ein Tetrazolring ist; A N ist; R⁴ Cl ist; p eine ganze Zahl von 1 ist, und LG₁ Cl oder Br ist.

26. Verbindung nach Anspruch 23, wobei die Verbindung der Formel XX ausgewählt ist aus der Gruppe bestehend aus:

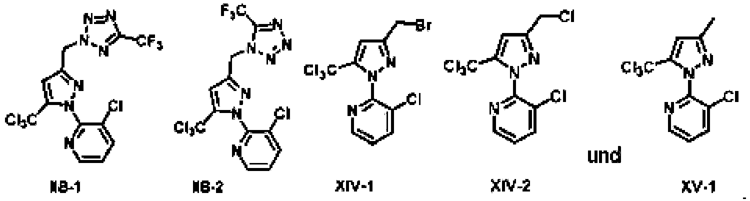

27. Verbindung der Formel III, 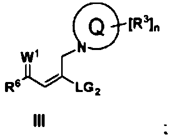 wobei Q ein 3-, 4- oder 5-gliedriger heterocyclischer Ring mit Ausnahme eines Triazolrings ist; R³, R⁶, n, W¹ und LG₂ jeweils wie in Anspruch 1 definiert sind.

28. Verbindungen der Formel III nach Anspruch 27, wobei
R⁶ CX₃ oder C(=O)W²R⁷ ist,
wobei R⁷ ausgewählt ist aus der Gruppe bestehend aus Wasserstoff, C₁-C₆-Alkyl, C₃-C₆-Cycloalkyl, Aryl und Arylalkyl;
W¹ und W² O sind; LG₂ X oder OR¹² ist; R¹² C₁-C₆-Alkyl ist; R³ C₁-C₆-Halogenalkyl ist; n eine ganze Zahl von 1 ist; Q ein 5-gliedriger heterocyclischer Ring mit Ausnahme eines Triazolrings ist; und jedes X unabhängig F, Cl, Br oder I ist.

29. Verbindungen der Formel III nach Anspruch 27, wobei
R⁶ CCl₃, CBr₃, C(=O)W²CH₃, C(=O)W²C₂H₅ ist; W¹ und W² O sind; LG₂ Cl, Br, I, OCH₃ oder OC₂H₅ ist; R³ Trifluoralkyl ist; n eine ganze Zahl von 1 ist; und Q ein Tetrazolring ist.

30. Verbindung nach Anspruch 27, wobei die Verbindung der Formel III ausgewählt ist aus der Gruppe bestehend aus:

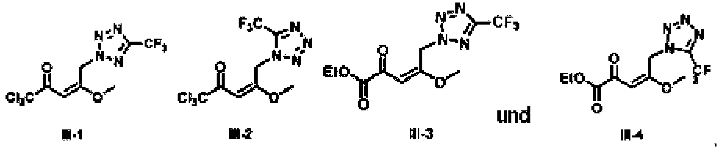

31. Verbindung der Formel IV-2:

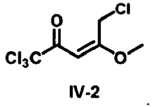

32. Verbindung der Formel XVI, 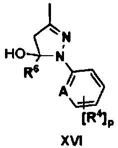 wobei R⁶ CX₃, C(W⁴R⁷)₃, CH(W⁴R⁷)₂, Allylgruppe, substituiertes oder unsubstituiertes Furanyl, 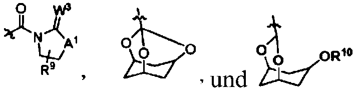 ist; A N ist; und R⁴, R⁷, R⁹, R¹⁰, A¹, p, W³, W⁴ und X jeweils wie in Anspruch 1 definiert sind.

33. Verbindung der Formel XVI nach Anspruch 32, wobei
R⁶ CX₃ ist; A N ist; R⁴ X ist; p eine ganze Zahl von 1 oder 2 ist; und jedes X unabhängig F, Cl, Br oder I ist.

34. Verbindung der Formel XVI nach Anspruch 32, wobei
R⁶ CCl₃ oder CBr₃ ist; A N ist; R⁴ Cl ist; und p eine ganze Zahl von 1 ist.

35. Verbindung nach Anspruch 32, wobei die Verbindung der Formel XVI XVI-1 ist:

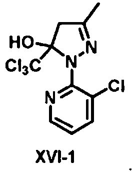

36. Verfahren zur Herstellung einer Verbindung der Formel II-1, 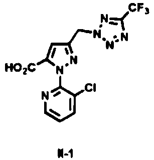 wobei das Verfahren die Schritte umfasst:
a. Umsetzen einer Verbindung der Formel IV-1 oder IV-2 mit einer Verbindung der Formel VII-1 in Gegenwart von einem oder mehreren geeigneten Lösungsmitteln und gegebenenfalls einem oder mehreren geeigneten Katalysatoren und/oder Reagenzien, um eine Mischung einer Verbindung der Formel III-1 und eine Verbindung der Formel III-2 zu erhalten, 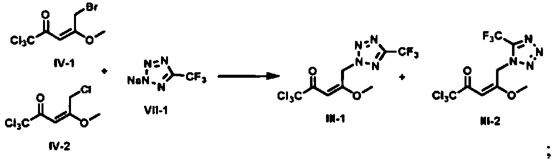
b. Cyclisieren der Verbindung der Formel III-1 oder der Verbindung der Formel III-1 und der Verbindung der Formel III-2 in der Mischung mit einem Hydrazin der Formel VIII-1 in einem oder mehreren geeigneten Lösungsmitteln und gegebenenfalls einem oder mehreren geeigneten Reagenzien, um eine Verbindung der Formel IIA-1 oder die Mischung der Verbindung der Formel IIA-1 und einer Verbindung der Formel IIA-2 zu erhalten, 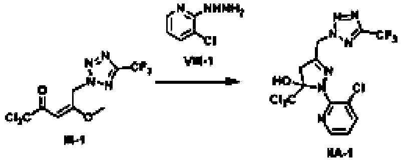 oder 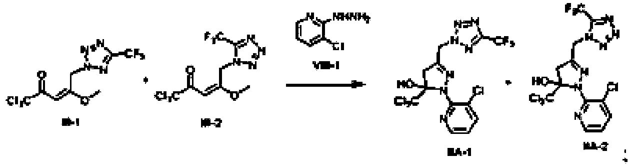
c. Eliminieren von Wasser aus der Verbindung der Formel IIA-1 oder der Verbindung der Formel IIA-1 und der Verbindung der Formel IIA-2 in der Mischung unter Verwendung von einem oder mehreren geeigneten Dehydratisierungsreagenzien in einem oder mehreren geeigneten Lösungsmitteln, um eine Verbindung der Formel IIB-1 oder die Mischung der Verbindung der Formel IIB-1 und einer Verbindung der Formel IIB-2 zu erhalten, 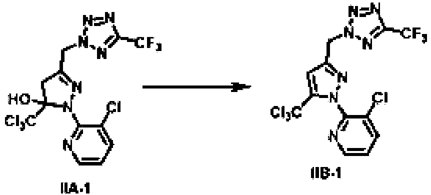 oder 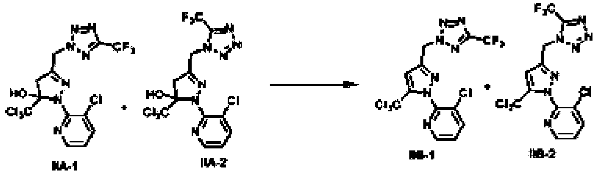 und
d. Konvertieren der Verbindung der Formel IIB-1 oder der Verbindung der Formel IIB-1 und der Verbindung der Formel IIB-2 in der Mischung in die Verbindung der Formel II-1 oder die Mischung der Verbindung der Formel II-1 und einer Verbindung der Formel II-2 unter Verwendung von einem oder mehreren geeigneten Reagenzien in einem oder mehreren geeigneten Lösungsmitteln und gegebenenfalls einem oder mehreren geeigneten Katalysatoren, 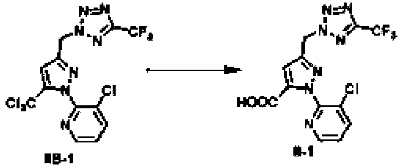 oder 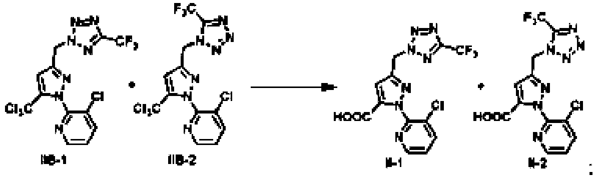
und
wobei die Verbindung der Formel III-1 oder die Mischung der Verbindungen der Formel III-1 und III-2, die in Schritt (a) erhalten wurde, die Verbindung der Formel IIA-1 oder der Mischung der Verbindungen der Formel IIA-1 und IIA-2, die in Schritt (b) erhalten wurde, und die Verbindung der Formel IIB-1 oder die Mischung der Verbindungen der Formel IIB-1 und IIB-2, die in Schritt (c) erhalten wurde, isoliert werden können oder nicht.

37. Verfahren nach Anspruch 36, wobei
i. das geeignete Lösungsmittel in dem Verfahrensschritt (a) ausgewählt ist aus der Gruppe bestehend aus Aceton, Acetonitril, Methyl-tert-butylether, Ethylacetat, Dioxan, Tetrahydrofuran, 1,2-Dimethoxyether;
ii. der geeignete Katalysator in dem Verfahrensschritt (a) Kaliumiodid ist; und
iii. der Verfahrensschritt (a) in einem Temperaturbereich von 50 °C bis 80 °C durchgeführt wird.

38. Verfahren nach Anspruch 36, wobei
i. das geeignete Lösungsmittel in dem Verfahrensschritt (b) ausgewählt ist aus der Gruppe bestehend aus Aceton, Acetonitril, Ethylalkohol, Essigsäure, Methyl-tert-butylether, Dichlorethan, Dichlormethan, Dioxan, Dimethylformamid, Dimethylacetamid, Dimethylsulfoxid, Ethylacetat, Tetrahydrofuran, 1,2-Dimethoxyether, Toluol, Xylol und N-Methyl-2-pyrrolidon;
ii. das geeignete Reagenz in dem Verfahrensschritt (b) ausgewählt ist aus der Gruppe bestehend aus Essigsäure, Trifluoressigsäure und Salzsäure; und
iii. der Verfahrensschritt (b) in einem Temperaturbereich von 20 °C bis 50 °C durchgeführt wird.

39. Verfahren nach Anspruch 36, wobei
i. das geeignete Lösungsmittel in dem Verfahrensschritt (c) ausgewählt ist aus der Gruppe bestehend aus Acetonitril, Methyl-tert-butylether, Dichlormethan, Dioxan, Thionylchlorid, Essigsäure, Methylalkohol, Ethylalkohol, Tetrahydrofuran, Isopropylalkohol und tert-Butylalkohol;
ii. das geeignete Dehydratisierungsreagenz in dem Verfahrensschritt (c) ausgewählt ist aus der Gruppe bestehend aus Schwefelsäure, Trifluoressigsäure, Thionylchlorid, Oxalylchlorid, methanolischer Salzsäure, Chlorwasserstoffgas, Essigsäure, Bromwasserstoff, Trifluormethansulfonsäure, Methansulfonsäure, p-Toluolsulfonsäure, Chlorwasserstoff-1,4-Dioxan und Silikagel; und
iii. der Verfahrensschritt (c) in einem Temperaturbereich von 20 °C bis 80 °C durchgeführt wird.

40. Verfahren nach Anspruch 36, wobei
i. das geeignete Lösungsmittel in dem Verfahrensschritt (d) ausgewählt ist aus der Gruppe bestehend aus Wasser, Acetonitril, Dioxan, Essigsäure, Schwefelsäure, Methylalkohol, Ethylalkohol, Tetrahydrofuran, Isopropylalkohol und tert-Butylalkohol;
ii. das geeignete Reagenz in dem Verfahrensschritt (d) eine Säure ausgewählt aus der Gruppe bestehend aus Schwefelsäure, Salzsäure und Essigsäure ist; und
iii. der Verfahrensschritt (d) in einem Temperaturbereich von 50 °C bis 120 °C durchgeführt wird.

41. Verfahren nach Anspruch 36, wobei die Verbindung der Formel IIB-1 alternativ nach dem Verfahren erhalten wird, welches die Schritte umfasst:
i. Cyclisieren einer Verbindung der Formel XVII-1 mit dem Hydrazin der Formel VIII-1 in einem oder mehreren geeigneten Lösungsmitteln und gegebenenfalls einem oder mehreren geeigneten Reagenzien, um eine Verbindung der Formel XVI-1 zu erhalten, 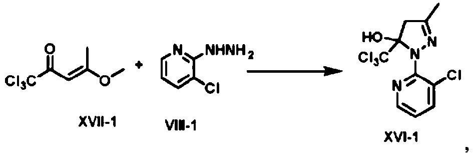 oder
Cyclisieren der Verbindung der Formel IV-2 und des Hydrazins der Formel VIII-1 in einem oder mehreren geeigneten Lösungsmitteln und gegebenenfalls einem oder mehreren geeigneten Reagenzien, um eine Verbindung der Formel XVIII-1 zu erhalten, 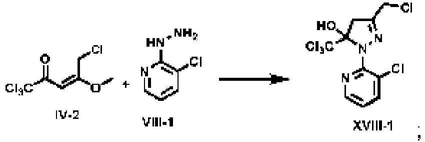
ii. Eliminieren von Wasser aus der Verbindung der Formel XVI-1 unter Verwendung von einem oder mehreren geeigneten Dehydratisierungsreagenzien in einem oder mehreren geeigneten Lösungsmitteln, um eine Verbindung der Formel XV-1 zu erhalten; 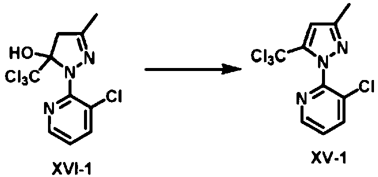 oder
Eliminieren von Wasser aus der Verbindung der Formel XVIII unter Verwendung von einem oder mehreren geeigneten Dehydratisierungsreagenzien in einem oder mehreren geeigneten Lösungsmitteln, um eine Verbindung der Formel XIV zu erhalten, 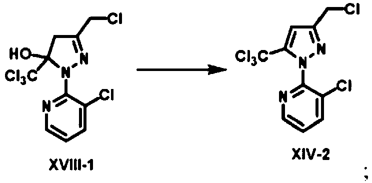
iii. Halogenieren der Verbindung der Formel XV-1 unter Verwendung eines geeigneten Halogenierungsmittels in einem oder mehreren geeigneten Lösungsmitteln und gegebenenfalls einem oder mehreren Radikalinitiatoren, um eine Verbindung der Formel XIV-1 zu erhalten, 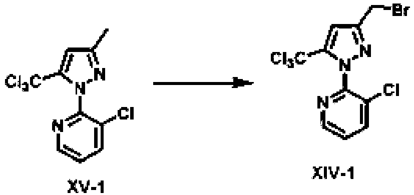 und
iv. Erhalten der Verbindung der Formel IIB-1 oder einer Mischung der Verbindung der Formel IIB-1 und einer Verbindung der Formel IIB-2 durch Umsetzen der Verbindung der Formel XIV-1 oder XIV-2 mit der Verbindung der Formel VII-1, 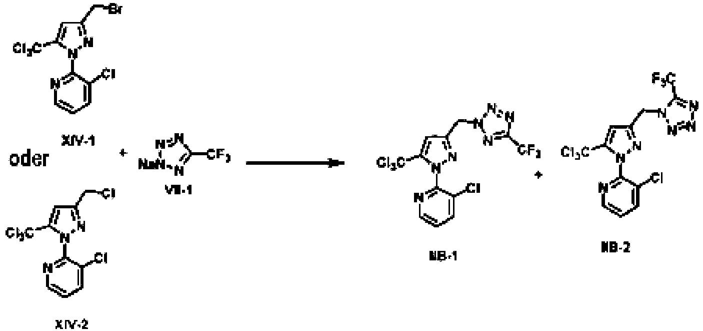 wobei die Verbindungen der Formel XVI-1 und XVIII-1, die in Schritt (i) erhalten wurden, die Verbindungen der Formel XV-1 und XIV-2, die in Schritt (ii) erhalten wurden, und die Verbindung der Formel XIV-1, die in Schritt (iii) erhalten wurde, isoliert werden können oder nicht.

42. Verfahren nach Anspruch 41, wobei
i. das geeignete Lösungsmittel in dem Verfahrensschritt (i) ausgewählt ist aus der Gruppe bestehend aus Aceton, Acetonitril, Ethylalkohol, Essigsäure, Methyl-tert-butylether, Dichlorethan, Dichlormethan, Dioxan, Dimethylformamid, Dimethylacetamid, Dimethylsulfoxid, Ethylacetat, Tetrahydrofuran, 1,2-Dimethoxyether, Toluol, Xylol und N-Methyl-2-pyrrolidon;
ii. das geeignete Reagenz in dem Verfahrensschritt (i) ausgewählt ist aus der Gruppe bestehend aus Essigsäure, Trifluoressigsäure und Salzsäure; und
iii. der Verfahrensschritt (i) in einem Temperaturbereich von 20 °C bis 50 °C durchgeführt wird.

43. Verfahren nach Anspruch 41, wobei
i. das geeignete Lösungsmittel in dem Verfahrensschritt (ii) ausgewählt ist aus der Gruppe bestehend aus Acetonitril, Methyl-tert-butylether, Dichlormethan, Dioxan, Thionylchlorid, Essigsäure, Methylalkohol, Ethylalkohol, Tetrahydrofuran, Isopropylalkohol und tert-Butylalkohol;
ii. das geeignete Dehydratisierungsreagenz in dem Verfahrensschritt (ii) ausgewählt ist aus der Gruppe bestehend aus Schwefelsäure, Trifluoressigsäure, Thionylchlorid, Oxalylchlorid, methanolischer Salzsäure, Chlorwasserstoffgas, Essigsäure, Bromwasserstoff, Trifluormethansulfonsäure, Methansulfonsäure, p-Toluolsulfonsäure, Chlorwasserstoff-1,4-Dioxan und Silikagel; und
iv. der Verfahrensschritt (ii) in einem Temperaturbereich von 20 °C bis 80 °C durchgeführt wird.

44. Verfahren nach Anspruch 41, wobei
i. das geeignete Lösungsmittel in dem Schritt (iii) ausgewählt ist aus der Gruppe bestehend aus Acetonitril und Ethanol;
ii. das Halogenierungsmittel in dem Schritt (iii) ausgewählt ist aus der Gruppe bestehend aus N-Chlorsuccinimid, N-Bromsuccinimid und Br₂;
iii. der Radikalinitiator in dem Schritt (iii) ausgewählt ist aus der Gruppe bestehend aus Dibenzoylperoxid, Wasserstoffperoxid, Azobisisobutyronitril, Peressigsäure, Metachlorperbenzoesäure, Trifluorperessigsäure, Trichlorperessigsäure; und
iv. der Schritt (iii) in einem Temperaturbereich von 30 °C bis 100 °C durchgeführt wird.

45. Verfahren nach Anspruch 41, wobei
i. das geeignete Lösungsmittel in dem Schritt (iv) ausgewählt ist aus der Gruppe bestehend aus Acetonitril und Ethanol;
ii. der Katalysator in dem Schritt (iv) Kaliumiodid ist; und
iii. der Schritt (iv) in einem Temperaturbereich von 30 °C bis 100 °C durchgeführt wird.

46. Verfahren zur Herstellung einer Verbindung der Formel II-1, 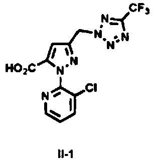 wobei das Verfahren die Schritte umfasst:
a. Umsetzen einer Verbindung der Formel IV-3 oder IV-4 mit einer Verbindung der Formel VII-1 in Gegenwart von einem oder mehreren geeigneten Lösungsmitteln und gegebenenfalls einem oder mehreren geeigneten Katalysatoren und/oder Reagenzien, um eine Verbindung der Formel III-3 und eine Verbindung der Formel III-4 zu erhalten, 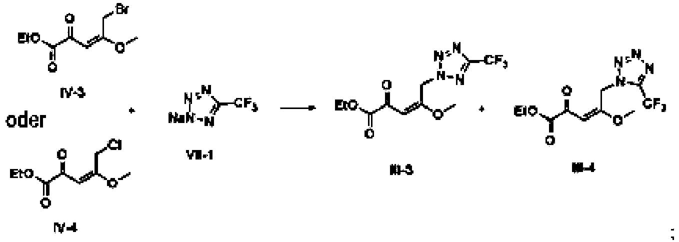
b. Cyclisieren der Verbindung der Formel III-3 oder der Verbindung der Formel III-3 und der Verbindung der Formel III-4 in der Mischung mit einem Hydrazin der Formel VIII-1 in einem oder mehreren geeigneten Lösungsmitteln und gegebenenfalls einem oder mehreren geeigneten Reagenzien, um eine Verbindung der Formel IIA-3 oder die Mischung der Verbindung der Formel IIA-3 und einer Verbindung der Formel IIA-4 zu erhalten, 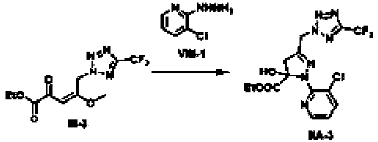 oder 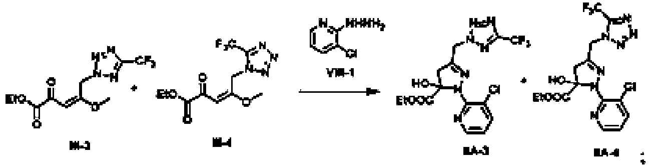
c. Eliminieren von Wasser aus der Verbindung der Formel IIA-3 oder der Verbindung der Formel IIA-3 und der Verbindung der Formel IIA-4 in der Mischung unter Verwendung von einem oder mehreren geeigneten Dehydratisierungsreagenzien in einem oder mehreren geeigneten Lösungsmitteln, um eine Verbindung der Formel IIB-3 oder die Mischung der Verbindung der Formel IIB-3 und einer Verbindung der Formel IIB-4 zu erhalten, 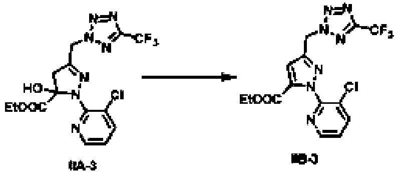 oder 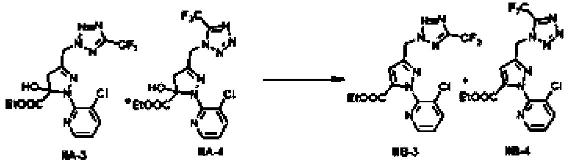 und
d. Konvertieren der Verbindung der Formel IIB-3 oder der Verbindung der Formel IIB-3 und der Verbindung der Formel IIB-4 in der Mischung in die Verbindung der Formel II-1 oder die Mischung der Verbindung der Formel II-1 und einer Verbindung der Formel II-2 unter Verwendung von einem oder mehreren geeigneten Reagenzien in einem oder mehreren geeigneten Lösungsmitteln und gegebenenfalls einem oder mehreren geeigneten Katalysatoren, 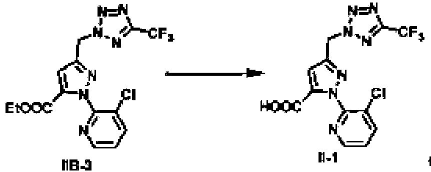 oder 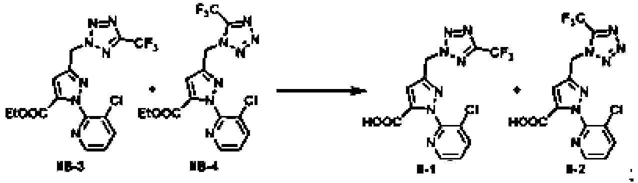 und
wobei die Verbindung der Formel III-3 oder die Mischung der Verbindungen der Formel III-3 und III-4, die in Schritt (a) erhalten wurde, die Verbindung der Formel IIA-3 oder der Mischung der Verbindungen der Formel IIA-3 und IIA-4, die in Schritt (b) erhalten wurde, und die Verbindung der Formel IIB-3 oder die Mischung der Verbindungen der Formel IIB-3 und IIB-4, die in Schritt (c) erhalten wurde, isoliert werden können oder nicht.

47. Verfahren nach Anspruch 46, wobei
i. das geeignete Lösungsmittel in dem Verfahrensschritt (a) ausgewählt ist aus der Gruppe bestehend aus Aceton, Acetonitril, Methyl-tert-butylether, Ethylacetat, Dioxan, Tetrahydrofuran, 1,2-Dimethoxyether;
ii. der geeignete Katalysator in dem Verfahrensschritt (a) Kaliumiodid ist; und
iii. der Verfahrensschritt (a) in einem Temperaturbereich von 50 °C bis 80 °C durchgeführt wird.

48. Verfahren nach Anspruch 46, wobei
i. das geeignete Lösungsmittel in dem Verfahrensschritt (b) ausgewählt ist aus der Gruppe bestehend aus Aceton, Acetonitril, Ethylalkohol, Essigsäure, Methyl-tert-butylether, Dichlorethan, Dichlormethan, Dioxan, Dimethylformamid, Dimethylacetamid, Dimethylsulfoxid, Ethylacetat, Tetrahydrofuran, 1,2-Dimethoxyether, Toluol, Xylol und N-Methyl-2-pyrrolidon;
ii. das geeignete Reagenz in dem Verfahrensschritt (b) ausgewählt ist aus der Gruppe bestehend aus Essigsäure, Trifluoressigsäure und Salzsäure; und
iii. der Verfahrensschritt (b) in einem Temperaturbereich von 20 °C bis 50 °C durchgeführt wird.

49. Verfahren nach Anspruch 46, wobei
i. das geeignete Lösungsmittel in dem Verfahrensschritt (c) ausgewählt ist aus der Gruppe bestehend aus Acetonitril, Methyl-tert-butylether, Dichlormethan, Dioxan, Thionylchlorid, Essigsäure, Methylalkohol, Ethylalkohol, Tetrahydrofuran, Isopropylalkohol und tert-Butylalkohol;
ii. das geeignete Dehydratisierungsreagenz in dem Verfahrensschritt (c) ausgewählt ist aus der Gruppe bestehend aus Schwefelsäure, Trifluoressigsäure, Thionylchlorid, Oxalylchlorid, methanolischer Salzsäure, Chlorwasserstoffgas, Essigsäure, Bromwasserstoff, Trifluormethansulfonsäure, Methansulfonsäure, p-Toluolsulfonsäure, Chlorwasserstoff-1,4-Dioxan und Silikagel; und
iii. der Verfahrensschritt (c) in einem Temperaturbereich von 20 °C bis 80 °C durchgeführt wird.

50. Verfahren nach Anspruch 46, wobei
i. das geeignete Lösungsmittel in dem Verfahrensschritt (d) ausgewählt ist aus der Gruppe bestehend aus Wasser, Acetonitril, Dioxan, Essigsäure, Schwefelsäure, Methylalkohol, Ethylalkohol, Tetrahydrofuran, Isopropylalkohol und tert-Butylalkohol;
ii. das geeignete Reagenz in dem Verfahrensschritt (d) eine Säure ausgewählt aus der Gruppe bestehend aus Schwefelsäure, Salzsäure und Essigsäure ist; und
iii. der Verfahrensschritt (d) in einem Temperaturbereich von 50 °C bis 120 °C durchgeführt wird.

51. Verfahren nach Anspruch 36, wobei das Verfahren des Weiteren den Schritt umfasst:
Umsetzen der Verbindung der Formel II-1, gegebenenfalls nach Konvertieren in eine Verbindung der Formel X-1, mit einer Verbindung der Formel IX-1, um die Verbindung der Formel I-1 zu erhalten, 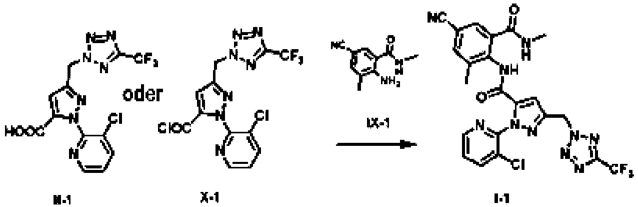 oder
Umsetzen der Verbindung der Formel II-1, gegebenenfalls nach Konvertieren in eine Verbindung der Formel X-1, mit einer Verbindung der Formel XI, um eine Verbindung der Formel XII zu erhalten, und Umsetzen der Verbindung der Formel XII mit Amin XIII-1, um die Verbindung der Formel I-1 zu erhalten, 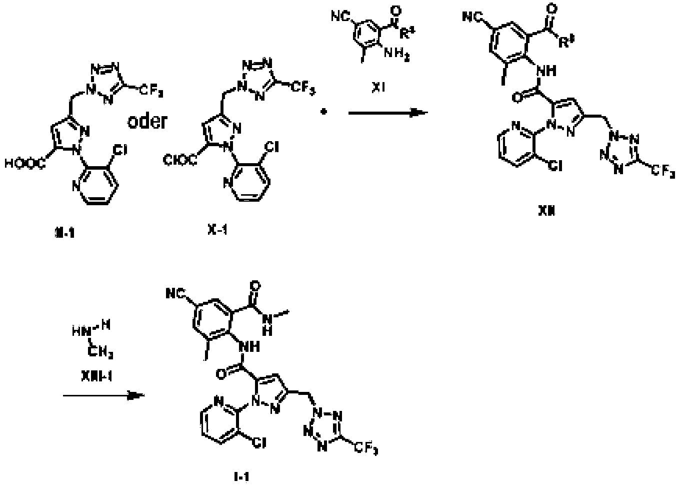
wobei R⁸ Hydroxy oder Cl ist.

52. Verfahren nach Anspruch 36, wobei das Verfahren ferner den Schritt umfasst:
Umsetzen der Verbindung der Formel II-1, gegebenenfalls nach Konvertieren in eine Verbindung der Formel X-1, mit einer Verbindung der Formel IX-1, um die Verbindung der Formel I-1 zu erhalten, 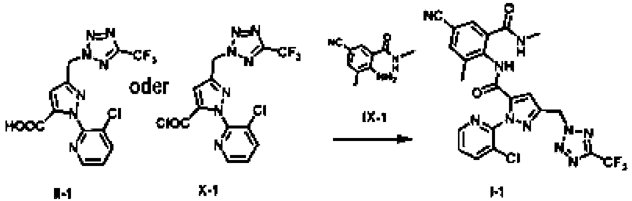 oder
Umsetzen der Verbindung der Formel II-1, gegebenenfalls nach Konvertieren in eine Verbindung der Formel X-1, mit einer Verbindung der Formel XI, um eine Verbindung der Formel XII zu erhalten, und Umsetzen der Verbindung der Formel XII mit Amin XIII-1, um die Verbindung der Formel I-1 zu erhalten, 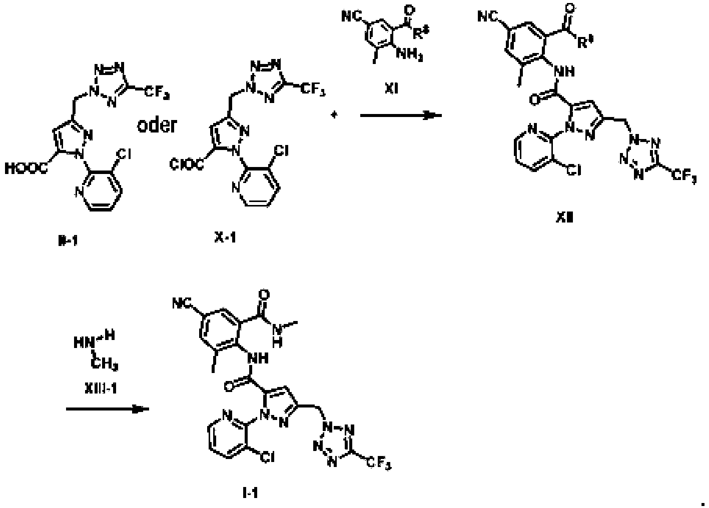
wobei R⁸ Hydroxy oder Cl ist.

## Revendications

1. Procédé de préparation d'un composé de formule II 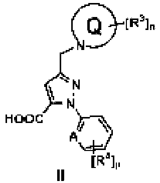 où,
A est N ou C ;
R³ et R⁴ sont choisis indépendamment dans le groupe constitué par hydrogène, halogène, cyano, nitro, hydroxy, alkyle en C₁-C₆, haloalkyle en C₁-C₆, cycloalkyle en C₃-C₆, halocycloalkyle en C₃-C₆, alcoxy en C₁-C₆, haloalcoxy en C₁-C₆, alkylthio en C₁-C₆, alkylsulfinyle en C₁-C₆, alkylsulfonyle en C₁-C₆, haloalkylthio en C₁-C₆, haloalkylsulfinyle en C₁-C₆, haloalkylsulfonyle en C₁-C₆ et NR^{a}R^{b} ; R^{a} et R^{b} sont choisis indépendamment dans le groupe constitué par hydrogène, alkyle en C₁-C₆, cycloalkyle en C₃-C₆ ; ou R^{a} et R^{b} forment ensemble avec l'atome N auquel ils sont attachés un cycle hétérocyclique substitué ou non substitué de 3 à 6 chaînons,
où les substituants sur le cycle hétérocyclique de 3 à 6 chaînons sont choisis dans le groupe constitué par halogène, cyano, nitro, hydroxy, alkyle en C₁-C₆, haloalkyle en C₁-C₆, cycloalkyle en C₃-C₆, halocycloalkyle en C₃-C₆, alcoxy en C₁-C₆, haloalcoxy en C₁-C₆, alkylthio en C₁-C₆, alkylsulfinyle en C₁-C₆, alkylsulfonyle en C₁-C₆, haloalkylthio en C₁-C₆, haloalkylsulfinyle en C₁-C₆ et haloalkylsulfonyle en C₁-C₆ ;
Q est un cycle hétérocyclique à 3, 4 ou 5 chaînons ;
n est un nombre entier de 0 à 4 ; et
p est un nombre entier de 0 à 5 ;
ledit procédé comprenant les étapes suivantes :
a. la mise en réaction d'un composé de formule IV avec un composé de formule VII en présence d'un ou plusieurs solvants appropriés et, éventuellement, d'un ou plusieurs catalyseurs et/ou réactifs appropriés pour obtenir un composé de formule III, 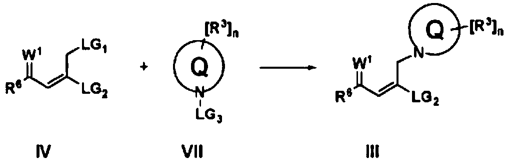 où
R⁶ est choisi dans le groupe constitué par CX₃, COW²(R⁷), C(W⁴R⁷)₃, CH(W⁴R⁷)₂, un groupe allylique, un furanyle substitué ou non substitué, 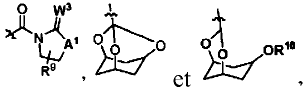 où la substitution sur le groupe furanyle est choisie dans le groupe constitué par halogène, cyano, nitro, hydroxy, alkyle en C₁-C₆, haloalkyle en C₁-C₆, cycloalkyle en C₃-C₆, halocycloalkyle en C₃-C₆, alcoxy en C₁-C₆, haloalcoxy en C₁-C₆, alkylthio en C₁-C₆, alkylsulfinyle en C₁-C₆, alkylsulfonyle en C₁-C₆, haloalkylthio en C₁-C₆, haloalkylsulfinyle en C₁-C₆ ou haloalkylsulfonyle en C₁-C₆ ;
W¹, W², W³, W⁴ et A¹ sont indépendamment O, S ou NR^{1c}; où R^{1c} est hydrogène, alkyle en C₁-C₆ ou cycloalkyle en C₃-C₆ ;
R⁷ est choisi dans le groupe constitué par hydrogène, alkyle en C₁-C₆ substitué ou non substitué, cycloalkyle en C₃-C₆ substitué ou non substitué, aryle substitué ou non substitué et arylalkyle substitué ou non substitué, ou
deux R⁷ forment ensemble avec l'atome auquel ils sont attachés un cycle carbocyclique ou hétérocyclique substitué ou non substitué de 3 à 6 chaînons,
où, la substitution sur alkyle en C₁-C₆, cycloalkyle en C₃-C₆, aryle et arylalkyle de R⁷ et du cycle carbocyclique ou hétérocyclique formé par deux R⁷ sont choisies indépendamment dans le groupe constitué par halogène, cyano, nitro, hydroxy, alkyle en C₁-C₆, haloalkyle en C₁-C₆, cycloalkyle en C₃-C₆, halocycloalkyle en C₃-C₆, alcoxy en C₁-C₆, haloalcoxy en C₁-C₆, alkylthio en C₁-C₆, alkylsulfinyle en C₁-C₆, alkylsulfonyle en C₁-C₆, haloalkylthio en C₁-C₆, haloalkylsulfinyle en C₁-C₆ et haloalkylsulfonyle en C₁-C₆ ;
R⁹ et R¹⁰ sont choisis indépendamment dans le groupe constitué par hydrogène, halogène, cyano, alkyle en C₁-C₆ substitué ou non substitué, alcoxy en C₁-C₆ substitué ou non substitué, cycloalkyle en C₃-C₆ substitué ou non substitué, alkylthio en C₁-C₆ substitué ou non substitué, alkylsulfinyle en C₁-C₆ substitué ou non substitué, alkylsulfonyle en C₁-C₆ substitué ou non substitué, aryle substitué ou non substitué et arylalkyle substitué ou non substitué,
où, la substitution sur alkyle en C₁-C₆, alcoxy en C₁-C₆, cycloalkyle en C₃-C₆, alkylthio en C₁-C₆, alkylsulfinyle en C₁-C₆, alkylsulfonyle en C₁-C₆, aryle et arylalkyle de R⁹ et R¹⁰ est choisie dans le groupe constitué par halogène, cyano, nitro, hydroxy, alkyle en C₁-C₆, haloalkyle en C₁-C₆, cycloalkyle en C₃-C₆, halocycloalkyle en C₃-C₆, alcoxy en C₁-C₆, haloalcoxy en C₁-C₆, alkylthio en C₁-C₆, alkylsulfinyle en C₁-C₆, alkylsulfonyle en C₁-C₆, haloalkylthio en C₁-C₆, haloalkylsulfinyle en C₁-C₆ et haloalkylsulfonyle en C₁-C₆ ;
LG₁ est choisi dans le groupe constitué par X, OR⁵ et OSi(R¹¹)₃,
où R⁵ est choisi dans le groupe constitué par hydrogène, alkyle en C₁-C₆ substitué ou non substitué, aryl-alkyle en C₁-C₆ substitué ou non substitué, aryle substitué ou non substitué, -(C=O)-alkyle en C₁-C₆, - (C=O)-haloalkyle en C₁-C₆, -(C=O)O-alkyle en C₁-C₆, -(C=O)O-haloalkyle en C₁-C₆, SO₂-alkyle en C₁-C₆, SO₂-haloalkyle en C₁-C₆ et SO₂-aryle substitué ou non substitué,
R¹¹ est choisi dans le groupe constitué par hydrogène, halogène, alkyle en C₁-C₆ substitué ou non substitué, aryle-alkyle en C₁-C₆ substitué ou non substitué, aryle substitué ou non substitué, -(C=O)-alkyle en C₁-C₆, -(C=O)-haloalkyle en C₁-C₆, -(C=O)O-alkyle en C₁-C₆, -(C=O)O-haloalkyle en C₁-C₆, SO₂-alkyle en C₁-C₆, SO₂-haloalkyle en C₁-C₆ et SO₂-aryle substitué ou non substitué ;
LG₂ est X, OR¹²,
où R¹² est choisi dans le groupe constitué par hydrogène, alkyle en C₁ -C₆ substitué ou non substitué, aryl-alkyle en C₁-C₆ substitué ou non substitué, aryle substitué ou non substitué, -(C=O)-alkyle en C₁-C₆, - (C=O)-haloalkyle en C₁-C₆, -(C=O)O-alkyle en C₁-C₆, -(C=O)O-haloalkyle en C₁-C₆, SO₂-alkyle en C₁-C₆, SO₂-haloalkyle en C₁-C₆, SO₂-aryle substitué ou non substitué, alkylthio et NR^{a}R^{b} ; R^{a} et R^{b} sont choisis indépendamment dans le groupe constitué par hydrogène, alkyle en C₁-C₆, cycloalkyle en C₃-C₆ ; ou R^{a} et R^{b} forment ensemble avec l'atome N auquel ils sont attachés un cycle hétérocyclique substitué ou non substitué de 3 à 6 chaînons,
où, la substitution sur alkyle en C₁-C₆, aryl-alkyle en C₁-C₆, aryle et SO₂-aryle des groupes LG₁ et LG₂ est choisie dans le groupe constitué par halogène, cyano, nitro, hydroxy, alkyle en C₁-C₆, haloalkyle en C₁-C₆, cycloalkyle en C₃-C₆, halocycloalkyle en C₃-C₆, alcoxy en C₁-C₆, haloalcoxy en C₁-C₆, alkylthio en C₁-C₆, alkylsulfinyle en C₁-C₆, alkylsulfonyle en C₁-C₆, haloalkylthio en C₁-C₆, haloalkylsulfinyle en C₁-C₆ et haloalkylsulfonyle en C₁-C₆ ;
LG₃ est choisi dans le groupe constitué par hydrogène, métal alcalin, halogène et Si(R¹¹)₃ ;
chaque X est indépendamment hydrogène, F, Cl, Br ou I ;
R³, n et Q sont chacun tels que définis ci-dessus ;
b. la cyclisation du composé de formule III avec une hydrazine de formule VIII dans un ou plusieurs solvants appropriés et, éventuellement, un ou plusieurs réactifs appropriés pour obtenir un composé de formule IIA, 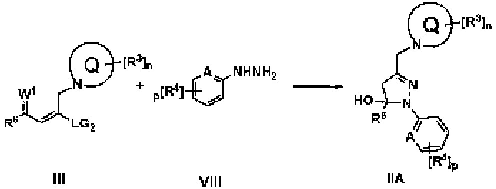 où R³, R⁴, R⁶, A, n, p, Q, W¹ et LG₂ sont chacun tels que définis ci-dessus ;
c. l'élimination d'eau du composé de formule IIA en utilisant un ou plusieurs réactifs déshydratants appropriés dans un ou plusieurs solvants appropriés pour obtenir un composé de formule IIB, 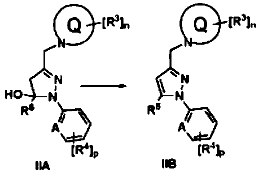 où R³, R⁴, R⁶, A, n, p et Q sont chacun tels que définis ci-dessus ; et
d. la conversion du composé de formule IIB en le composé de formule II en utilisant un ou plusieurs réactifs appropriés dans un ou plusieurs solvants appropriés et, éventuellement, un ou plusieurs catalyseurs appropriés, 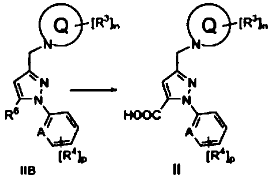
où R³, R⁴, R⁶, A, n, p et Q sont chacun tels que définis ci-dessus ; et
où le composé de formule III obtenu à l'étape (a), le composé de formule IIA obtenu à l'étape (b) et le composé de formule IIB obtenu à l'étape (c) peuvent être isolés ou non.

2. Procédé selon la revendication 1, où
i. le solvant approprié à l'étape de procédé (a) est choisi dans le groupe constitué par l'acétone, l'acétonitrile, l'éther méthyl-tert-butylique, le chlorobenzène, le dichloroéthane, le dichlorométhane, le dioxane, l'acétate d'éthyle, le diméthylformamide, le diméthylacétamide, le diméthylsulfoxyde, le 2-méthyltétrahydrofurane, le tétrahydrofurane, de 1,2-diméthoxyéther, le toluène, le p-xylène et la N-méthyl-2-pyrrolidone ;
ii. le catalyseur approprié à l'étape de procédé (a) est choisi dans le groupe constitué par l'iodure de potassium, l'iodure de sodium, l'iodure de cuivre et l'iodure cuivrique, et
iii. l'étape de procédé (a) est réalisée dans une plage de températures allant de 20 °C à 150 °C.

3. Procédé selon la revendication 1, où
i. le solvant approprié à l'étape de procédé (b) est choisi dans le groupe constitué par l'acétone, l'acétonitrile, l'alcool éthylique, l'acide acétique, l'éther méthyl-tert-butylique, le chlorobenzène, le dichloroéthane, le dichlorométhane, le dioxane, le diméthylformamide, le diméthylacétamide, le diméthylsulfoxyde, l'acétate d'éthyle, le 2-méthyltétrahydrofurane, le tétrahydrofurane, le 1,2-diméthoxyéther, le toluène, le xylène et la N-méthyl-2-pyrrolidone ;
ii. le réactif approprié à l'étape de procédé (b) est choisi dans le groupe constitué par l'acide acétique, l'acide trifluoroacétique, l'acide chlorhydrique, l'acide bromhydrique, l'acide sulfurique, l'acide méthanesulfonique, l'acide triflique, l'acide phosphorique et l'acide p-toluènesulfonique ; et
iii. l'étape de procédé (b) est réalisée dans une plage de températures allant de 20 °C à 150 °C.

4. Procédé selon la revendication 1, où
i. le solvant approprié à l'étape de procédé (c) est choisi dans le groupe constitué par l'acétonitrile, l'éther méthyl-tert-butylique, le dichlorométhane, le dioxane, le chlorure de thionyle, l'acide acétique, l'alcool méthylique, l'alcool éthylique, le tétrahydrofurane, l'alcool isopropylique et l'alcool tert-butylique ;
ii. le réactif déshydratant approprié à l'étape de procédé (c) est choisi dans le groupe constitué par l'acide sulfurique, l'acide trifluoroacétique, le trichlorure de phosphore, l'oxychlorure de phosphore, le chlorure de thionyle, l'anhydride acétique, l'anhydride trifluoroacétique, le chlorure d'oxalyle, le phosgène, le diphosgène, l'acide chlorhydrique méthanolique, le gaz chlorhydrique, l'acide acétique, le bromure d'hydrogène, l'acide triflique, l'acide méthanesulfonique, l'acide p-toluènesulfonique, le chlorure d'hydrogène-1,4-dioxane et le gel de silice ; et
iii. l'étape de procédé (c) est réalisée dans une plage de températures allant de 20 °C à 150 °C.

5. Procédé selon la revendication 1, où
i. le solvant approprié à l'étape de procédé (d) est choisi dans le groupe constitué par l'eau, l'acétonitrile, le dioxane, l'acide sulfurique, l'acide acétique, l'alcool méthylique, l'alcool éthylique, le tétrahydrofurane, l'alcool isopropylique et l'alcool tert-butylique ;
ii. le réactif approprié à l'étape de procédé (d) est un acide choisi dans le groupe constitué par l'acide sulfurique, l'acide chlorosulfurique, l'acide chlorhydrique, l'acide fluorhydrique, l'acide hydroborique, l'acide phosphorique, l'acide acétique, l'acide trifluoroacétique, l'acide p-toluènesulfonique, l'acide méthanesulfonique et l'acide trifluorométhanesulfonique ; ou
iii. le réactif approprié à l'étape de procédé (d) est une base choisie dans le groupe constitué par les trialkylamines, la pyridine, les alkylpyridines, les phosphazines, le 1,8-diazabicyclo[5.4.0]undécène (DBU), l'hydroxyde de lithium, l'hydroxyde de sodium, l'hydroxyde de potassium, le carbonate de sodium, le carbonate de potassium, l'acétate de sodium, l'acétate de potassium, l'acétate de lithium, le méthoxyde de sodium, l'éthoxyde de sodium, le tert-butoxyde de sodium et le tert-butoxyde de potassium ;
iv. le catalyseur utilisé à l'étape de procédé (d) est choisi dans le groupe constitué par le chlorure ferrique (FeCl₃), le chlorure d'aluminium (AlCl₃), le trifluorure de bore (BF₃), le trichlorure d'antimoine (SbCl₃) et le phosphate monosodique (NaH₂PO₄) ; et
v. l'étape de procédé (d) est réalisée dans une plage de températures allant de 20 °C à 150 °C.

6. Procédé selon la revendication 1, où le composé de formule IIB est obtenu en variante par le procédé comprenant les étapes suivantes :
i. la cyclisation d'un composé de formule XVII avec l'hydrazine de formule VIII dans un ou plusieurs solvants appropriés et, éventuellement, un ou plusieurs réactifs appropriés pour obtenir un composé de formule XVI, 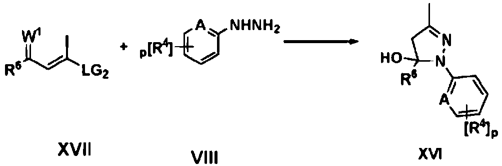
où R⁶ est CX₃, X est F, Cl, Br et I ; R⁴, A, p, W¹ et LG₂ sont chacun tels que définis dans la revendication 1 ;
ou
la cyclisation du composé de formule IV et de l'hydrazine de formule VIII dans un ou plusieurs solvants appropriés et, éventuellement, un ou plusieurs réactifs appropriés pour obtenir un composé de formule XVIII, 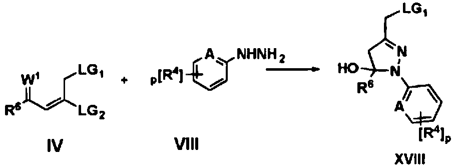
où R⁶ est CX₃, LG₁ est Cl ; X est F, Cl, Br et I, de préférence Cl ; R⁴, A, p, W¹ et LG₂ sont chacun tels que définis dans la revendication 1 ;
ii. l'élimination d'eau du composé de formule XVI en utilisant un ou plusieurs réactifs déshydratants appropriés dans un ou plusieurs solvants appropriés pour obtenir un composé de formule XV ; 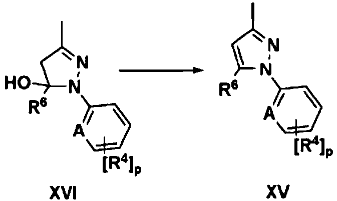
où R⁶ est CX₃, X est F, Cl, Br et I ; R⁴, A et p sont chacun tels que définis dans la revendication 1 ;
ou
l'élimination d'eau du composé de formule XVIII en utilisant un ou plusieurs réactifs déshydratants appropriés dans un ou plusieurs solvants appropriés pour obtenir un composé de formule XIV, 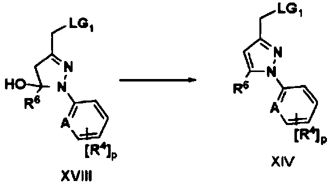
où R⁶ est CX₃, LG₁ est Cl, X est F, Cl, Br et I ; R₄, A et p sont chacun tels que définis dans la revendication 1 ;
iii. l'halogénation du composé de formule XV en utilisant un agent d'halogénation approprié dans un ou plusieurs solvants appropriés et, éventuellement, un ou plusieurs initiateurs radicalaires pour obtenir un composé de formule XIV, 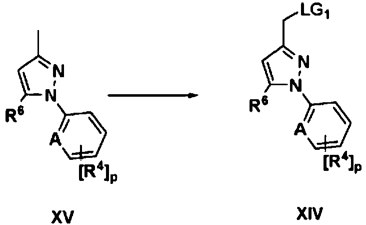 où R⁶ est CX₃, LG₁ est X, X est F, Cl, Br et I ; R⁴, A et p sont chacun tels que définis dans la revendication 1 ; et
iv. l'obtention du composé de formule IIB par mise en réaction du composé de formule XIV avec le composé de formule VII, 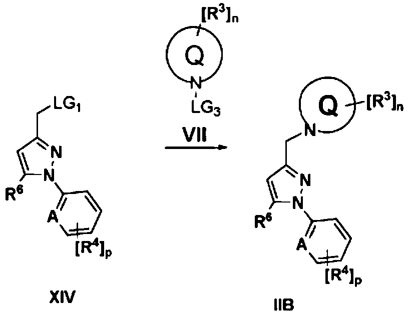
où R⁶ est CX₃, LG₁ est X, X est F, Cl, Br et I ; R³, R⁴, A, Q, n, p et LG₃ sont chacun tels que définis dans la revendication 1 ; et
les composés de formule XVI et XVIII obtenus à l'étape (i), les composés de formule XV et XIV obtenus à l'étape (ii) et le composé XIV obtenu à l'étape (iii) peuvent être isolés ou non.

7. Procédé selon la revendication 6, où
i. le solvant approprié à l'étape (i) est choisi dans le groupe constitué par l'acétone, l'acétonitrile, l'alcool éthylique, l'éther méthyl-tert-butylique, le chlorobenzène, le dichloroéthane, le dichlorométhane, le dioxane, le diméthylformamide, le diméthylacétamide, le diméthylsulfoxyde, l'acétate d'éthyle, le 2-méthyltétrahydrofurane, le tétrahydrofurane, le 1,2-diméthoxyéther, le toluène, le xylène et la N-méthyl-2-pyrrolidone ;
ii. le réactif approprié à l'étape (i) est choisi dans le groupe constitué par l'acide acétique, l'acide trifluoroacétique, l'acide chlorhydrique, l'acide bromhydrique, l'acide sulfurique, l'acide méthanesulfonique, l'acide triflique, l'acide phosphorique et l'acide p-toluènesulfonique ; et
iii. l'étape (i) est réalisée dans une plage de températures allant de 20 °C à 150 °C.

8. Procédé selon la revendication 6, où
i. le solvant approprié à l'étape (ii) est choisi dans le groupe constitué par l'eau, l'acétonitrile, l'éther méthyl-tert-butylique, le dichlorométhane, le dioxane, le chlorure de thionyle, l'acide acétique, l'alcool méthylique, l'alcool éthylique, le tétrahydrofurane, l'alcool isopropylique et l'alcool tert-butylique ;
ii. le réactif déshydratant approprié à l'étape (ii) est choisi dans le groupe constitué par l'acide sulfurique, l'acide trifluoroacétique, le trichlorure de phosphore, l'oxychlorure de phosphore, le chlorure de thionyle, l'anhydride acétique, l'anhydride trifluoroacétique, le chlorure d'oxalyle, le phosgène, le diphosgène, l'acide chlorhydrique méthanolique, le gaz chlorhydrique, l'acide acétique, le bromure d'hydrogène, l'acide triflique, l'acide méthanesulfonique, l'acide p-toluènesulfonique et le gel de silice ; et
iii. l'étape (ii) est réalisée dans une plage de températures allant de 20 °C à 150 °C.

9. Procédé selon la revendication 6, où
i. le solvant approprié à l'étape (iii) est choisi dans le groupe constitué par l'acétone, l'acétonitrile, l'éther méthyl-tert-butylique, le chlorobenzène, le dichloroéthane, le dichlorométhane, le dioxane, l'acétate d'éthyle, le diméthylformamide, le diméthylacétamide, le diméthylsulfoxyde, le 2-méthyltétrahydrofurane, le tétrahydrofurane, le 1,2-diméthoxyéther, le toluène, le p-xylène et la N-méthyl-2-pyrrolidone ;
ii. l'agent d'halogénation à l'étape (iii) est choisi dans le groupe constitué par un N-halosuccinimide : le N-chlorosuccinimide, le N-bromosuccinimide et le N-iodosuccinimide ; X₂ : Cl₂, Br₂ ou I₂ ; X₂/hv : Cl₂/hv, Br₂/hv ou I₂/hv ; une N-halosaccharine : la N-chlorosaccharine, la N-bromosaccharine et la N-iodosaccharine ; et une halohydantoïne : la N-chlorohydantoïne, la N-bromohydantoïne et la N-iodohydantoïne ;
iii. l'initiateur radicalaire à l'étape (iii) est choisi dans le groupe constitué par le peroxyde de dibenzoyle, le peroxyde d'hydrogène, le peroxydicarbonate de di(n-propyle), le peroxybenzoate de t-butyle, le peroxyde de méthyléthylcétone, le 2,5-diméthyl-2,5-di(t-butylperoxy)-3-hexyne, le peroxyde de di(t-butyle), le peroxyde d'acétone, le peroxyde de dicumyle, l'azobisisobutyronitrile, le peroxydicarbonate de bis(2-éthylhexyle), le (peroxybis(propane-2,2-diyl))dibenzène, l'acide peracétique, l'acide métachloroperbenzoïque, le réactif de Payne, le monoperphtalate de magnésium, l'acide trifluoroperacétique, l'acide trichloroperacétique, l'acide 2,4-dinitroperbenzoïque, l'acide de Caro et le caroate de potassium ; et
iv. l'étape (iii) est réalisée dans une plage de températures allant de 20 °C à 150 °C.

10. Procédé selon la revendication 6, où
i. le solvant approprié à l'étape (iv) est choisi dans le groupe constitué par l'acétone, l'acétonitrile, l'éther méthyl-tert-butylique, le chlorobenzène, le dichloroéthane, le dichlorométhane, le dioxane, l'acétate d'éthyle, le diméthylformamide, le diméthylacétamide, le diméthylsulfoxyde, le 2-méthyltétrahydrofurane, le tétrahydrofurane, le 1,2-diméthoxyéther, le toluène, le p-xylène et la N-méthyl-2-pyrrolidone ;
ii. le catalyseur à l'étape (iv) est choisi dans le groupe constitué par l'iodure de potassium, l'iodure de sodium, l'iodure de cuivre et l'iodure cuivrique ; et
iii. l'étape (iv) est réalisée dans une plage de températures allant de 20 °C à 150 °C.

11. Procédé selon la revendication 1, où le composé de formule IV est obtenu par les étapes de procédé suivantes :
i. la conversion d'un composé de formule IV-A en un composé de formule IV-B en utilisant un réactif approprié dans un ou plusieurs solvants appropriés, 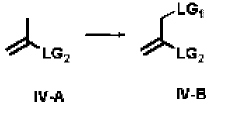 où LG₁ et LG₂ sont chacun tels que définis dans la revendication 1 ;
ii. la mise en réaction du composé de formule IV-B avec un composé de formule IV-C dans un solvant approprié et, éventuellement, en utilisant un réactif approprié pour obtenir le composé de formule IV, 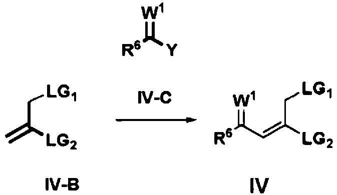
où Y est OR⁵, X ou -O(C=O)CX₃ ; X est F, Cl, Br ou I ; R⁵, R⁶, W¹, LG₁ et LG₂ sont chacun tels que définis dans la revendication 1,
et
où le composé de formule IV-B peut être isolé ou non.

12. Procédé selon la revendication 11, où
i. le réactif approprié à l'étape (i) est choisi dans le groupe constitué par HX, NaX, KX, CuX₂, MgX₂, CsX, ZnX₂, SOCl₂, SO₂Cl₂, COCl₂, X₂, C(=O)(OCl₃)₂, t-BuOCl, NaOCl, la chloramine-T, les *N-*halosuccinimides, la N-halosaccharine, l'halohydantoïne, POX₃, PX₃ et PX₅ ; où X ou halo est Cl, Br, I ou F ;
ii. le solvant approprié à l'étape (i) est choisi dans le groupe constitué par l'acétone, l'acétonitrile, l'éther méthyl-tert-butylique, le chlorobenzène, le dichloroéthane, le dichlorométhane, le dioxane, l'acétate d'éthyle, le diméthylformamide, le diméthylacétamide, le diméthylsulfoxyde, le 2-méthyltétrahydrofurane, le tétrahydrofurane, le 1,2-diméthoxyéther, le toluène, le p-xylène et la N-méthyl-2-pyrrolidone ; et
iii. l'étape (i) est réalisée dans une plage de températures allant de 20 °C à 100 °C.

13. Procédé selon la revendication 11, où
i. le réactif approprié à l'étape (ii) est choisi dans le groupe constitué par la pyridine, la triéthylamine, la N,N-diisopropyléthylamine, la 2,6-di-tert-butylpyridine, le 1,5-diazabicyclo(4.3.0)non-5-ène, le 1,8-diazabicycloundéc-7-ène, le diisopropylamide de lithium, le bis(triméthylsilyl)amide de sodium et le bis(triméthylsilyl)amide de potassium ;
ii. le solvant approprié à l'étape (ii) est choisi dans le groupe constitué par l'acétone, l'acétonitrile, l'éther méthyl-tert-butylique, le chlorobenzène, le dichloroéthane, le dichlorométhane, le dioxane, l'acétate d'éthyle, le diméthylformamide, le diméthylacétamide, le diméthylsulfoxyde, le 2-méthyltétrahydrofurane, le tétrahydrofurane, le 1,2-diméthoxyéther, le toluène, le p-xylène et la N-méthyl-2-pyrrolidone ; et
iii. l'étape (ii) est réalisée dans une plage de températures allant de 20 °C à 100 °C.

14. Procédé selon la revendication 1, où ledit procédé comprend en outre l'étape suivante :
la mise en réaction du composé de formule II, éventuellement après conversion en un composé de formule X, avec un composé de formule IX pour obtenir le composé de formule I, 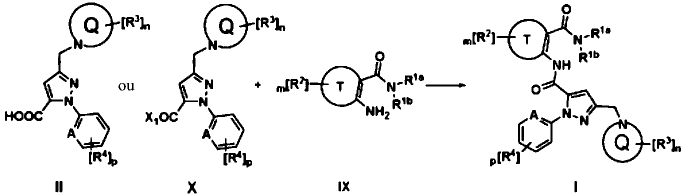 où
X₁ est Cl ou Br ;
R^{1a} et R^{1b} sont choisis indépendamment dans le groupe constitué par hydrogène, alkyle en C₁-C₆, haloalkyle en C₁-C₆, cycloalkyle en C₃-C₆, (alkyle en C₁-C₆)-cycloalkyle en C₃-C₆ et (cycloalkyle en C₃-C₆)-alkyle en C₁-C₆ ; ou
R^{1a} et R^{1b} forment ensemble avec l'atome N auquel ils sont attachés N=S(=O)₀₋₂(alkyle en C₁-C₆)₂ ;
T est un cycle aryle ou hétéroaryle ou un cycle ou système cyclique aryle ou hétéroaryle fusionné ou bicyclique ;
R² est choisi dans le groupe constitué par hydrogène, halogène, cyano, nitro, alkyle en C₁-C₆, haloalkyle en C₁-C₆ et cycloalkyle en C₃-C₆ ;
m est un nombre entier de 0 à 6 ; et
R³, R⁴, A, n, p et Q sont chacun tels que définis dans la revendication 1 ;
ou
la mise en réaction du composé de formule II, éventuellement après conversion en un composé de formule X, avec un composé de formule XI pour obtenir un composé de formule XII, et la mise en réaction du composé de formule XII avec une amine XIII appropriée pour obtenir le composé de formule I, 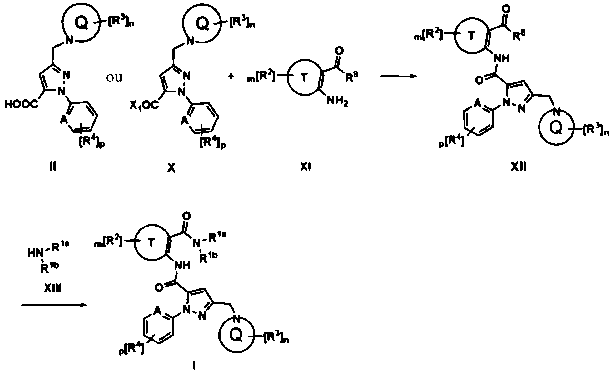 où
X₁ est Cl ou Br ;
R^{1a} et R^{1b} sont choisis indépendamment dans le groupe constitué par hydrogène, alkyle en C₁-C₆, haloalkyle en C₁-C₆, cycloalkyle en C₃-C₆, (alkyle en C₁-C₆)-cycloalkyle en C₃-C₆ et (cycloalkyle en C₃-C₆)-alkyle en C₁-C₆ ; ou
R^{1a} et R^{1b} forment ensemble avec l'atome N auquel ils sont attachés N=S(=O)₀₋₂(alkyle en C₁-C₆)₂ ;
T est un cycle aryle ou hétéroaryle ou un cycle ou système cyclique aryle ou hétéroaryle fusionné ou bicyclique ;
R² est choisi dans le groupe constitué par hydrogène, halogène, cyano, nitro, alkyle en C₁-C₆, haloalkyle en C₁-C₆ et cycloalkyle en C₃-C₆ ;
R⁸ est choisi dans le groupe constitué par hydroxy, Cl et OR⁷ ;
m est un nombre entier de 0 à 6 ; et
R³, R⁴, R⁷, A, n, p et Q sont chacun tels que définis dans la revendication 1.

15. Procédé selon les revendications 1 et 14, où
R^{1a} est hydrogène ; R^{1b} est choisi dans le groupe constitué par hydrogène, alkyle en C₁-C₆ et (cycloalkyle en C₃-C₆)-alkyle en C₁-C₆ ;
R² est choisi dans le groupe constitué par halogène, cyano et alkyle en C₁-C₆ ; R³ est haloalkyle en C₁-C₆ ;
R⁴ est X ;
R⁶ est CX₃ ou C(=O)W²R⁷,
où R⁷ est choisi dans le groupe constitué par hydrogène, les groupes alkyle en C₁-C₆, cycloalkyle en C₃-C₆, aryle et arylalkyle ;
W¹ et W² sont O ;
LG₁ est X ;
LG₂ est X ou OR¹² ; R¹² est alkyle en C₁-C₆ ;
LG₃ est hydrogène ou métal alcalin ;
A est N ;
Q est un cycle hétérocyclique à 3, 4 ou 5 chaînons ;
T est un cycle phényle ;
Y est X ;
n est un nombre entier égal à 1 ;
m est un nombre entier égal à 2 ;
p est un nombre entier égal à 1 ou 2 ; et
chaque X est indépendamment F, Cl, Br ou I.

16. Procédé selon les revendications 1 et 14, où
R^{1a} est hydrogène ; R^{1b} est alkyle en C₁-C₆ ;
R² est choisi dans le groupe constitué par Cl, cyano et méthyle ;
R³ est trifluorométhyle ;
R⁴ est Cl ;
R⁶ est CCl₃, CBr₃, C(=O)W²CH₃ ou C(=O)W²C₂H₅,
W¹ et W² sont O ;
LG₁ est Cl, Br ou I ;
LG₂ est Cl, Br, I, OCH₃ ou OC₂H₅ ;
LG₃ est métal alcalin ;
A est N ;
Q est un cycle tétrazole ;
T est un cycle phényle ;
Y est Cl ;
n est un nombre entier égal à 1 ;
m est un nombre entier égal à 2 ; et
p est un nombre entier égal à 1.

17. Procédé selon la revendication 6, où
R³ est haloalkyle en C₁-C₆ ;
R⁴ est X ;
R⁶ est CX₃ ou C(=O)W²R⁷,
où R⁷ est choisi dans le groupe constitué par hydrogène, alkyle en C₁-C₆, cycloalkyle en C₃-C₆, aryle et arylalkyle ;
W¹ et W² sont O ;
LG₁ est X ;
LG₂ est X ou OR¹² ; R¹² est alkyle en C₁-C₆ ;
LG₃ est hydrogène ou métal alcalin ;
A est N ;
Q est un cycle hétérocyclique à 3, 4 ou 5 chaînons ;
n est un nombre entier égal à 1 ;
p est un nombre entier égal à 1 ou 2 ; et
chaque X est indépendamment F, Cl, Br ou I.

18. Procédé selon la revendication 6, où
R³ est trifluorométhyle ;
R⁴ est Cl ;
R⁶ est CCl₃, CBr₃, C(=O)W²CH₃ ou C(=O)W²C₂H₅,
W¹ et W² sont O ;
LG₁ est Cl, Br ou I ;
LG₂ est Cl, Br, I, OCH₃ ou OC₂H₅ ;
LG₃ est métal alcalin ;
A est N ;
Q est un cycle tétrazole ;
n est un nombre entier égal à 1 ; et
p est un nombre entier égal à 1.

19. Composé de formule XIX, 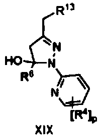
où R¹³ est 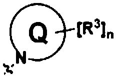 ou LGᵢ ; R³, R⁴, R⁶, n, p et Q sont chacun tels que définis dans la revendication 1,
à condition que lorsque R¹³ est LG₁, alors R⁶ est CX₃, où LG₁ est Cl, Br ou I ; et X est F, Cl, Br ou I.

20. Composé selon la revendication 19, où
R⁶ est CX₃ ou C(=O)W²R⁷,
où R⁷ est choisi dans le groupe constitué par hydrogène, alkyle en C₁-C₆, cycloalkyle en C₃-C₆, aryle et arylalkyle ;
W² est O ; R³ est haloalkyle en C₁-C₆ ; R⁴ est X ; n est un nombre entier égal à 1 ; Q est un cycle hétérocyclique à 5 chaînons ; A est N ; p est un nombre entier égal à 1 ou 2 ; et chaque X est indépendamment F, Cl, Br ou I.

21. Composé selon la revendication 19, où
R⁶ est CCl₃, CBr₃, C(=O)W²CH₃, C(=O)W²C₂H₅ ; W² est O ; R³ est trifluoroalkyle ; R⁴ est Cl ; n est un nombre entier égal à 1 ; Q est un cycle tétrazole ; A est N ; p est un nombre entier égal à 1 et LG₁ est Cl ou Br.

22. Composé selon la revendication 19, où le composé de formule XIX est choisi dans le groupe constitué par :

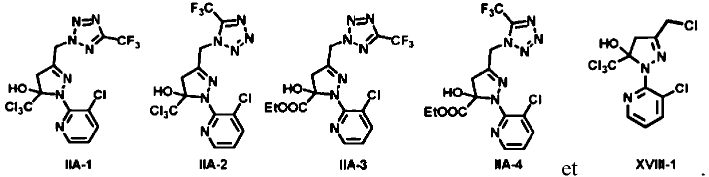

23. Composé de formule XX, 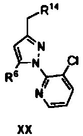
où R¹⁴ est 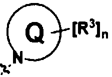 ou LG₁ ou hydrogène R⁶ est CX₃, C(W⁴R⁷)₃, CH(W⁴R⁷)₂, un groupe allylique, un furanyle substitué ou non
substitué, 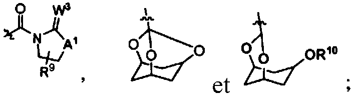 R³, R⁴, R⁷, R⁹, R¹⁰, LG₁, A, A¹, n, p, Q, W³, W⁴, X et les substituants sur furanyle sont chacun tels que définis dans la revendication 1,
à condition que lorsque R¹⁴ est LG₁ ou hydrogène, alors R⁶ est CX₃, où LG₁ et X sont Cl, Br ou I.

24. Composé selon la revendication 23, où
R⁶ est CX₃, R³ est haloalkyle en C₁-C₆ ; n est un nombre entier égal à 1 ; Q est un cycle hétérocyclique à 5 chaînons ; A est N ; R⁴ est X ; p est un nombre entier égal à 1 ou 2.

25. Composé selon la revendication 23, où
R⁶ est CCl₃ ou CBr₃ ; R³ est trifluoroalkyle ; n est un nombre entier égal à 1 ; Q est un cycle tétrazole ; A est N ; R⁴ est Cl ; p est un nombre entier égal à 1, et LG₁ est Cl ou Br.

26. Composé selon la revendication 23, où le composé de formule XX est choisi dans le groupe constitué par :

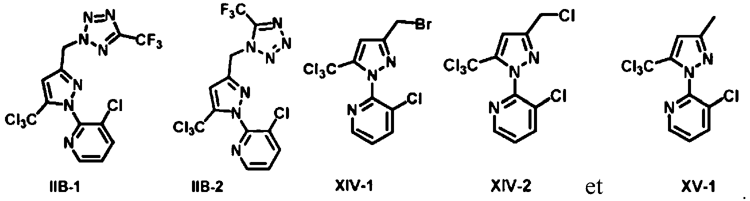

27. Composé de formule III, 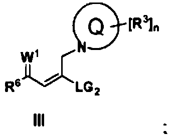 où Q est un cycle hétérocyclique à 3, 4 ou 5 chaînons à l'exclusion d'un cycle triazole ; R³, R⁶, n, W¹ et LG₂ sont chacun tels que définis dans la revendication 1.

28. Composés de formule III selon la revendication 27, où
R⁶ est CX₃ ou C(=O)W²R⁷,
où R⁷ est choisi dans le groupe constitué par hydrogène, alkyle en C₁-C₆, cycloalkyle en C₃-C₆, aryle et arylalkyle ;
W¹ et W² sont O ; LG₂ est X ou OR¹²; R¹² est alkyle en C₁-C₆ ; R³ est haloalkyle en C₁-C₆ ; n est un nombre entier égal à 1 ; Q est un cycle hétérocyclique à 5 chaînons à l'exclusion d'un cycle triazole ; et chaque X est indépendamment F, Cl, Br ou I.

29. Composés de formule III selon la revendication 27, où
R⁶ est CCl₃, CBr₃, C(=O)W²CH₃, C(=O)W²C₂H₅ ; W¹ et W² sont O ; LG₂ est Cl, Br, I, OCH₃ ou OC₂H₅ ; R³ est trifluoroalkyle ; n est un nombre entier égal à 1 ; et Q est un cycle tétrazole.

30. Composé selon la revendication 27, où le composé de formule III est choisi dans le groupe constitué par :

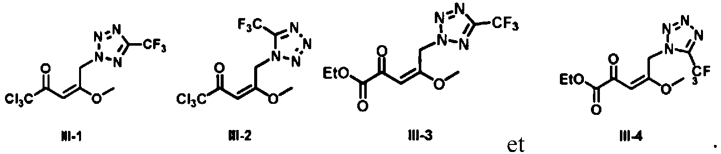

31. Composé de formule IV-2 :

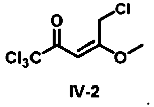

32. Composé de formule XVI, 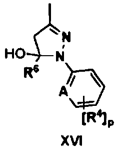
où R⁶ est CX₃, C(W⁴R⁷)₃, CH(W⁴R⁷)₂, un groupe allylique, un furanyle substitué ou
non substitué, 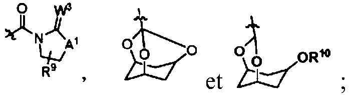 A est N ; et R⁴, R⁷, R⁹, R¹⁰, A¹, p, W³, W⁴ et X sont chacun tels que définis dans la revendication 1.

33. Composé de formule XVI tel que revendiqué dans la revendication 32, où
R⁶ est CX₃ ; A est N ; R⁴ est X ; p est un nombre entier égal à 1 ou 2 ; et chaque X est indépendamment F, Cl, Br ou I.

34. Composé de formule XVI tel que revendiqué dans la revendication 32, où
R⁶ est CCl₃ ou CBr₃ ; A est N ; R⁴ est Cl ; et p est un nombre entier égal à 1.

35. Composé selon la revendication 32, où le composé de formule XVI est XVI-1 :

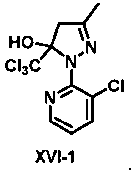

36. Procédé de préparation d'un composé de formule II-1 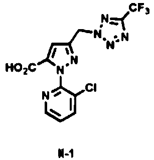 ledit procédé comprenant les étapes suivantes :
a. la mise en réaction d'un composé de formule IV-1 ou IV-2 avec un composé de formule VII-1 en présence d'un ou plusieurs solvants appropriés et, éventuellement, d'un ou plusieurs catalyseurs et/ou réactifs appropriés pour obtenir un mélange d'un composé de formule III-1 et d'un composé de formule III-2, 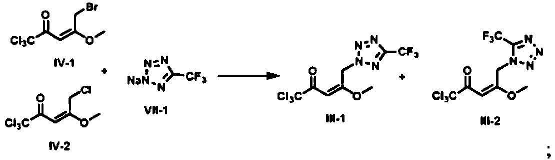
b. la cyclisation du composé de formule III-1, ou du composé de formule III-1 et du composé de formule III-2 dans le mélange, avec une hydrazine de formule VIII-1 dans un ou plusieurs solvants appropriés et, éventuellement, un ou plusieurs réactifs appropriés pour obtenir respectivement un composé de formule IIA-1 ou le mélange du composé de formule IIA-1 et d'un composé de formule IIA-2, 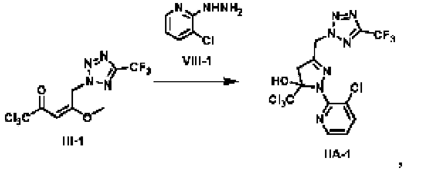 ou 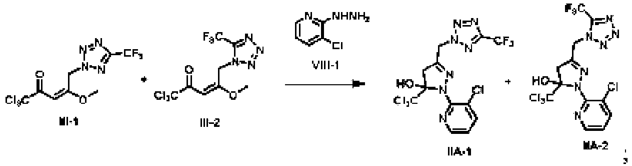
c. l'élimination d'eau du composé de formule IIA-1, ou du composé de formule IIA-1 et du composé de formule IIA-2 dans le mélange, en utilisant un ou plusieurs réactifs déshydratants appropriés dans un ou plusieurs solvants appropriés pour obtenir respectivement un composé de formule IIB-1 ou le mélange du composé de formule IIB-1 et d'un composé de formule IIB-2, 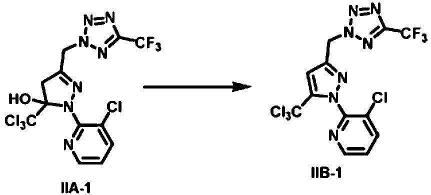 ou 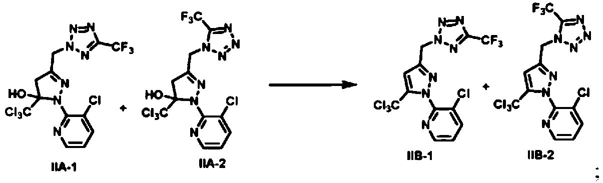 et
d. la conversion du composé de formule IIB-1, ou du composé de formule IIB-1 et du composé de formule IIB-2 dans le mélange, respectivement en le composé de formule II-1 ou en le mélange du composé de formule II-1 et d'un composé de formule II-2, en utilisant un ou plusieurs réactifs appropriés dans un ou plusieurs solvants appropriés et, éventuellement, un ou plusieurs catalyseurs appropriés, 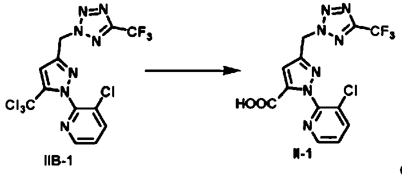 ou 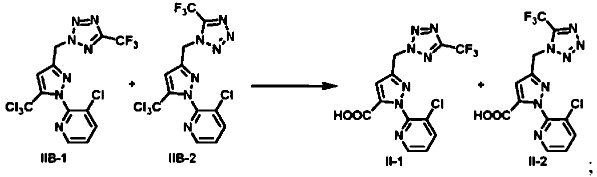
et
où, le composé de formule III-1 ou le mélange des composés de formule III-1 et III-2 obtenu à l'étape (a), le composé de formule IIA-1 ou le mélange des composés de formule IIA-1 et IIA-2 obtenu à l'étape (b) et le composé de formule IIB-1 ou le mélange des composés de formule IIB-1 et IIB-2 obtenu à l'étape (c) peuvent être isolés ou non.

37. Procédé selon la revendication 36, où
i. le solvant approprié à l'étape de procédé (a) est choisi dans le groupe constitué par l'acétone, l'acétonitrile, l'éther méthyl-tert-butylique, l'acétate d'éthyle, le dioxane, le tétrahydrofurane, le 1,2-diméthoxyéther ;
ii. le catalyseur approprié à l'étape de procédé (a) est l'iodure de potassium ; et
iii. l'étape de procédé (a) est réalisée dans une plage de températures allant de 50 °C à 80 °C.

38. Procédé selon la revendication 36, où
i. le solvant approprié à l'étape de procédé (b) est choisi dans le groupe constitué par l'acétone, l'acétonitrile, l'alcool éthylique, l'acide acétique, l'éther méthyl-tert-butylique, le dichloroéthane, le dichlorométhane, le dioxane, le diméthylformamide, le diméthylacétamide, le diméthylsulfoxyde, l'acétate d'éthyle, le tétrahydrofurane, le 1,2-diméthoxyéther, le toluène, le xylène et la N-méthyl-2-pyrrolidone ;
ii. le réactif approprié à l'étape de procédé (b) est choisi dans le groupe constitué par l'acide acétique, l'acide trifluoroacétique et l'acide chlorhydrique ; et
iii. l'étape de procédé (b) est réalisée dans une plage de températures allant de 20 °C à 50 °C.

39. Procédé selon la revendication 36, où
i. le solvant approprié à l'étape de procédé (c) est choisi dans le groupe constitué par l'acétonitrile, l'éther méthyl-tert-butylique, le dichlorométhane, le dioxane, le chlorure de thionyle, l'acide acétique, l'alcool méthylique, l'alcool éthylique, le tétrahydrofurane, l'alcool isopropylique et l'alcool tert-butylique ;
ii. le réactif déshydratant approprié à l'étape de procédé (c) est choisi dans le groupe constitué par l'acide sulfurique, l'acide trifluoroacétique, le chlorure de thionyle, le chlorure d'oxalyle, l'acide chlorhydrique méthanolique, le gaz chlorhydrique, l'acide acétique, le bromure d'hydrogène, l'acide triflique, l'acide méthanesulfonique, l'acide p-toluènesulfonique, le chlorure d'hydrogène-1,4-dioxane et le gel de silice ; et
iii. l'étape de procédé (c) est réalisée dans une plage de températures allant de 20 °C à 80 °C.

40. Procédé selon la revendication 36, où
i. le solvant approprié à l'étape de procédé (d) est choisi dans le groupe constitué par l'eau, l'acétonitrile, le dioxane, l'acide acétique, l'acide sulfurique, l'alcool méthylique, l'alcool éthylique, le tétrahydrofurane, l'alcool isopropylique et l'alcool tert-butylique ;
ii. le réactif approprié à l'étape de procédé (d) est un acide choisi dans le groupe constitué par l'acide sulfurique, l'acide chlorhydrique et l'acide acétique ; et
iii. l'étape de procédé (d) est réalisée dans une plage de températures allant de 50 °C à 120 °C.

41. Procédé selon la revendication 36, où le composé de formule IIB-1 est obtenu en variante par le procédé comprenant les étapes suivantes :
i. la cyclisation d'un composé de formule XVII-1 avec l'hydrazine de formule VIII-1 dans un ou plusieurs solvants appropriés et, éventuellement, un ou plusieurs réactifs appropriés pour obtenir un composé de formule XVI-1, 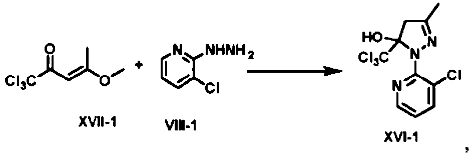 ou
la cyclisation du composé de formule IV-2 et de l'hydrazine de formule VIII-1 dans un ou plusieurs solvants appropriés et, éventuellement, un ou plusieurs réactifs appropriés pour obtenir un composé de formule XVIII-1, 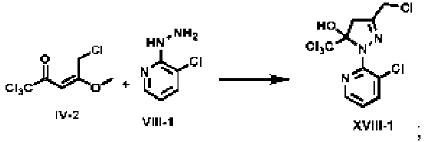
ii. l'élimination d'eau du composé de formule XVI-1 en utilisant un ou plusieurs réactifs déshydratants appropriés dans un ou plusieurs solvants appropriés pour obtenir un composé de formule XV-1 ; 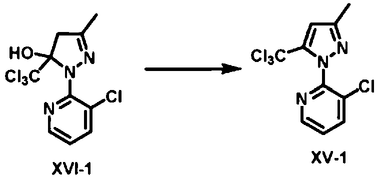 ou
l'élimination d'eau du composé de formule XVIII en utilisant un ou plusieurs réactifs déshydratants appropriés dans un ou plusieurs solvants appropriés pour obtenir un composé de formule XIV, 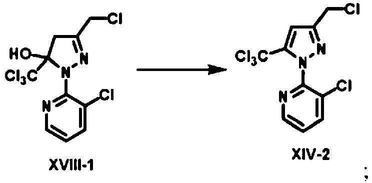
iii. l'halogénation du composé de formule XV-1 en utilisant un agent d'halogénation approprié dans un ou plusieurs solvants appropriés et, éventuellement, un ou plusieurs initiateurs radicalaires pour obtenir un composé de formule XIV-1, 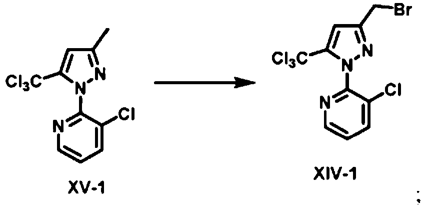 et
iv. l'obtention du composé de formule IIB-1 ou d'un mélange du composé de formule IIB-1 et d'un composé de formule IIB-2 par mise en réaction du composé de formule XIV-1 ou XIV-2 avec le composé de formule VII-1, 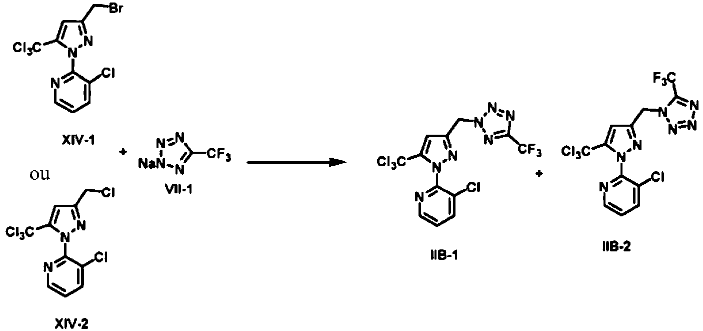 où les composés de formule XVI-1 et XVIII-1 obtenus à l'étape (i), les composés de formule XV-1 et XIV-2 obtenus à l'étape (ii) et le composé XIV-1 obtenu à l'étape (iii) peuvent être isolés ou non.

42. Procédé selon la revendication 41, où
i. le solvant approprié à l'étape de procédé (i) est choisi dans le groupe constitué par l'acétone, l'acétonitrile, l'alcool éthylique, l'acide acétique, l'éther méthyl-tert-butylique, le dichloroéthane, le dichlorométhane, le dioxane, le diméthylformamide, le diméthylacétamide, le diméthylsulfoxyde, l'acétate d'éthyle, le tétrahydrofurane, le 1,2-diméthoxyéther, le toluène, le xylène et la N-méthyl-2-pyrrolidone ;
ii. le réactif approprié à l'étape de procédé (i) est choisi dans le groupe constitué par l'acide acétique, l'acide trifluoroacétique et l'acide chlorhydrique ; et
iii. l'étape de procédé (i) est réalisée dans une plage de températures allant de 20 °C à 50 °C.

43. Procédé selon la revendication 41, où
i. le solvant approprié à l'étape de procédé (ii) est choisi dans le groupe constitué par l'acétonitrile, l'éther méthyl-tert-butylique, le dichlorométhane, le dioxane, le chlorure de thionyle, l'acide acétique, l'alcool méthylique, l'alcool éthylique, le tétrahydrofurane, l'alcool isopropylique et l'alcool tert-butylique ;
ii. le réactif déshydratant approprié à l'étape (ii) du procédé est choisi dans le groupe constitué par l'acide sulfurique, l'acide trifluoroacétique, le chlorure de thionyle, le chlorure d'oxalyle, l'acide chlorhydrique méthanolique, le gaz chlorhydrique, l'acide acétique, le bromure d'hydrogène, l'acide triflique, l'acide méthanesulfonique, l'acide p-toluènesulfonique, le chlorure d'hydrogène-1,4-dioxane et le gel de silice ; et
iv. l'étape de procédé (ii) est réalisée dans une plage de températures allant de 20 °C à 80 °C.

44. Procédé selon la revendication 41, où
i. le solvant approprié à l'étape (iii) est choisi dans le groupe constitué par l'acétonitrile et l'éthanol ;
ii. l'agent d'halogénation à l'étape (iii) est choisi dans le groupe constitué par le N-chlorosuccinimide, le N-bromosuccinimide et Br₂ ;
iii. l'initiateur radicalaire à l'étape (iii) est choisi dans le groupe constitué par le peroxyde de dibenzoyle, le peroxyde d'hydrogène, l'azobisisobutyronitrile, l'acide peracétique, l'acide métachloroperbenzoïque, l'acide trifluoroperacétique et l'acide trichloroperacétique ; et
iv. l'étape (iii) est réalisée dans une plage de températures allant de 30 °C à 100 °C.

45. Procédé selon la revendication 41, où
i. le solvant approprié à l'étape (iv) est choisi dans le groupe constitué par l'acétonitrile et l'éthanol ;
ii. le catalyseur à l'étape (iv) est l'iodure de potassium ; et
iii. l'étape (iv) est réalisée dans une plage de températures allant de 30 °C à 100 °C.

46. Procédé de préparation d'un composé de formule II-1 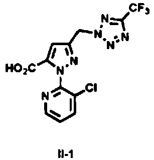 ledit procédé comprenant les étapes suivantes :
a. la mise en réaction d'un composé de formule IV-3 ou IV-4 avec un composé de formule VII-1 en présence d'un ou plusieurs solvants appropriés et, éventuellement, d'un ou plusieurs catalyseurs et/ou réactifs appropriés, pour obtenir un mélange d'un composé de formule III-3 et d'un composé de formule III-4, 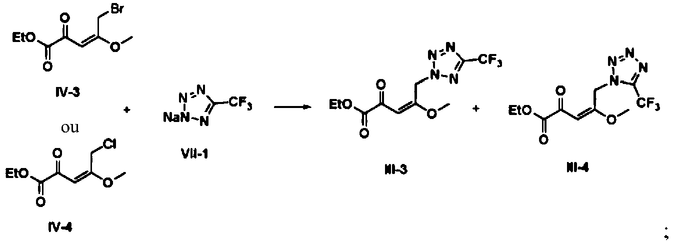
b. la cyclisation du composé de formule III-3, ou du composé de formule III-3 et du composé de formule III-4 dans le mélange, avec une hydrazine de formule VIII-1 dans un ou plusieurs solvants appropriés et, éventuellement, un ou plusieurs réactifs appropriés pour obtenir respectivement un composé de formule IIA-3 ou le mélange du composé de formule IIA-3 et d'un composé de formule IIA-4, 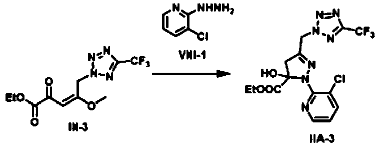 ou 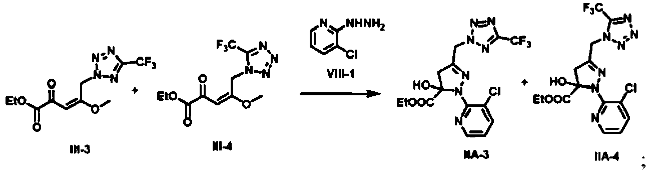
c. l'élimination d'eau du composé de formule IIA-3, ou du composé de formule IIA-3 et du composé de formule IIA-4 dans le mélange, en utilisant un ou plusieurs réactifs déshydratants appropriés dans un ou plusieurs solvants appropriés pour obtenir respectivement un composé de formule IIB-3 ou le mélange du composé de formule IIB-3 et d'un composé de formule IIB-4, 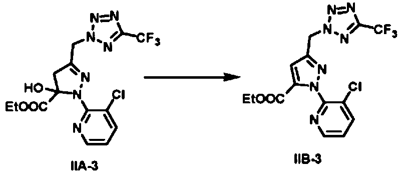 ou 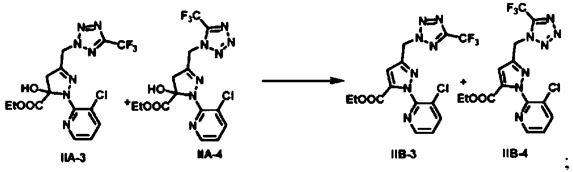 et
d. la conversion du composé de formule IIB-3, ou du composé de formule IIB-3 et du composé de formule IIB-4 dans le mélange, respectivement en le composé de formule II-1 ou en le mélange du composé de formule II-1 et d'un composé de formule II-2, en utilisant un ou plusieurs réactifs appropriés dans un ou plusieurs solvants appropriés et, éventuellement, un ou plusieurs catalyseurs appropriés, 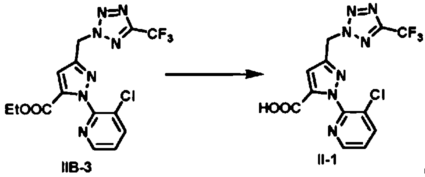 ou 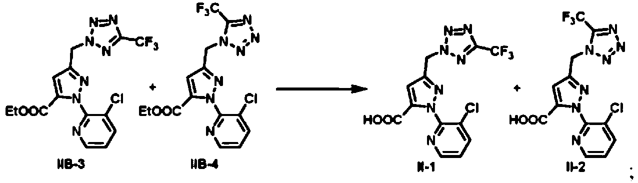
et
où, le composé de formule III-3 ou le mélange des composés de formule III-3 et III-4 obtenu à l'étape (a), le composé de formule IIA-3 ou le mélange des composés de formule IIA-3 et IIA-4 obtenu à l'étape (b) et le composé de formule IIB-3 ou le mélange des composés de formule IIB-3 et IIB-4 obtenu à l'étape (c) peuvent être isolés ou non.

47. Procédé selon la revendication 46, où
i. le solvant approprié à l'étape de procédé (a) est choisi dans le groupe constitué par l'acétone, l'acétonitrile, l'éther méthyl-tert-butylique, l'acétate d'éthyle, le dioxane, le tétrahydrofurane, le 1,2-diméthoxyéther ;
ii. le catalyseur approprié à l'étape de procédé (a) est l'iodure de potassium ; et
iii. l'étape de procédé (a) est réalisée dans une plage de températures allant de 50 °C à 80 °C.

48. Procédé selon la revendication 46, où
i. le solvant approprié à l'étape de procédé (b) est choisi dans le groupe constitué par l'acétone, l'acétonitrile, l'alcool éthylique, l'acide acétique, l'éther méthyl-tert-butylique, le dichloroéthane, le dichlorométhane, le dioxane, le diméthylformamide, le diméthylacétamide, le diméthylsulfoxyde, l'acétate d'éthyle, le tétrahydrofurane, le 1,2-diméthoxyéther, le toluène, le xylène et la N-méthyl-2-pyrrolidone ;
ii. le réactif approprié à l'étape de procédé (b) est choisi dans le groupe constitué par l'acide acétique, l'acide trifluoroacétique et l'acide chlorhydrique ; et
iii. l'étape de procédé (b) est réalisée dans une plage de températures allant de 20 °C à 50 °C.

49. Procédé selon la revendication 46, où
i. le solvant approprié à l'étape de procédé (c) est choisi dans le groupe constitué par l'acétonitrile, l'éther méthyl-tert-butylique, le dichlorométhane, le dioxane, le chlorure de thionyle, l'acide acétique, l'alcool méthylique, l'alcool éthylique, le tétrahydrofurane, l'alcool isopropylique et l'alcool tert-butylique ;
ii. le réactif déshydratant approprié à l'étape de procédé (c) est choisi dans le groupe constitué par l'acide sulfurique, l'acide trifluoroacétique, le chlorure de thionyle, le chlorure d'oxalyle, l'acide chlorhydrique méthanolique, le gaz chlorhydrique, l'acide acétique, le bromure d'hydrogène, l'acide triflique, l'acide méthanesulfonique, l'acide p-toluènesulfonique, le chlorure d'hydrogène-1,4-dioxane et le gel de silice ; et
iii. l'étape de procédé (c) est réalisée dans une plage de températures allant de 20 °C à 80 °C.

50. Procédé selon la revendication 46, où
i. le solvant approprié à l'étape de procédé (d) est choisi dans le groupe constitué par l'eau, l'acétonitrile, le dioxane, l'acide acétique, l'acide sulfurique, l'alcool méthylique, l'alcool éthylique, le tétrahydrofurane, l'alcool isopropylique et l'alcool tert-butylique ;
ii. le réactif approprié à l'étape de procédé (d) est un acide choisi dans le groupe constitué par l'acide sulfurique, l'acide chlorhydrique et l'acide acétique ; et
iii. l'étape de procédé (d) est réalisée dans une plage de températures allant de 50 °C à 120 °C.

51. Procédé selon la revendication 36, où ledit procédé comprend en outre l'étape suivante :
la mise en réaction du composé de formule II-1, éventuellement après conversion en un composé de formule X-1, avec un composé de formule IX-1 pour obtenir le composé de formule I-1, 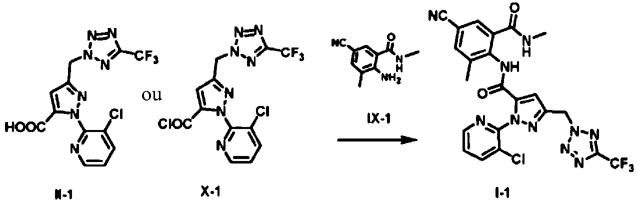 ou
la mise en réaction du composé de formule II-1, éventuellement après conversion en un composé de formule X-1, avec un composé de formule XI pour obtenir un composé de formule XII, et la mise en réaction du composé de formule XII avec l'amine XIII-1 pour obtenir le composé de formule I-1, 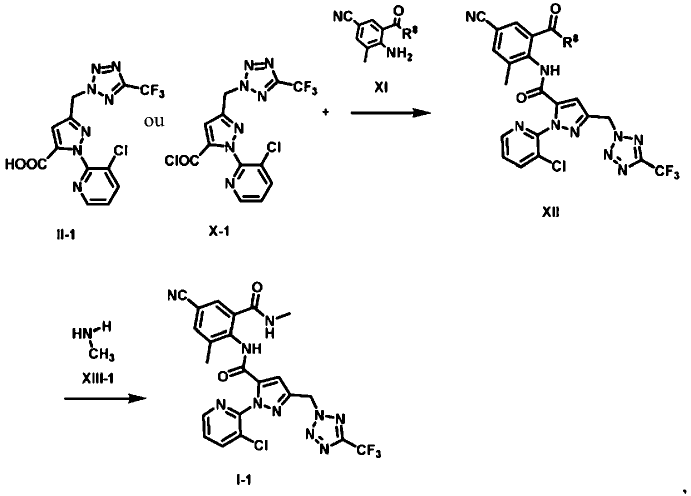
où R⁸ est un groupe hydroxy ou Cl.

52. Procédé selon la revendication 36, où ledit procédé comprend en outre l'étape suivante :
la mise en réaction du composé de formule II-1, éventuellement après conversion en un composé de formule X-1, avec un composé de formule IX-1 pour obtenir le composé de formule I-1, 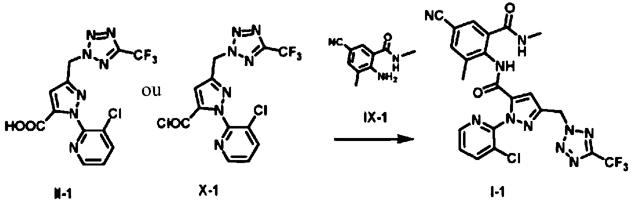 ou
la mise en réaction du composé de formule II-1, éventuellement après conversion en un composé de formule X-1, avec un composé de formule XI pour obtenir un composé de formule XII, et la mise en réaction du composé de formule XII avec l'amine XIII-1 pour obtenir le composé de formule I-1, 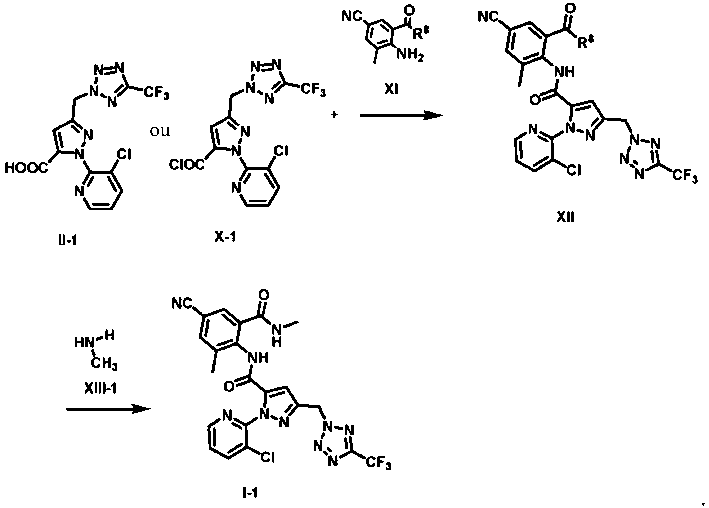
où R⁸ est un groupe hydroxy ou Cl.
